# EUROPEAN PATENT SPECIFICATION

(11) **EP 2 358 907 B1**
(45) Date of publication and mention of the grant of the patent: **20.05.2015**
(21) Application number: 09826774.3
(22) Date of filing: 12.11.2009
(51) Int. Cl.: C12Q 1/68

(54) **AUTISM ASSOCIATED GENETIC MARKERS**
MIT AUTISMUS ASSOZIIERTE GENETISCHE MARKER
MARQUEURS GÉNÉTIQUES ASSOCIÉS À L'AUTISME

(30) Priority: 12.11.2008 US 113963 P
(43) Date of publication of application: 24.08.2011
(73) Proprietor: University of Utah Research Foundation, Salt Lake City UT 84108 (US); Lineagen, Inc., Salt Lake City, UT 84109 (US)
(72) Inventor: LEPPERT, Mark F., Salt Lake City Utah 84108 (US); MCMAHON, William M., Salt Lake City Utah 84108 (US); MATSUNAMI, Nori, Salt Lake City Utah 84108 (US); PAUL, Michael S., Salt Lake City, UT 84108 (US); LINDELL, Alex S., Salt Lake City, UT 84108 (US)
(74) Representative: Bradley, Adrian
(86) International application number: PCT/US2009/064252
(87) International publication number: WO 2010/056897

(56) References cited:
- WO-A2-2005/007812
- WO-A2-2005/019474
- WO-A2-2005/055807
- WO-A2-2005/067603
- US-A1- 2007 218 068
- THE AUTISM GENOME PROJECT CONSORTIUM ET AL: "Genome-wide Linkage Analyses of Quantitative and Categorical Autism Subphenotypes", BIOLOGICAL PSYCHIATRY, ELSEVIER SCIENCE, NEW YORK, NY, US, vol. 64, no. 7, 1 October 2008 (2008-10-01), pages 561-570, XP025348895, ISSN: 0006-3223 [retrieved on 2008-07-16]
- YRIGOLLEN C M ET AL: "Genes Controlling Affiliative Behavior as Candidate Genes for Autism", BIOLOGICAL PSYCHIATRY, ELSEVIER SCIENCE, NEW YORK, NY, US, vol. 63, no. 10, 15 May 2008 (2008-05-15), pages 911-916, XP022633536, ISSN: 0006-3223, DOI: 10.1016/J.BIOPSYCH.2007.11.015 [retrieved on 2008-01-22]
- GUPTA ET AL: "Recent Advances in the Genetics of Autism", BIOLOGICAL PSYCHIATRY, ELSEVIER SCIENCE, NEW YORK, NY, US, vol. 61, no. 4, 2 February 2007 (2007-02-02), pages 429-437, XP005871285, ISSN: 0006-3223, DOI: 10.1016/J.BIOPSYCH.2006.06.020
- JUDITH CONROY ET AL: "Fine mapping and association studies in a candidate region for autism on chromosome 2q31-q32", AMERICAN JOURNAL OF MEDICAL GENETICS PART B: NEUROPSYCHIATRIC GENETICS, vol. 150B, no. 4, 7 October 2008 (2008-10-07), pages 535-544, XP55025530, ISSN: 1552-4841, DOI: 10.1002/ajmg.b.30854
- VRIJENHOEK ET AL.: 'Recurrent CNVs Disrupt Three Candidate Genes in Schizophrenia Patients.' AM J HUM GENET. vol. 83, no. 4, October 2008, pages 504 - 510, XP008148657
- ALLEN-BRADY ET AL.: 'Genome-wide linkage in Utah autism pedigrees.' MOLECULAR PSYCHIATRY 19 May 2009, XP008148658
- DATABASE SNP 25 May 2006 'rs792065_SNP. Reference SNP(refSNP) Cluster Report: rs792065.', XP008148661 Database accession no. rs792065
- AREHART-TREICHEL.: 'Overlap Found Between Autism, Schizophrenia-Spectrum Disorders.' PSYCHIATRIC NEWS. vol. 43, no. 19, October 2008, page 20, XP008148659
- KATO ET AL.: 'MOCSphaser: a haplotype inference tool from a mixture of copy number variation and single nucleotide polymorphism data.' BIOINFORMATICS. vol. 24, no. 14, July 2008, pages 1645 - 1646, XP008148662
- M. Kusenda ET AL: "The role of rare structural variants in the genetics of autism spectrum disorders", Cytogenet Genome Res, 10 September 2008 (2008-09-10), pages 36-43, XP055048619, Retrieved from the Internet: URL:http://www.ncbi.nlm.nih.gov/pmc/articl es/PMC2920182/pdf/cgr0123-0036.pdf [retrieved on 2013-01-03]
- Jasmin Roohi ET AL: "A de novo apparently balanced translocation [46,XY,t(2;9)(p13;p24)] interruptingRAB11FIP5 identifies a potential candidate gene for autism spectrum disorder", American Journal of Medical Genetics Part B: Neuropsychiatric Genetics, vol. 147B, no. 4, 1 January 2008 (2008-01-01), pages 411-417, XP055048620, ISSN: 1552-4841, DOI: 10.1002/ajmg.b.30755

## Description

### Federally Sponsored Research or Development

This patent application was supported by R01 MH06359 from NIH, and GCRC grant number M01-RR025764 from the National Center for Research Resources.

### Technical Field

This invention relates to the field of disease risk, susceptibility, prediction, diagnosis and prognosis. In addition, the invention relates to the use of genetic markers for detecting the risk of disease in an individual. The methods and compositions disclosed herein are particularly useful for the detection, diagnosis, and prognosis of individuals at risk of developing, or affected with, autism and/or autism spectrum disorders. More particularly, the invention is related to determining the risk of individuals to autism and autism spectrum disorders and methods for disease diagnosis and prognosis.

### Background

Autism spectrum disorders (ASDs) are complex, heterogeneous, behaviorally-defined disorders characterized by impairments in social interaction and communication as well as by repetitive and stereotyped behaviors and interests. While environmental elements, such as peri- and post-natal stress, likely contribute to the development of autism, evidence of chromosomal abnormalities, mutations in single genes, and multiple gene polymorphisms in autistic individuals show that autism is a genetic disorder.

ASDs include Autistic Disorder (autism), Asperger Disorder, and Pervasive Developmental Disorder - Not Otherwise Specified (PDD-NOS). Prevalence estimates for ASDs have been reported to be approximately 1 in every 100 children in the general population. In families with an autistic child, recurrence rates are estimated to be greater than 15% that an additional offspring will also have autism (Landa RJ, Holman KC, Garrett-Mayer E. Social and communication development in toddlers with early and later diagnosis of autism spectrum disorders. Arch Gen Psychiatry 2007;64:853-64; Landa RJ. Diagnosis of autism spectrum disorders in the first 3 years of life. Nat Clin Pract Neurol 2008;4:138-47).

The current state-of-the-art diagnosis of ASD is a series of various behavioral questionnaires. Because the ASD phenotype is so complicated, a molecular-based test would greatly improve the accuracy of diagnosis at an earlier age, when phenotypic/behavioral assessment is not possible, or integrated with pehnotypic/behavioral assessment. Also, diagnosis at an earlier age would allow initiation of ASD treatment at an earlier age which may be beneficial to short and long-term outcomes.

Genetic factors play a substantial role in ASDs (Abrahams BS, Geschwind DH. Advances in autism genetics: on the threshold of a new neurobiology. Nat Rev Genet 2008;9:341-55). Previous genome-wide linkage and association studies have implicated multiple genetic regions may be involved in autism and ASDs. Such heterogeneity increases the value of studies that include large extended pedigrees. Many autism studies have focused on small families (sibling pairs, or two parents and an affected offspring) to try to localize autism predisposition genes. These collections of small families may include cases with many different susceptibility loci. Subjects affected with ASDs who are members of a large extended family may be more likely to share the same genetic causes through their common ancestors. Within such families, autism may be more genetically homogeneous. Additionally, these family members are more likely to share similar environmental exposures, facilitating possible future analyses of gene by environment interaction effects.

### Brief Description of the Drawings

FIGs. 1a-1b show genome-wide linkage results from SNP set using: 1a) recessive and 1b) dominant models.
FIGs. 2a-2d show linkage results for chromosomes 15, 2, 7, and 13, respectively.
FIG. 3 shows chromosome 15 LOP scores.
FIG. 4 shows chromosome 15 NPL LOD scores.
FIG. 5 shows the chromosome location and SNP classification of 4,477 functional SNPs identified in 26 ASD-affected individuals.
FIG. 6 shows the chromosome location and SNP classification of 388 candidate SNPs identified in 26 ASD-affected individuals.
FIG. 7 shows the chromosome location and the copy number variant (CNV) classification of 4,449 CNVs identified in a population of 55 autism-affected individuals.
FIG. 8 shows the chromosome location and classification 28 candidate CNVs identified in a population of 55 autism-affected individuals.

### Detailed Description

Disclosed are molecules, materials, compositions, and components that can be used for, can be used in conjunction with, can be used in preparation for, or are products of the disclosed methods and compositions. These and other materials are disclosed herein, and it is understood that when combinations, subsets, interactions, groups, etc. of these materials are disclosed and while specific reference of each various individual and collective combinations and permutation of these molecules and compounds may not be explicitly disclosed, each is specifically contemplated and described herein. For example, if a nucleotide or nucleic acid is disclosed and discussed and a number of modifications that can be made to a number of molecules including the nucleotide or nucleic acid are discussed, each and every combination and permutation of nucleotide or nucleic acid and the modifications that are possible are specifically contemplated unless specifically indicated to the contrary. This concept applies to all aspects of this application including, but not limited to, steps in methods of making and using the disclosed molecules and compositions. Thus, if there are a variety of additional steps that can be performed it is understood that each of these additional steps can be performed with any specific embodiment or combination of embodiments of the disclosed methods, and that each such combination is specifically contemplated and should be considered disclosed.

Those skilled in the art will recognize, or be able to ascertain using no more than routine experimentation, many equivalents to the specific embodiments of the method and compositions described herein. Such equivalents are intended to be encompassed by the following claims.

It is understood that the disclosed methods and compositions are not limited to the particular methodology, protocols, and reagents described as these may vary. It is also to be understood that the terminology used herein is for the purpose of describing particular embodiments only, and is not intended to limit the scope of the present invention which will be limited only by the appended claims.

Unless defined otherwise, all technical and scientific terms used herein have the meanings that would be commonly understood by one of skill in the art in the context of the present specification.

It must be noted that as used herein and in the appended claims, the singular forms "a," "an," and "the" include plural reference unless the context clearly dictates otherwise Thus, for example, reference to "a nucleotide" includes a plurality of such nucleotides, reference to "the nucleotide" is a reference to one or more nucleotides and equivalents thereof known to those skilled in the art, and so forth.

"Optional" or "optionally" means that the subsequently described event, circumstance, or material may or may not occur or be present, and that the description includes instances where the event, circumstance, or material occurs or is present and instances where it does not occur or is not present.

Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10" as well as "greater than or equal to 10" is also disclosed. It is also understood that the throughout the application, data is provided in a number of different formats, and that this data represents endpoints and starting points and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

As used herein, the term "subject" means any target of administration. The subject can be a vertebrate, for example, a mammal. Thus, the subject can be a human. The term does not denote a particular age or sex. Thus, adult and newborn subjects, as well as fetuses, whether male or female, are intended to be covered. A patient refers to a subject afflicted with a disease or disorder. Unless otherwise specified, the term "patient" includes human and veterinary subjects.

As used herein, the term "biomarker" or "biological marker" means an indicator of a biologic state and may include a characteristic that is objectively measured as an indicator of normal biological processes, pathologic processes, or pharmacologic responses to a therapeutic or other intervention. In one embodiment, a biomarker may indicate a change in expression or state of a protein that correlates with the risk or progression of a disease, or with the susceptibility of the disease in an individual. In certain embodiments, a biomarker may include one or more of the following: genes, proteins, glycoproteins, metabolites, cytokines, and antibodies.

As used herein, the term *"in vitro* diagnostic" means any form of diagnostic test product or test service, including but not limited to a FDA approved, or cleared, In Vitro Diagnostic (IVD), Laboratory Developed Test (LDT), or Direct-to-Consumer (DTC), that may be used to assay a sample and detect or indicate the presence of, the predisposition to, or the risk of, diseases, disorders, conditions, infections and/or therapeutic responses. In one embodiment, an *in vitro* diagnostic may be used in a laboratory or other health professional setting. In another embodiment, an *in vitro* diagnostic may be used by a consumer at home. *In vitro* diagnostic test comprise those reagents, instruments, and systems intended for use in the *in vitro* diagnosis of disease or other conditions, including a determination of the state of health, in order to cure, mitigate, treat, or prevent disease or its sequelae. In one embodiment, *in vitro* diagnostic products may be intended for use in the collection, preparation, and examination of specimens taken from the human body. In certain embodiments, *in vitro* diagnostic tests and products may comprise one or more laboratory tests such as one or more *in vitro* diagnostic tests. As used herein, the term "laboratory test" means one or more medical or laboratory procedures that involve testing samples of blood, urine, or other tissues or substances in the body.

In one embodiment, the methods and *in vitro* diagnostic tests and products described herein may be used for the diagnosis of autism and ASD in at-risk patients, patients with non-specific symptoms possibly associated with autism, and/or patients presenting with related disorders. In another embodiment, the methods and *in vitro* diagnostic tests described herein may be used for screening for risk of progressing from at-risk, non-specific symptoms possibly associated with ASD, and/or fully-diagnosed ASD. In certain embodiments, the methods and *in vitro* diagnostic tests described herein can be used to rule out screening of diseases and disorders that share symptoms with ASD. In yet another embodiment, the methods and *in vitro* diagnostic tests described herein may indicate diagnostic information to be included in the current diagnostic evaluation in patients suspected of having autism.

In one embodiment, an *in vitro* diagnostic test kit, may comprise one or more devices, tools, and equipment configured to collect a genetic sample from an individual. In one embodiment of an *in vitro* diagnostic test kit, tools to collect a genetic sample may include one or more of a swab, a scalpel, a syringe, a scraper, a container, and other devices and reagents designed to facilitate the collection, storage, and transport of a genetic sample. In one embodiment, an *in vitro* diagnostic test may include reagents or solutions for collecting, stabilizing, storing, and processing a genetic sample. Such reagents and solutions for nucleotide collecting, stabilizing, storing, and processing are well known by those of skill in the art and may be indicated by specific methods used by an *in vitro* diagnostic test as described herein. In another embodiment, an *in vitro* diagnostic test kit as disclosed herein, may comprise a microarray apparatus and reagents, a flow cell apparatus and reagents, a multiplex nucleotide sequencer and reagents, and additional hardware and software necessary to assay a genetic sample for certain genetic markers and to detect and visualize certain genetic markers.

The present disclosure also includes nucleic acid molecules that are oligonucleotides capable of hybridizing, under stringent hybridization conditions, with complementary regions of a gene associated with ASD containing a genetic polymorphism described herein. A nucleic acid can be DNA or RNA, and single-or double-stranded. Oligonucleotides can be naturally occurring or synthetic, but are typically prepared by synthetic means. Oligonucleotides, as described herein, may include segments of DNA, or their complements. The DNA segments can be between 5 and 100 contiguous bases, and often range from 5, 10, 12, 15, 20, or 25 nucleotides to 10, 15, 30, 25, 20, 50 or 100 nucleotides. Nucleic acids between 5-10, 5-20, 10-20, 12-30, 15-30, 10-50, 20-50 or 20-100 bases are common. The genetic polymorphic site can occur within any position of the DNA segment.

Oligonucleotides can be RNA, DNA, or derivatives of either. The minimum size of such oligonucleotides is the size required for formation of a stable hybrid between an oligonucleotide and a complementary sequence on a nucleic acid molecule. The present disclosure includes oligonucleotides that can be used as, for example, probes to identify nucleic acid molecules or primers to produce nucleic acid molecules. Preferred oligonucleotide probes or primers include a single base change of a polymorphism or the wildtype nucleotide that is located at the same position. Preferably the nucleotide of interest occupies a central position of a probe.

In one embodiment, the nucleotide of interest occupies a 3' position of a primer. In another embodiment, an array of oligonucleotides are provided, where discrete positions on the array are complementary to one or more of the provided polymorphic sequences. Such an array may comprise a series of oligonucleotides, each of which can specifically hybridize to a different polymorphism. Arrays of interest may further comprise sequences, including polymorphisms, of other genetic sequences, particularly other sequences of interest for pharmacogenetic screening. As with other human polymorphisms, the polymorphisms described also have more general applications, such as forensic, paternity testing, linkage analysis and positional cloning.

Autism is typically characterized as part of a spectrum of disorders (ASD) including Asperger syndrome (AS) and other pervasive developmental disorders (PDD-NOS). Autism shall be construed as any condition of impaired social interaction and communication with restricted repetitive and stereotyped patterns of behavior, interests and activities present before the age of 3, to the extent that health may be impaired. AS is distinguished from autistic disorder by the lack of a clinically significant delay in language development in the presence of the impaired social interaction and restricted repetitive behaviors, interests, and activities that characterize ASDs. PDD-NOS is used to categorize individuals who do not meet the strict criteria for autism but who come close, either by manifesting atypical autism or by nearly meeting the diagnostic criteria in two or three of the key areas.

Autism-associated disorders, diseases or pathologies include, more specifically, any metabolic and immune disorders, epilepsy, anxiety, depression, attention deficit hyperactivity disorder, speech delay or language impairment, motor incoordination, mental retardation, schizophrenia and bipolar disorder. The various embodiments and examples disclosed herein may be used in various subjects, particularly human, including adults and children and at the prenatal stage.

Described herein are methods directed to the use of genetic markers for detecting the risk, diagnosing, and predicting ASD in an individual. In one embodiment, the methods disclosed herein may be used to indicate if an individual is at risk of ASD. In one embodiment, the methods disclosed herein may be used to diagnose ASD in an individual. In one embodiment, the methods disclosed may be used to characterize the clinical course or status of ASD in a subject. In one embodiment, the methods as disclosed herein may be used to predict a response in a subject to an existing treatment for ASD, or a treatment for ASD that is in development or has yet to be developed. The methods described herein can be employed to screen for any type of ASD including, any metabolic and immune disorders, epilepsy, anxiety, depression, attention deficit hyperactivity disorder, speech delay or language impairment, motor incoordination, mental retardation, schizophrenia and bipolar disorder.

The term "genetic marker" as used herein refers to one or more inherited or *de novo* variations in DNA structure with a known physical location on a chromosome. Genetic markers include variations, or polymorphisms, in specific nucleotides or chromosome regions. Examples of genetic markers include, single nucleotide polymorphisms (SNPs), and copy number variations and copy number changes (CNVs). Genetic markers can be used to associate an inherited phenotype, such as a disease, with a responsible genotype. Genetic markers may be used to track the inheritance of a nearby gene that has not yet been identified, but whose approximate location is known. The genetic marker itself may be a part of a gene's coding region or regulatory region. For example, a genetic marker may be a functional polymorphism that may alter gene function or gene expression. Alternatively, a genetic marker may be a non-functional polymorphism.

In one embodiment, the detection of the presence of a genetic marker or functional polymorphism associated with a gene linked to ASD may indicate that the subject is affected with ASD or is at risk of developing ASD. A subject who is at increased risk of developing ASD is one who is predisposed to the disease, has genetic susceptibility for the disease and/or is more likely to develop the disease than subjects in which the genetic marker is present or is is absent.

As used herein, markers for diagnosis, prediction and prognosis of ASD are genetic and/or biological markers, the presence of or the absence of may be used to indicate or predict the status of ASD in an individual. In one embodiment, the presence of or the absence of certain genetic markers for diagnosis, prediction and prognosis of ASD may indicate whether an individual may be affected with ASD, if an individual may be predisposed to ASD, and the likely outcome of ASD therapy in an individual.

As used herein, the term "regulatory sequence" is a segment of DNA where regulatory proteins such as transcription factors may bind. Regulatory sequences may be positioned near the gene being regulated. For example, regulatory sequences may be positions upstream of the gene being regulated. Regulatory sequences control gene expression and subsequent protein expression.

As used herein, term "linked" describes a region of a chromosome that is shared more frequently in patients or subjects, including family members, affected by a particular disease or disorder than would be expected or observed by chance, thereby indicating that the gene or genes or other identified marker(s) within the linked chromosome region contain or are associated with an allele that is correlated with the presence of, or increased or decreased risk of, the disease or disorder. Once linkage is established, association studies can be used to narrow the region of interest or to identify the marker correlated with the disease or disorder.

As used herein, the term "validated genetic marker" or "verified marker", such as a validated SNP or a verified SNP, describes SNPs that have been genotyped and confirmed to be present in one or more individuals. In one embodiment, genetic marker validation, such as SNP validation, may be performed with various techniques including primer extension; hybridization, ligation, PCR amplification, and restriction enzyme digestion. In another embodiment, SNP validation may be performed using DNA melting curve analysis or DNA sequencing, or a combination thereof.

In the methods described herein, the detection of a genetic marker in a subject can be carried out according to methods well known in the art. For example DNA is obtained from any suitable sample from the subject that will contain DNA and is then prepared and analyzed according to well-established protocols for the presence of genetic markers. In some embodiments, analysis of the DNA may include assaying the DNA for the presence of or the absence of particular genetic markers or nucleotide sequences. In one such embodiment, a DNA assay can be carried out by amplification of the region of interest according to amplification protocols well known in the art (e.g., polymerase chain reaction, ligase chain reaction, strand displacement amplification, transcription-based amplification, self-sustained sequence replication (3SR), Qβ replicase protocols, nucleic acid sequence-based amplification (NASBA), repair chain reaction (RCR) and boomerang DNA amplification (BDA)). The amplification product can then be visualized directly in a gel by staining or the product can be detected by hybridization with a detectable probe. When amplification conditions allow for amplification of all allelic types of a genetic marker, the types can be distinguished by a variety of well-known methods, such as hybridization with an allele-specific probe, secondary amplification with allele-specific primers, restriction endonuclease digestion, or electrophoresis. Thus, the present disclosure can further provide oligonucleotides for use as primers and/or probes for detecting and/or identifying genetic markers according to the methods of this invention.

In one embodiment, the presence of or the absence of one or more genetic markers may be visualized by staining or marking the genetic markers with molecular dyes, probes, or other analytes and reagents specific to the genetic markers of interest. In one such embodiment, the genetic markers may be detected by automated methods comprising fluorescent probes, melting curve analysis, and other genetic marker detection methods known by those of skill in the art. In one embodiment, one or more genetic markers may be detected and the detected genetic markers may be visualized on a display showing the location of the genetic markers on a genetic sample. In one such embodiment, the detection of one or more genetic markers may be detected by an electronic device which generates a signal that may be shown on a display in order for a user to visualize the presence of or the absence of one or more genetic markers, and/or the location of one or more genetic markers.

In one embodiment, the methods disclosed herein may include the analysis and assay of a genetic sample for the presence of or the absence of one or more genetic markers, the method further comprising the use of one or more DNA sequencing methods. In one such embodiment, the methods disclosed herein may include next-generation sequencing methods such as those used by next-generation sequencing platforms, such as SOLiD (Applied Biosystems, Inc., Foster City, CA, USA), 454 (454 Life Sciences, Branford, CT, USA), Illumina Genome Analyzer ((Illumina, Inc., San Diego, CA, USA), Helicos (Helicos BioSciences Corporation, Cambridge MA, USA), and Sanger. In one embodiment, DNA sequencing be performed using methods well known in the art including mass spectrometry technology and whole genome sequencing technologies (e.g: those used by Pacific Biosciences, Menlo Park, CA, USA), etc.

In one embodiment, genetic markers may be associated with ASD according to methods well known in the art and as disclosed in the examples provided herein for correlating genetic markers with various phenotypic traits, including disease states, disorders and pathological conditions and levels of risk associated with developing a disease, disorder or pathological condition. In one embodiment, identifying such correlation may include conducting analyses that establish a statistically significant association and/or a statistically significant correlation between the presence of a genetic marker or a combination of markers and the phenotypic trait in the subject. In one such embodiment, an analysis that identifies a statistical association (e.g., a significant association) between a genetic marker or combination of genetic markers and a phenotype of interest establishes a correlation between the presence of the genetic marker, or combination of genetic markers in a subject, and the particular phenotype being analyzed.

In one embodiment, genetic markers may be associated with ASD by identifying the unique polymorphic genetic markers that are present in a population affected by ASD but are not present in a normal population. In one such example, genetic samples may be collected from individuals affected with ASD and the genetic samples may be assayed for the presence of or the absence of one or more genetic polymorphisms. The genetic polymorphisms present in the ASD-affected population are compared with the genetic polymorphisms in a normal healthy population and the genetic polymorphisms unique to the ASD-affected population may be associated with ASD. In one such embodiment, the unique genetic markers in an ASD-affected population may be certain chromosome regions, SNPs, CNVs, and other genetic markers.

The embodiments and examples herein disclose methods comprising the detection of one or more genetic markers in a subject that are associated with autism or ASD. Within the context of the present invention, the term "detection" includes the detection, diagnosis, monitoring, dosing, comparison, etc., at various stages, including early, pre-symptomatic stages and late stages in adults and children and pre-birth. Diagnosis or detection typically includes the prognosis, the assessment of a predisposition or risk of development, the characterization of a subject to define most appropriate treatment (pharmacogenetics), etc.

In one embodiment, the present disclosure provides methods to determine the risk of ASD in an individual. In one such ebodiment, the methods disclosed herein may determine whether an individual is at risk of developing autism, ASD, or an autism-associated disorder or suffers from autism, ASD, or an autism-associated disorder. Other embodiments provide methods to determine whether an individual is likely to respond positively to an ASD therapy or whether an individual is at risk of developing an adverse side effect to an ASD therapy.

Another embodiment includes methods of detecting the presence of or predisposition to autism, an ASD, or an autism-associated disorder in a subject, the method comprising detecting in a sample from the subject the presence of one or more genetic markers associated with autism or ASD. The presence of a genetic marker linked with autism or ASD may indicate a risk of ASD, or may be indicative of the presence or predisposition to autism, an ASD, or an autism-associated disorder.

Another particular object described resides in a method of detecting the protection from autism, an ASD, or an autism-associated disorder in a subject, the method comprising detecting the presence of or the absence of one or more genetic markers in a sample from the subject, the presence of or the absence of the one or more genetic markers being indicative of the protection from autism, an ASD, or an autism-associated disorder.

The teachings disclosed herein provide a collection of polymorphisms in genes or chromosomal regions associated with autism, an ASD, or an autism-associated disorder. Detection of polymorphisms is useful in designing and performing diagnostic assays for evaluation of genetic risks or susceptibility for ASD and other related conditions. Analysis of polymorphisms is also useful in designing prophylactic and therapeutic regimes customized to ASD treatments. Detection of polymorphisms is also useful for conducting clinical trials of drugs for treatment of ASD. The teachings disclosed herein also provide methods and compositions for clinical screening and diagnosis of ASD in a subject and for identifying patients most likely to respond to a particular therapeutic treatment, for monitoring the results of ASD therapy, and for drug screening and drug development. A drug or pharmaceutical agent means any substance used in the prevention, diagnosis, alleviation, treatment or cure of a disease. These terms include a vaccine, for example.

Polymorphism refers to the occurrence of two or more genetically determined alternative sequences or alleles in a population. A polymorphic genetic marker or site is the locus at which divergence occurs. In one embodiment, genetic markers have at least two alleles, each occurring at a frequency of greater than 1%, and more preferably greater than 10% or 20% of a selected population. A polymorphic locus may be as small as one base pair.

Polymorphic genetic markers may include SNPs, restriction fragment length polymorphisms, variable number of tandem repeats, hypervariable regions, minisatellites, dinucleotide repeats, trinucleotide repeats, tetranucleotide repeats, simple sequence repeats, and insertion elements. Polymorphic genetic markers as disclosed herein may also include cytogenetic abnormalities such as structural genomic changes like DNA copy number changes or CNVs. In one embodiment, CNVs may include deletions, insertions, inversions, and duplications of the nucleotides within one or more chromosomes of an individual.

A SNP occurs at a polymorphic site occupied by a single nucleotide, which is the site of variation between allelic sequences. A single nucleotide polymorphism may arise due to substitution of one nucleotide for another at the polymorphic site. A transition is the replacement of one purine by another purine or one pyrimidine by another pyrimidine. A transversion is the replacement of a purine by a pyrimidine or vice versa. Single nucleotide polymorphisms can also arise from a deletion of a nucleotide or an insertion of a nucleotide relative to a reference allele.

In one embodiment, the presence of or the absence of one or more genetic markers may be predictive of whether an individual is at risk for or susceptible to ASD. In one such embodiment, one or more genetic markers may be associated with a disease phenotype by the use of a genome wide association study (GWAS). As generally know by those of skill in the art, a GWAS is an examination of genetic polymorphism across a given genome, designed to identify genetic associations with a trait or phenotype of interest, such as autism, an ASD, or an autism-associated disorder. If certain genetic polymorphisms are detected more frequently in people with ASD, the variations are said to be "associated" with ASD. The polymorphisms associated with ASD may either directly cause the disease phenotype or they may be in linkage disequilibrium (LD) with nearby genetic mutations that influence the individual variation in the disease phenotype. As used herein, LD is the non-random association of alleles at two or more loci.

In one embodiment, a GWAS may be conducted using a DNA microarray as generally known in the art. Array-based detection can be performed to detect genetic polymorphisms. Commercially available arrays, e.g., from Affymetrix, Inc. (Santa Clara, Calif.) or other manufacturers may be used to detect polymorphisms. Reviews regarding the operation of nucleic acid arrays include Sapolsky et al. (1999) "High-throughput polymorphism screening and genotyping with high-density oligonucleotide arrays." Genetic Analysis: Biomolecular Engineering 14:187-192; Lockhart (1998) "Mutant yeast on drugs" Nature Medicine 4:1235-1236; Fodor (1997) "Genes, Chips and the Human Genome." FASEB Journal 11:A879; Fodor (1997) "Massively Parallel Genomics." Science 277: 393-395; and Chee et al.. (1996) "Accessing Genetic Information with High-Density DNA Arrays." Science 274:610-614.

As generally known in the art, a variety of probe arrays can be used for detection of polymorphisms that can be correlated to the phenotypes of interest. In one embodiment, DNA probe array chips or larger DNA probe array wafers (from which individual chips would otherwise be obtained by breaking up the wafer) may be used. In one such embodiment, DNA probe array wafers may comprise glass wafers on which high density arrays of DNA probes (short segments of DNA) have been placed. Each of these wafers can hold, for example, millions of DNA probes that are used to recognize sample DNA sequences (e.g., from individuals or populations that may comprise polymorphisms of interest). The recognition of sample DNA by the set of DNA probes on the glass wafer takes place through DNA hybridization. When a DNA sample hybridizes with an array of DNA probes, the sample binds to those probes that are complementary to the sample DNA sequence. By evaluating to which probes the sample DNA for an individual hybridizes more strongly, it is possible to determine whether a known sequence of nucleic acid is present or not in the sample, thereby determining whether a polymorphism found in the nucleic acid is present.

In one embodiment, the use of DNA probe arrays to obtain allele information typically involves the following general steps: design and manufacture of DNA probe arrays, preparation of the sample, hybridization of sample DNA to the array, detection of hybridization events, and data analysis to determine sequence. In one such embodiment, wafers may be manufactured using a process adapted from semiconductor manufacturing to achieve cost effectiveness and high quality, and are available, e.g., from Affymetrix, Inc. of Santa Clara, Calif.

Provided herein are methods for diagnosis and prediction of ASD in an individual using genetic analysis to assay for the presence of one or more genetic markers. In one such embodiment, the methods may include the steps of collecting a sample from an individual and assaying the sample for the presence of or the absence of one or more of the genetic markers disclosed herein, wherein the detection of the one or more genetic markers may indicate whether an individual is affected with ASD or may be predisposed to ASD. The sample can be a nucleotide sample comprising at least a portion of the genome of an individual. In one embodiment, the collection of a sample from an individual may comprise purifying the genetic sample. In another embodiment, the collection of a sample from an individual may comprise collecting a genetic sample, purifying the genetic sample, and amplifying at least a portion of the nucleotides in the purified genetic sample. In one such embodiment, purifying the genetic sample may comprise well known methods of DNA purification, including the necessary reagents and solutions for nucleotide storage and processing. In one embodiment, amplifying at least a portion of the nucleotides in a genetic sample may comprise standard DNA amplification methods, such as PCR amplification and other methods known by those of skill in the art.

In one embodiment, the methods disclosed herein may comprise assaying the presence of one or more polymorphisms in an individual which may include methods generally known in the art. In one such embodiment, methods for assaying a genetic polymorphism in an individual may include assaying an individual for the presence of or the absence of a SNP associated with ASD using one or more genotyping assays such as a SNP array, PCR-based SNP genotyping, DNA hybridization, fluorescence microscopy, and other methods known by those of skill in the art. In another embodiment, methods for assaying the presence of or the absence of one or more SNP markers associated with ASD may include providing a nucleotide sample from an individual and assaying the nucleotide sample for the presence of or the absence of one or more SNP markers. In one embodiment, the sample may be a biological fluid or tissue comprising nucleated cells including genomic material. Examples of biological fluids include, e.g., whole blood, serum, plasma, cerebrospinal fluid, urine, tears or saliva. Examples of tissue include, e.g., connective tissue, muscle tissue, nervous tissue, epithelial tissue, and combinations thereof.

In one embodiment, the methods disclosed herein may include the step of completing the Autism Diagnotic Observation Schedule (ADOS) (Lord et al., 1989) and/or completing the Autism Diagnostic Interview-Revised (ADI-R) (Lord C, et al., 1993, Infant Mental Health, 14:234-52) for an individual. In another embodiment, the methods disclosed herein may comprise the step of completing the Social Communication Questionnaire (SCQ) (Berument SK, Rutter M, Lord C, Pickles A, Bailey A. Autism Screening Questionnaire. Los Angeles, CA: Western Psychological Services; 1999). In another embodiment, the methods disclosed herein may comprise the step of completing the SCQ and the ADI-R. In another embodiment, the methods disclosed herein may comprise screening an individual for symptoms fitting an AGRE (Autism Genetic Resource Exchange) classification of autism, broad spectrum (patterns of impairment along the spectrum of pervasive developmental disorders, including PDD-NOS and AS).

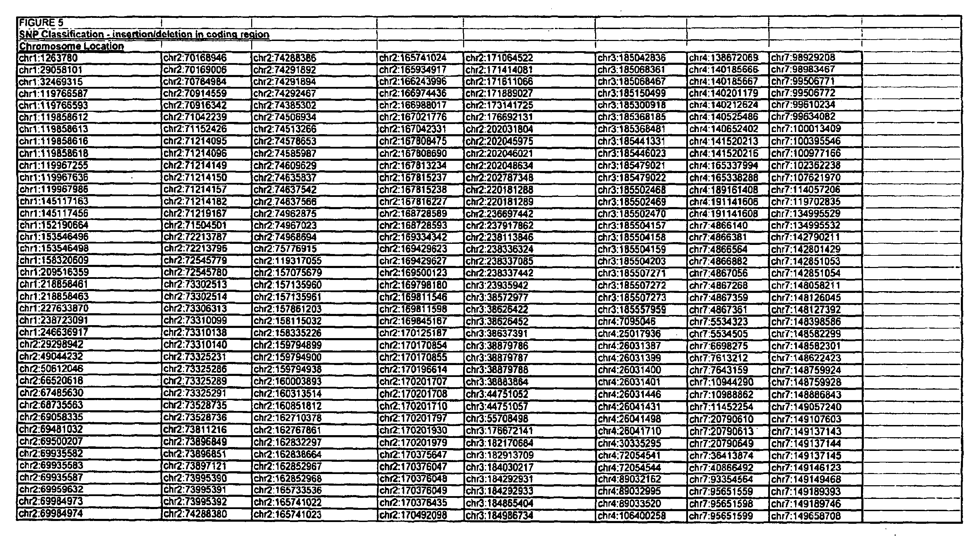

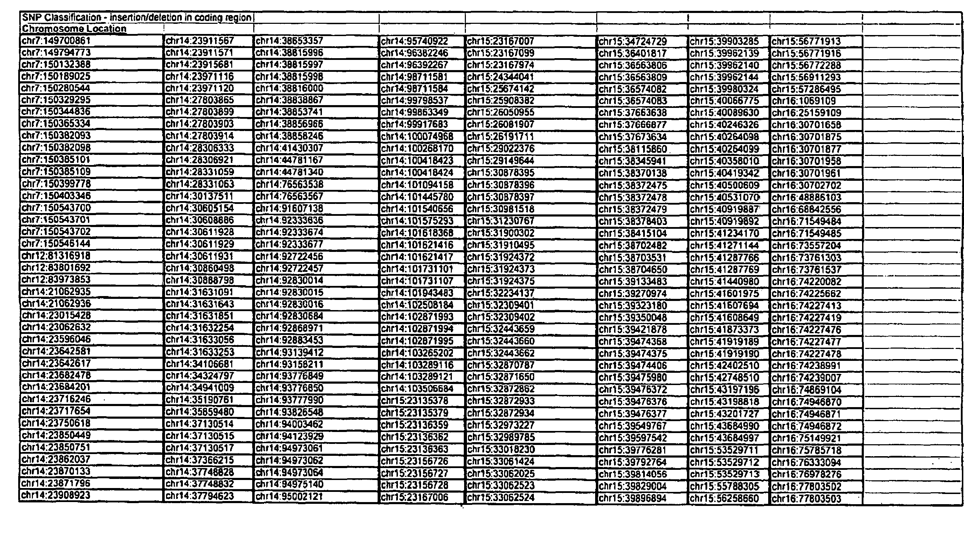

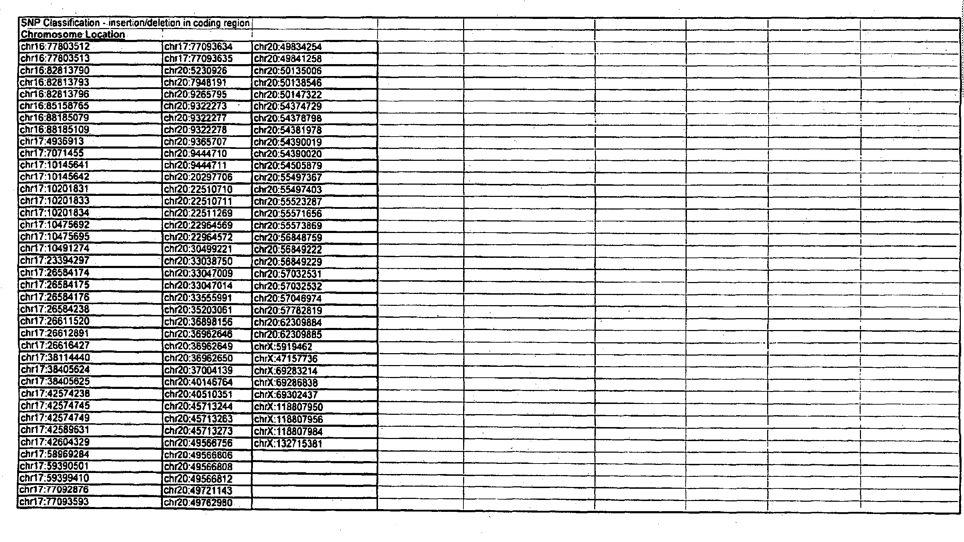

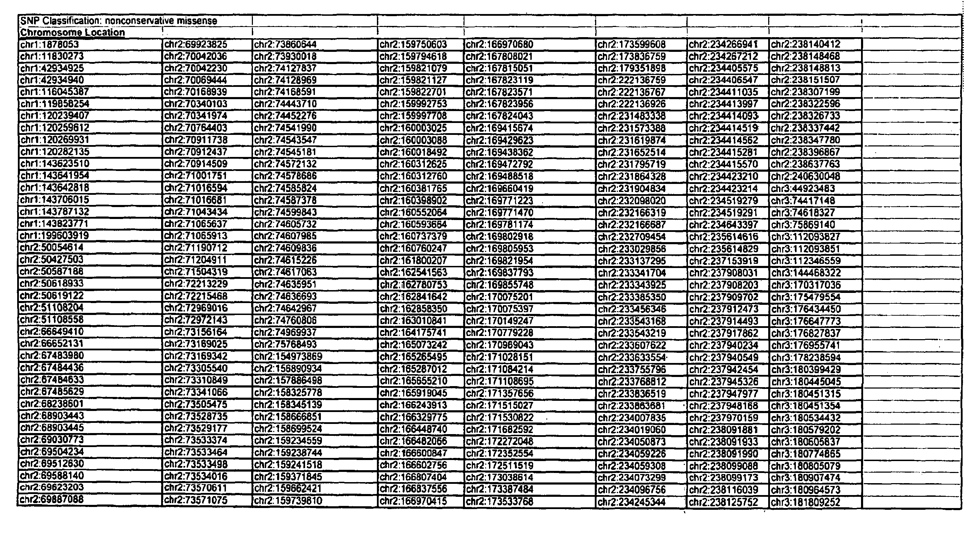

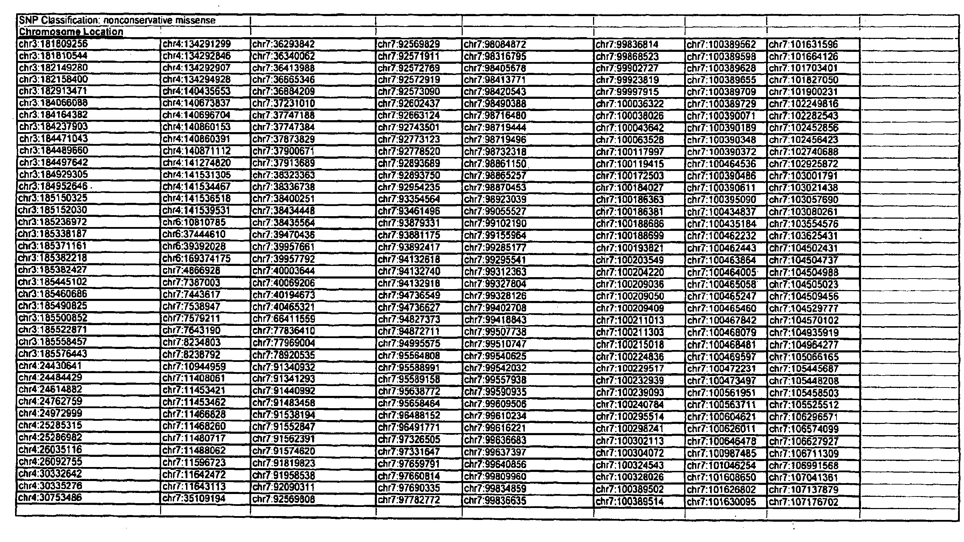

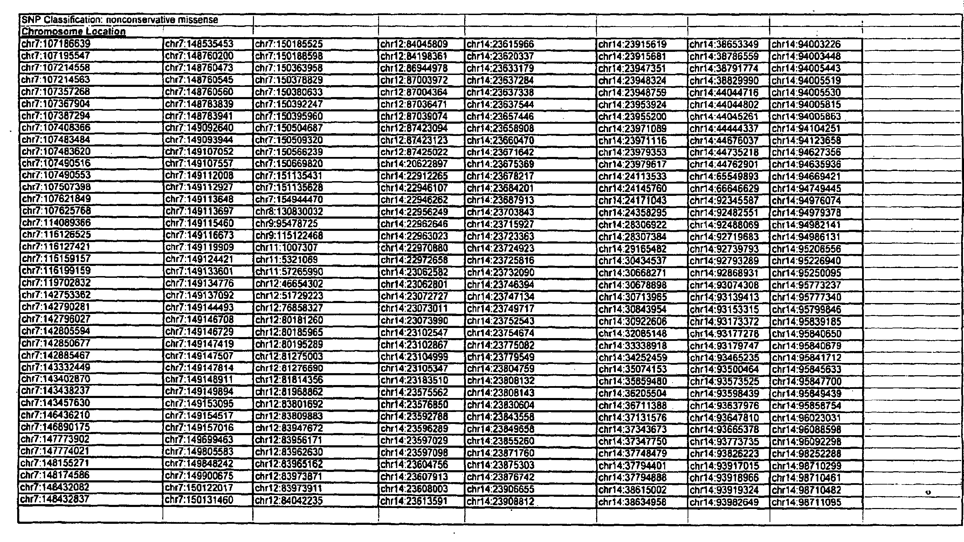

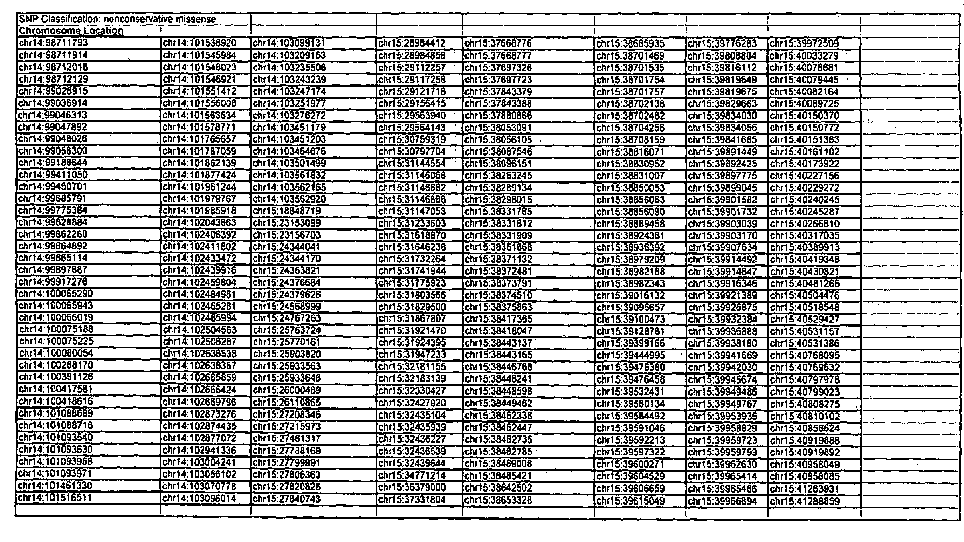

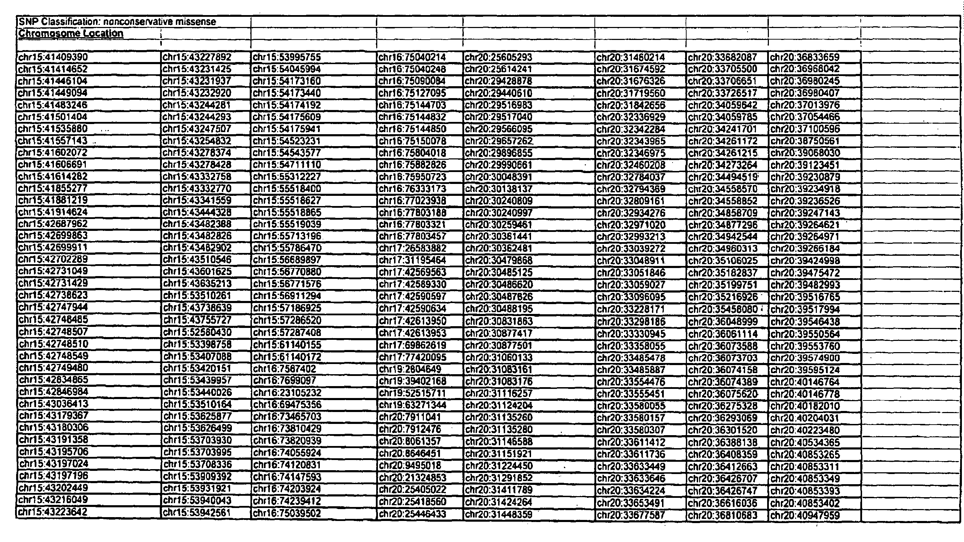

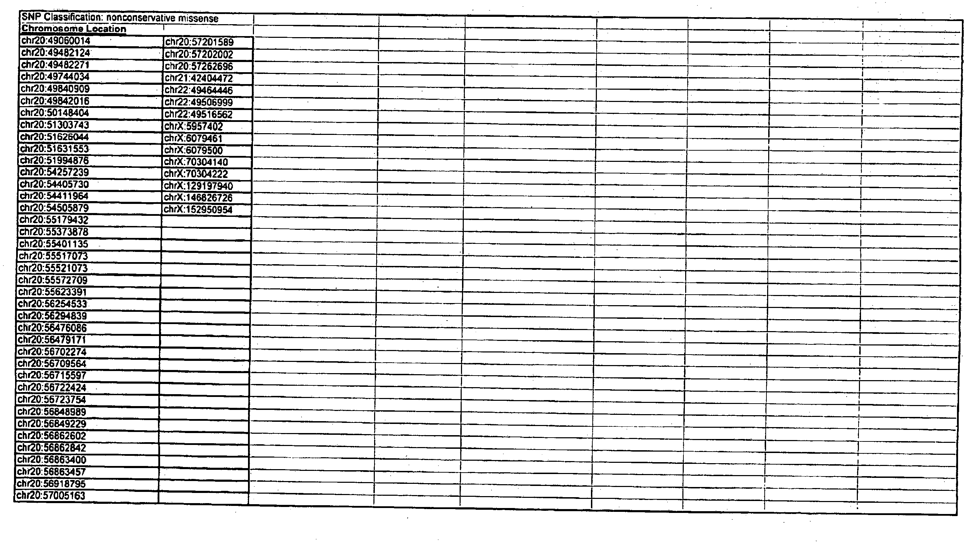

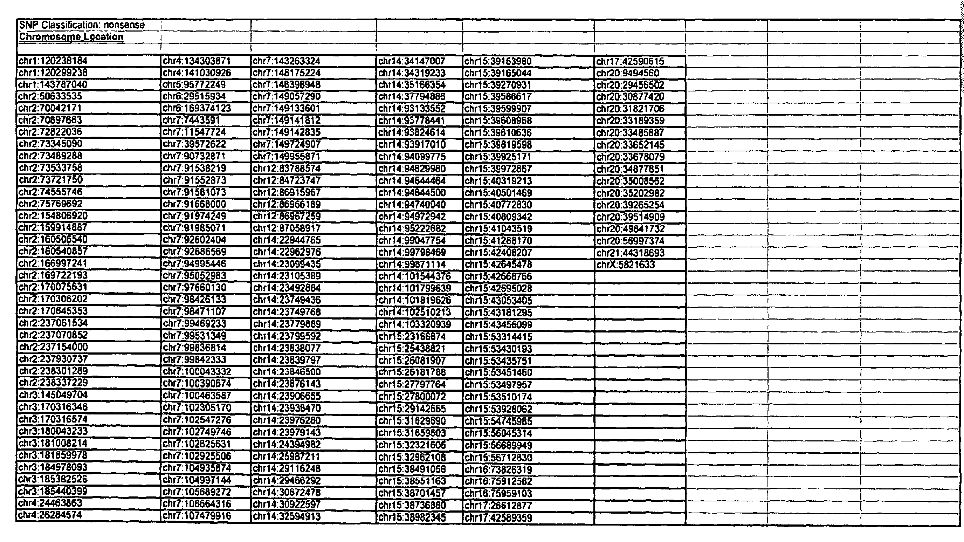

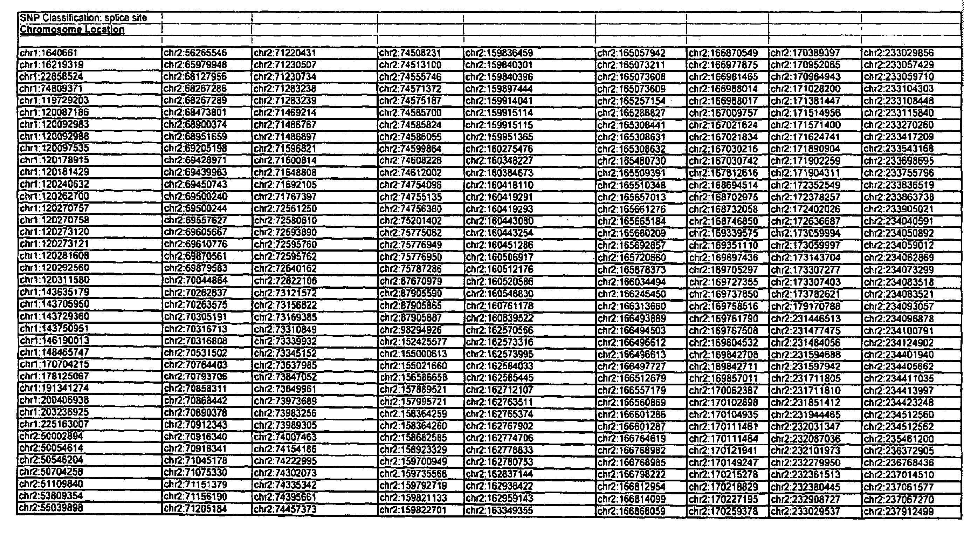

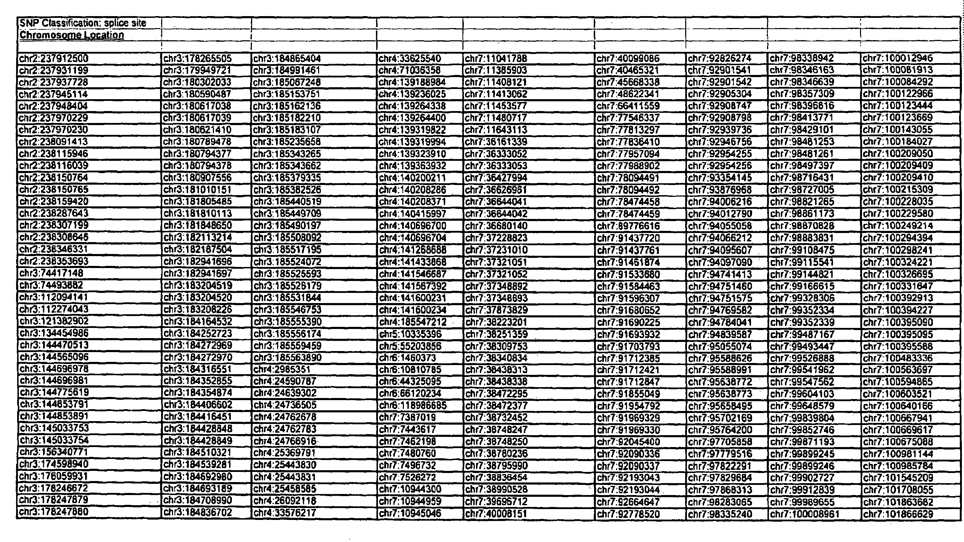

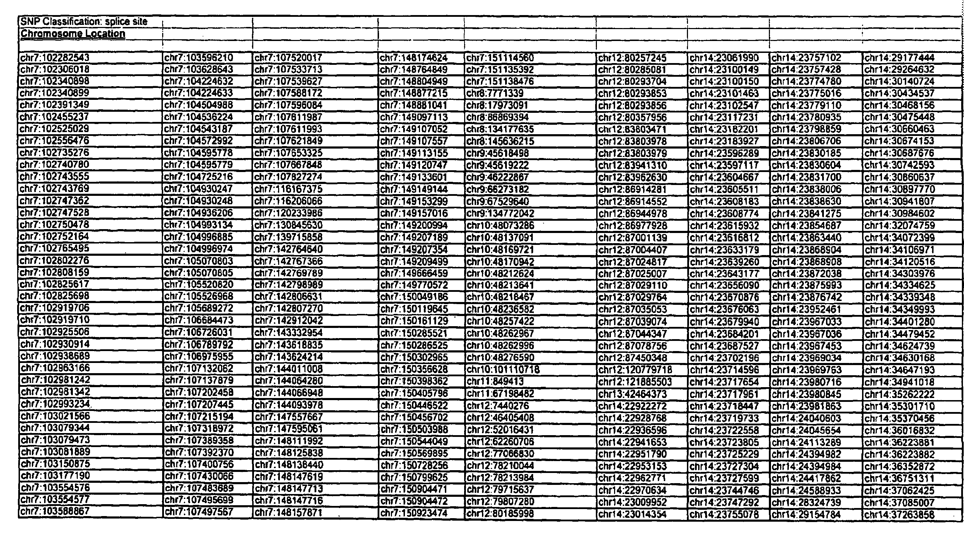

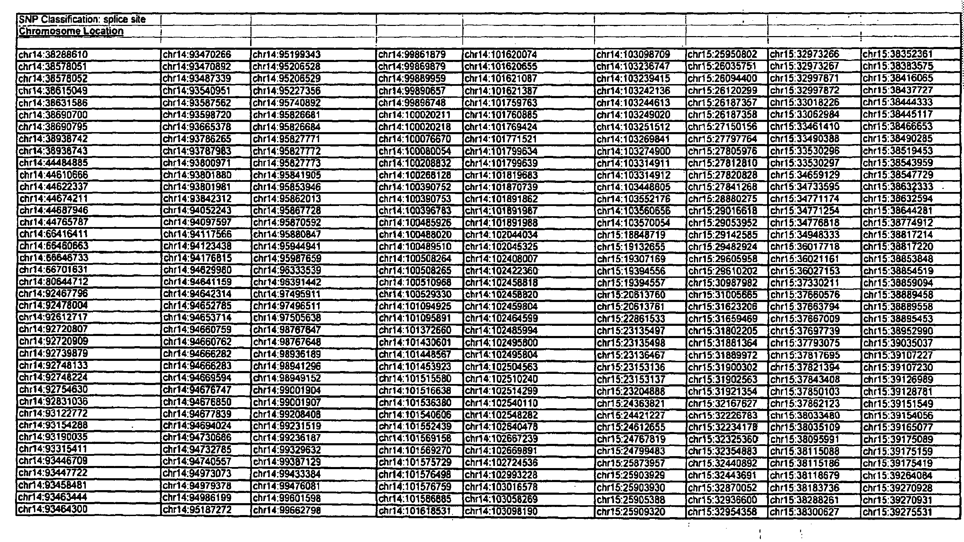

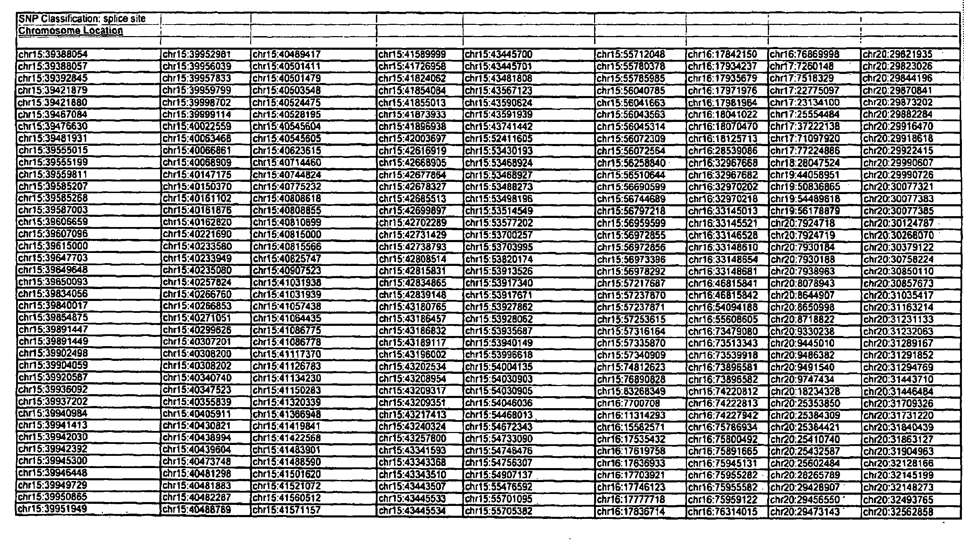

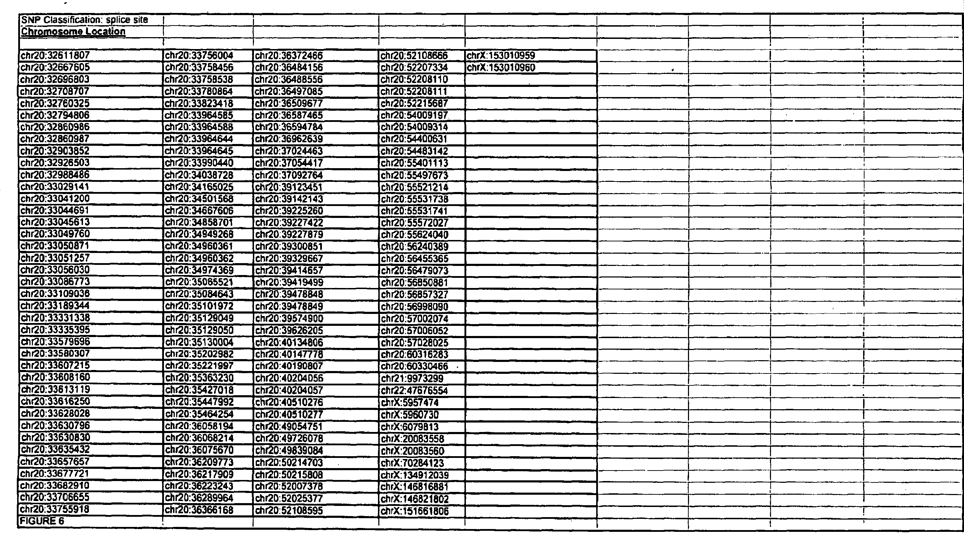

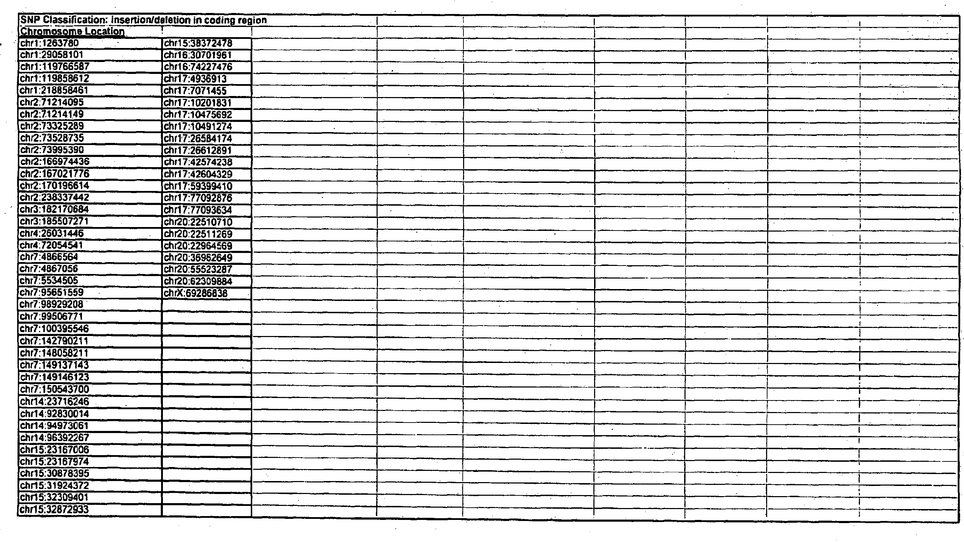

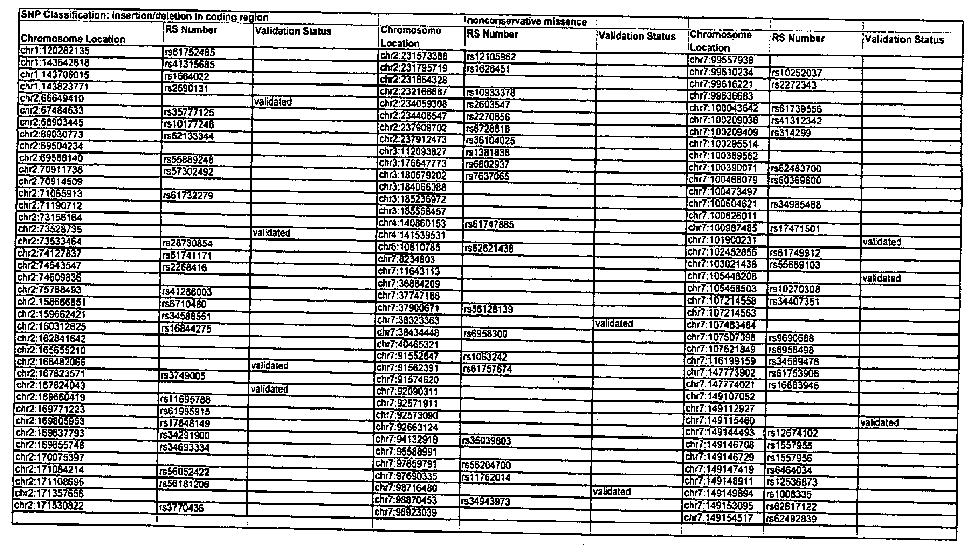

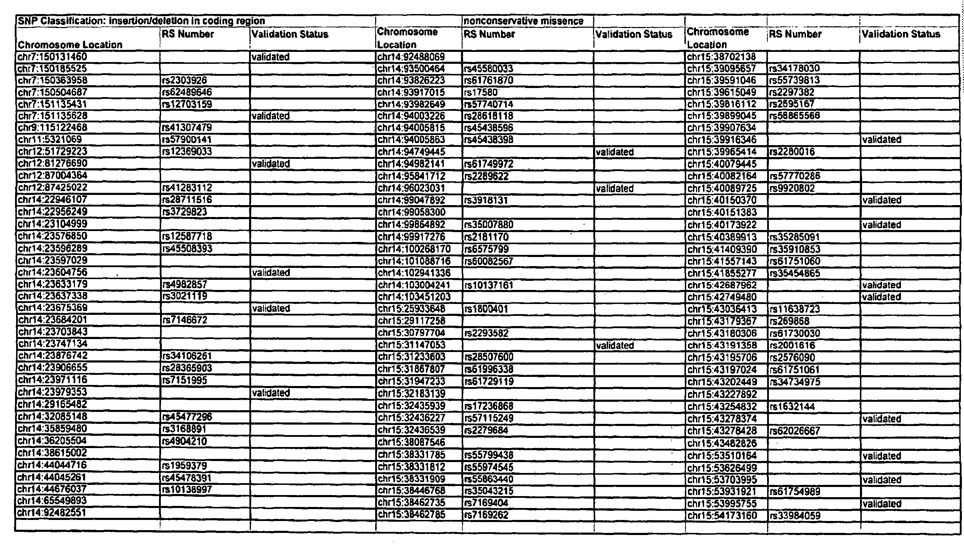

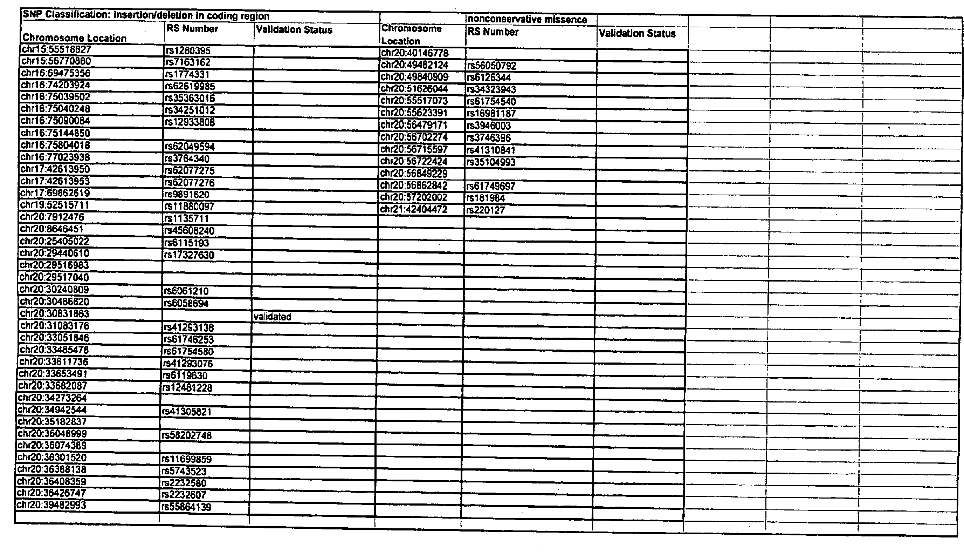

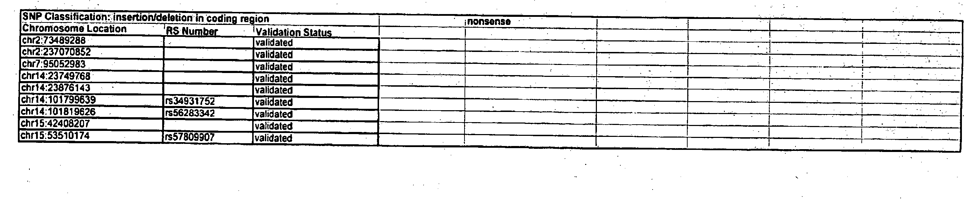

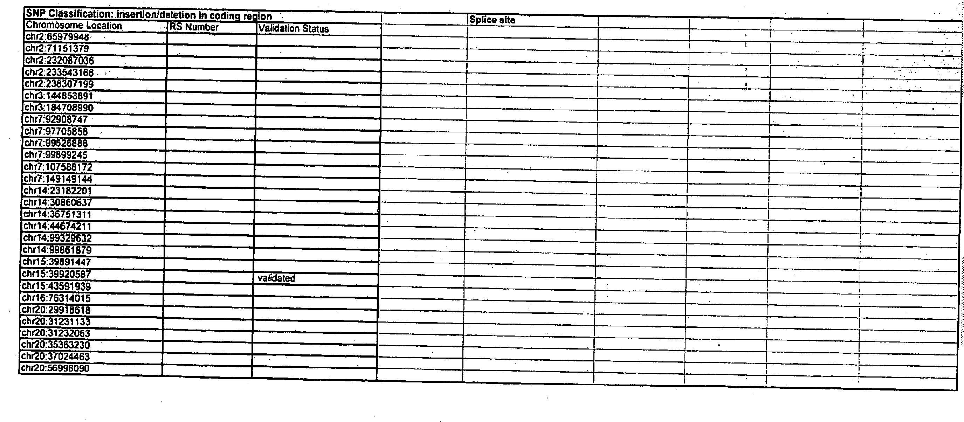

In another embodiment, a sample collected from an individual may be assayed for the presence of one or more SNPs from FIG. 5, wherein the presence of one or more of the SNPs from FIG. 5 may indicate whether an individual is affected with ASD or may be predisposed to ASD. In one such embodiment, a nucleotide sample may be collected from an individual and one or more of the SNPs from FIG. 5 may be detected using genetic analysis of the nucleotide sample, wherein the detection of the one or more SNPs from FIG. 5 may indicate whether an individual is affected with ASD or may be predisposed to ASD.

In one embodiment, the genetic marker associated with autism or ASD may be one or several SNP(s) or a haplotype of SNPs associated with autism or ASD. In one embodiment the SNP(s) may be selected from those SNP(s) located in any region of any chromosome that shows association with one or more autism phenotypes. In one such embodiment, the SNPs may be selected from one or more of rs792065, rs1570056, rs1990790, rs1419437, rs6490970, rs8033248, rs723049, rs11856, rs383902, rs725463. In another embodiment, the SNPs may be selected from one or more SNP at the following chromosome locations: chr1:1263780, chr1:29058101, chr1:119766587, chr1:119858612, chr1:218858461, chr2:71214095, chr2:71214149, chr2:73325289, chr2:73528735, chr2:73995390, chr2:166974436, chr2:167021776, chr2:170196614, chr2:238337442, chr3:182170684, chr3:185507271, chr4:26031446, chr4:72054541, chr7:4866564, chr7:4867056, chr7:5534505, chr7:95651559, chr7:98929208, chr7:99506771, chr7:100395546, chr7:142790211, chr7:148058211, chr7:149137143, chr7:149146123, chr7:150543700, chr14:23716246, chr14:92830014, chr14:94973061, chr14:96392267, chr15:23167006, chr15:23167974, chr15:30878395, chr15:31924372, chr15:32309401, chr15:32872933, chr15:38372478, chr16:30701961, chr16:74227476, chr17:4936913, chr17:7071455, chr17:10201831, chr17:10475692, chr17:10491274, chr17:26584174, chr17:26612891, chr17:42574238, chr17:42604329, chr17:59399410, chr17:77092876, chr17:77093634, chr20:22510710, chr20:22511269, chr20:22964569, chr20:36962649, chr20:40146764, chr20:55523287, chr20:62309884, chrX:69286838, chr1:120282135, chr1:143642818, chr1:143706015, chr1:143823771, chr2:66649410, chr2:67484633, chr2:68903445, chr2:69030773, chr2:69504234, chr2:69588140, chr2:70911738, chr2:70.914509, chr2:71065913, chr2:71190712, chr2:73156164, chr2:73528735, chr2:73533464, chr2:74127837, chr2:74543547, chr2:74609836, chr2:75768493, chr2:158666851, chr2:159662421, chr2:160312625, chr2:162841642, chr2:165655210, chr2:166482066, chr2:167823571, chr2:167824043, chr2:169660419, chr2:169771223, chr2:169805953, chr2:169837793, chr2:169855748, chr2:170075397, chr2:171084214, chr2:171108695, chr2:171357656, chr2:171530822, chr2:231573388, chr2:231795719, chr2:231864328, chr2:232166687, chr2:234059308, chr2:234406547, chr2:237909702, chr2:237912473, chr3:112093827, chr3:176647773, chr3:180579202, chr3:184066088, chr3:185236972, chr3:185558457, chr4:140860153, chr4:141539531, chr6:10810785, chr7:8234803, chr7:11643113, chr7:36884209, chr7:37747188, chr7:37900671, chr7:38323363, chr7:38434448, chr7:40465321, chr7:91552847, chr7:91562391, chr7:91574620, chr7:92090311, chr7:92571911, chr7:92573090, chr7:92663124, chr7:94132918, chr7:95588991, chr7:97659791, chr7:97690335, chr7:98716480, chr7:98870453, chr7:98923039, chr7:99557938, chr7:99610234, chr7:99616221, chr7:99636683, chr7:100043642, chr7:100209036, chr7:100209409, chr7:100295514, chr7:100389562, chr7:100390071, chr7:100468079, chr7:100473497, chr7:100604621, chr7:100626011, chr7:100987485, chr7:101900231, chr7:102452856, chr7:103021438, chr7:105448208, chr7:105458503, chr7:107214558, chr7:107214563, chr7:107483484, chr7:107507398, chr7:107621849, chr7:116199159, chr7:147773902, chr7:147774021, chr7:149107052, chr7:149112927, chr7:149115460, chr7:149144493, chr7:149146708, chr7:149146729, chr7:149147419, chr7:149148911, chr7:149149894, chr7:149153095, chr7:149154517, chr7:150131460, chr7:150185525, chr7:150363958, chr7:150504687, chr7:151135431, chr7:151135628, chr9:115122468, chr11:5321069, chr12:51729223, chr12:81276690, chr12:87004364, chr12:87425022, chr14:22946107, chr14:22956249, chr14:23104999, chr14:23576850, chr14:23596289, chr14:23597029, chr14:23604756, chr14:23633179, chr14:23637338, chr14:23675369, chr14:23684201, chr14:23703843, chr14:23747134, chr14:23876742, chr14:23906655, chr14:23971116, chr14:23979353, chr14:29165482, chr14:32085148, chr14:35859480, chr14:36205504, chr14:38615002, chr14:44044716, chr14:44045261, chr14:44676037, chr14:65549893. chr14:92482551, chr14:92488069, chr14:93500464, chr14:93826223, chr14:93917015, chr14:93982649, chr14:94003226, chr14:94005815, chr14:94005863, chr14:94749445, chr14:94982141, chr14:95841712, chr14:96023031, chr14:99047892, chr14:99058300, chr14:99864892, chr14:99917276, chr14:100268170, chr14:101088716, chr14:102941336, chr14:103004241, chr14:103451203, chr15:25933648, chr15:29117258, chr15:30797704, chr15:31147053, chr15:31233603, chr15:31867807, chr15:31947233, chr15:32183139, chr15:32435939, chr15:32436227, chr15:32436539, chr15:38087546, chr15:38331785, chr15:38331812, chr15:38331909, chr15:38446768, chr15:38462735, chr15:38462785, chr15:38702138, chr15:39095657, chr15:39591046, chr15:39615049, chr15:39816112, chr15:39899045, chr15:39907634, chr15:39916346, chr15:39965414, chr15:40079445, chr15:40082164, chr15:40089725, chr15:40150370, chr15:40151383, chr15:40173922, chr15:40389913, chr15:41409390, chr15:41557143, chr15:41855277, chr15:42687962, chr15:42749480, chr15:43036413, chr15:43179367, chr15:43180306, chr15:43191358, chr15:43195706, chr15:43197024, chr15:43202449, chr15:43227892, chr15:43254832, chr15:43278374, chr15:43278428, chr15:43482826, chr15:53510164, chr15:53626499, chr15:53703995, chr15:53931921, chr15:53995755, chr15:54173160, chr15:55518627, chr15:56770880, chr16:69475356, chr16:74203924, chr16:75039502, chr16:75040248, chr16:75090084, chr16:75144850, chr16:75804018, chr16:77023938, chr17:42613950, chr17:42613953, chr17:69862619, chr19:52515711, chr20:7912476, chr20:8646451, chr20:25405022, chr20:29440610, chr20:29516983, chr20:29517040, chr20:30240809, chr20:30486620, chr20:30831863, chr20:31083176, chr20:33051846, chr20:33485478, chr20:33611736, chr20:33653491, chr20:33682087, chr20:34273264, chr20:34942544, chr20:35182837, chr20:36048999, chr20:36074389, chr20:36301520, chr20:36388138, chr20:36408359, chr20:36426747, chr20:39482993, chr20:40146778, chr20:49482124, chr20:49840909, chr20:51626044, chr20:55517073, chr20:55623391, chr20:56479171, chr20:56702274, chr20:56715597, chr20:56722424, chr20:56849229, chr20:56862842, chr20:57202002, chr21:42404472, chr2:73489288, chr2:237070852, chr7:95052983, chr14:23749768, chr14:23876143, chr14:101799639, chr14:101819626, chr15:42408207, chr15:53510174, chr2:65979948, chr2:71151379, chr2:232087036, chr2:233543168, chr2:238307199, chr3:144853891, chr3:184708990, chr7:92908747, chr7:97705858, chr7:99526888, chr7:99899245, chr7:107588172, chr7:149149144, chr14:23182201, chr14:30860637, chr14:36751311, chr14:44674211, chr14:99329632, chr14:99861879, chr15:39891447, chr15:39920587, chr15:43591939, chr16:76314015, chr20:29918618, chr20:31231133, chr20:31232063, chr20:35363230, chr20:37024463, and chr20:56998090.

In one embodiment, the genetic markers associated with autism or an ASD may be selected from the group of markers that may be in LD with alleles or loci that may associated with autism. In one embodiment, a genetic marker may be in LD with a chromosome location on any one of human chromosomes 1-22 and the X and the Y chromosomes. In one such embodiment, the genetic marker(s) may be selected from genetic markers in LD with human chromosome location 2p25.3-p24.1, 6q22.32-q24.1, 7q31.31-q32.3, 7q31.31-q32.3, 13q12.11-q12.3, 15q13.1-q14, 15q14-q21.1, 15q21.2-q22.1, 15q21.1-q22.2, or combinations thereof. In another such embodiment, the genetic marker(s) may be selected from genetic markers in LD with one or more of human chromosome locations 1p12, 1q21, 2p14, 2q23-q31, 2q37, 3q13, 3q26-q27, 4p15, 4q28-q31, 7p21, 7p14, 7q21-q31, 7q31, 7q35-36, 12q21, 12q21, 14q11-q21, 14q32, 15q11, 15q12-q21, 15q21-q22, 16q22-23, 20p12, 20p11-q13 and 20q13.

In yet another embodiment, the one or more genetic markers may include genetic markers in LD with genes of interest. In one embodiment, the genetic markers may be in LD with autism susceptibility genes. In one such embodiment, the genetic marker(s) may be in LD with genes located in chromosome 15 such as ubiquitin protein ligase E3A, UBEA, GABA-A receptor, and GABRB3. In another embodiment, the genetic markers may located at, or in LD with, chromosome 15 regions with boundaries of 27,440,000 bp - 32,790,000 bp; 32,790,000 bp-43,260,000 bp; and 50,770,000 bp - 56,800,000 bp. In another such embodiment, the genetic markers used according to the methods disclosed herein may be in LD, or associated with, neuroligins, neurexins, contactin associated protein (CNTNAP2), serotonin transporter (SLC6A4), Engrailed 2 (EN2), reelin (RELN), Ca+ - dependent activator protein for secretion 2 (CADPS2), met proto-oncogene (MET), neurobeachin gene (NBEAL2) and oxytocin receptor (OXTR).

In still another embodiment, the genetic marker(s) may be associated with or in LD with one or more genes of interest such as NOTCH2, NRXN1, C2orf32 (CNRIP1), AAK1, SCN7A, CNTN3, NHE9 (SLC9A9), DIA1 (c3orf58), NLGN1, KCNMB2, KCNMB3, FXR1, PCDH7, BC036345, PCDH10, RNF8, MAGI2MET, KCND2, CNTNAP2, EN2, NPAS3, GEPH, M84131, Prader-Willi/Angelman (NIPA1), UBE3A, GABRB3, GABRA5, GABRG3, CHRNA7, SCG5, RYR3, GPR176, DYX1C1, PYGO1, NEDD4, Gcom1, GRINL1A, ALDH1A2, ADAM10, HSP90Bd, A2BP1, SLC6A4, EPB41L1, DLGAP4, NNAT, SLC32A1, PPP1R16B, PTPRT, CBLN4, SHANK3, NLGN4X, NLGN3, NHE6 (SLC9A6), FMR1, MECP2 and NLGN4Y.

In one embodiment, one or more diagnostic and predictive markers associated with ASD may be selected from a group of genetic markers including cytogenetic abnormalities such as structural genomic changes like DNA copy number changes or CNVs. In one embodiment, CNVs may include deletions, insertions, inversions, and duplications within one or more chromosomes of an individual.

In one embodiment, methods for identifying individuals affected by ASD or at risk of developing ASD are provided. In one embodiment, the methods may comprise collecting a genetic sample, such as a nucleotide sample, from an individual and assaying the nucleotide sample in order to detect the presence of one or more CNVs, including DNA deletions, DNA duplications, DNA translocations, and DNA inversions, that may be associated with ASD and wherein, the presence of certain CNVs indicate that the individual is affected with ASD, or is at an increased risk of ASD, or is predisposed to develop ASD. In another such embodiment, the methods may comprise collecting a genetic sample from an individual and assaying the genetic sample in order to detect and identify genomic regions that have CNVs, such as genomic regions with fewer than two or more than two genomic copies. In one embodiment, the methods disclosed herein may comprise collecting a genetic sample, purifying the genetic sample, and assaying the purified genetic sample for cytogenetic abnormalities such as structural genomic changes like DNA copy number changes or CNVs. In another embodiment, the methods disclosed herein may comprise collecting a genetic sample, purifying the genetic sample, and amplifying at least a portion of the purified genetic sample, and assaying the amplified genetic sample for CNVs.

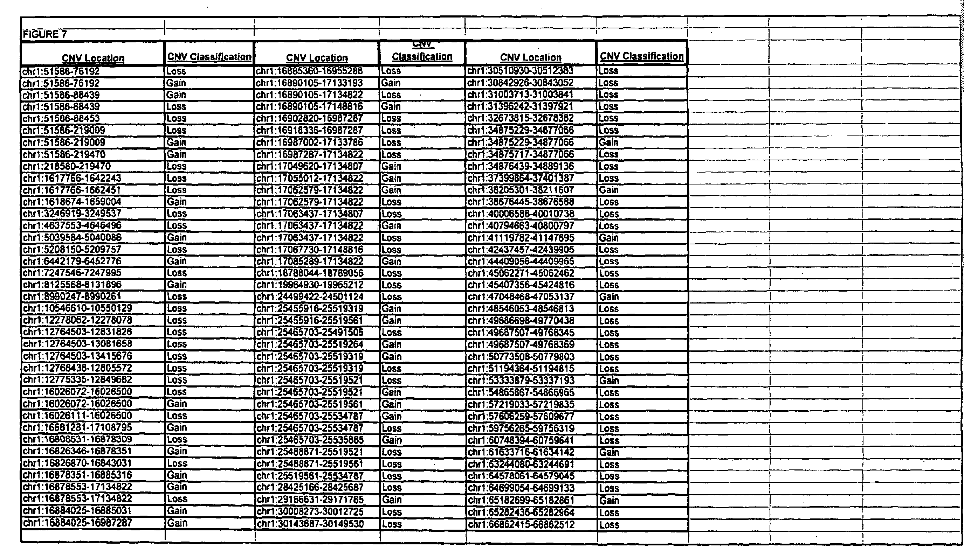

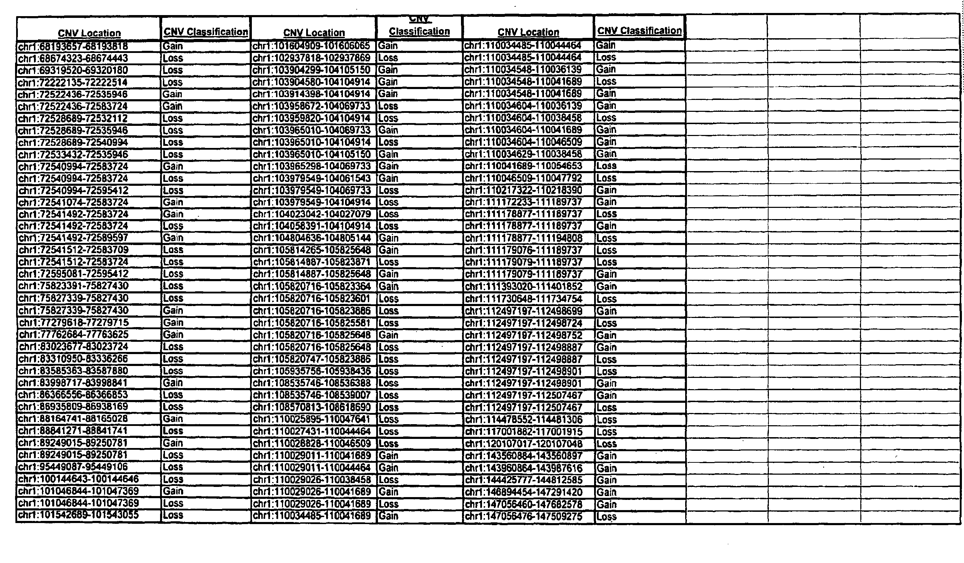

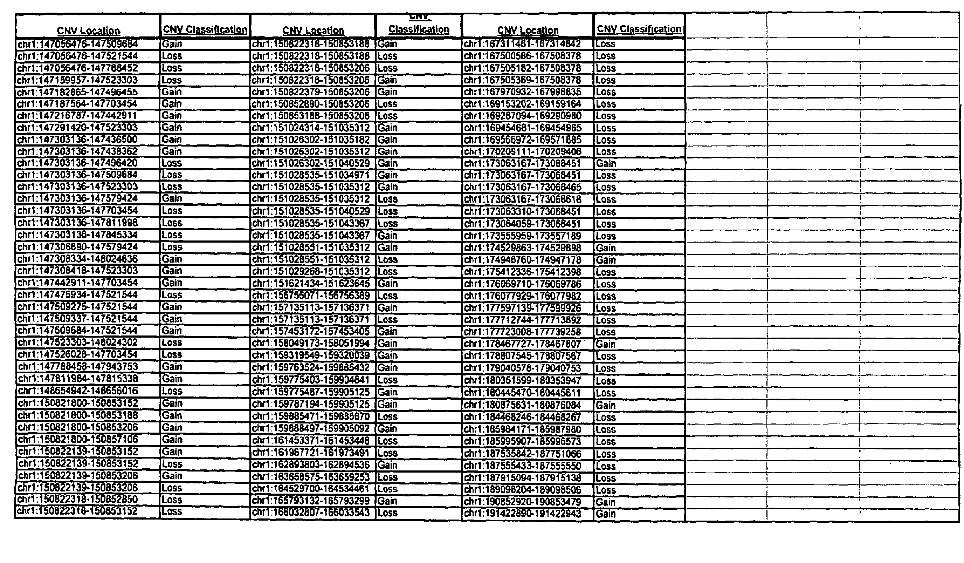

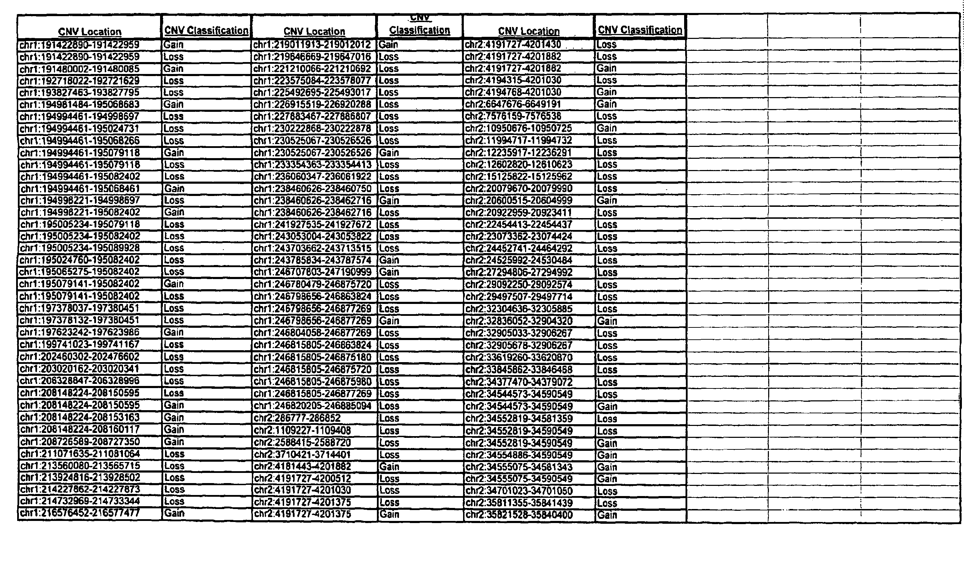

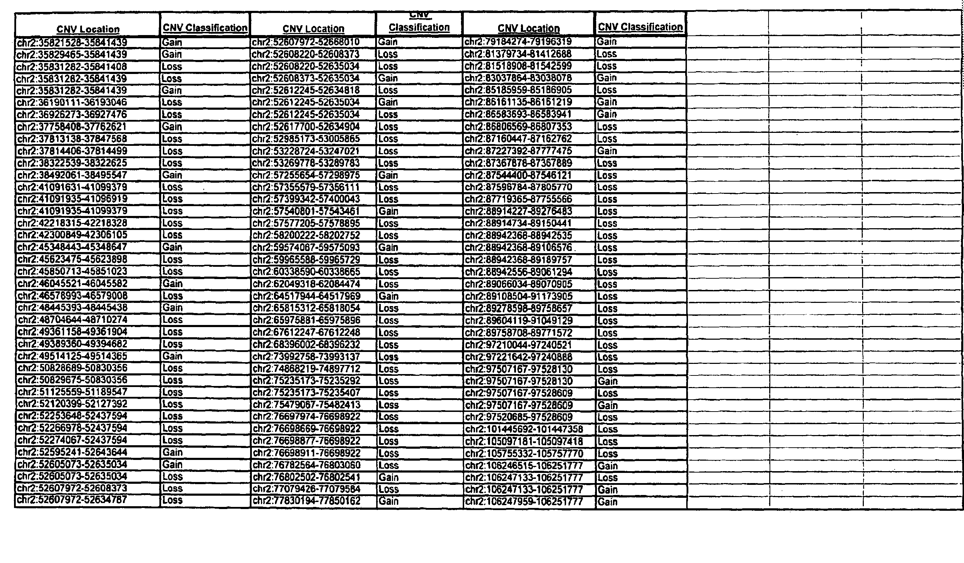

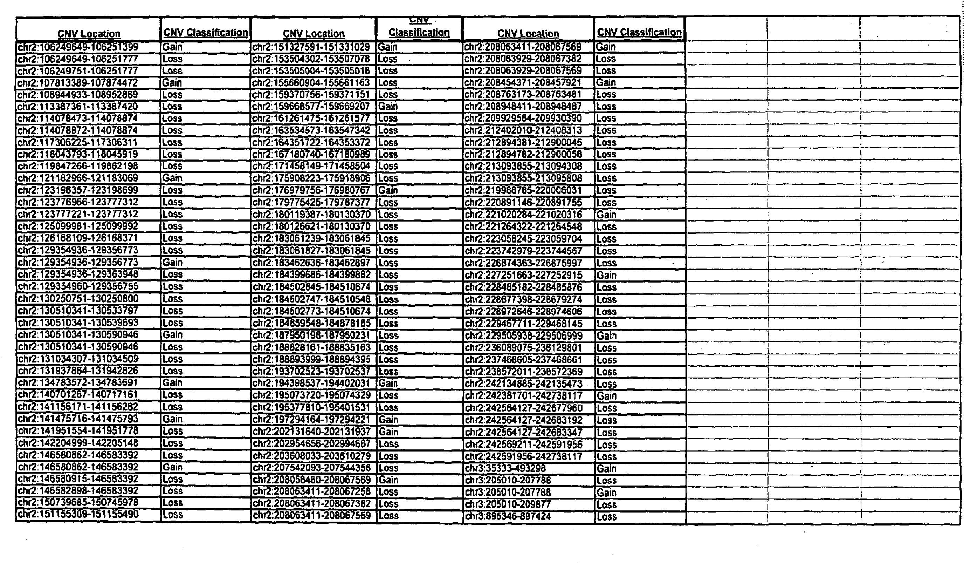

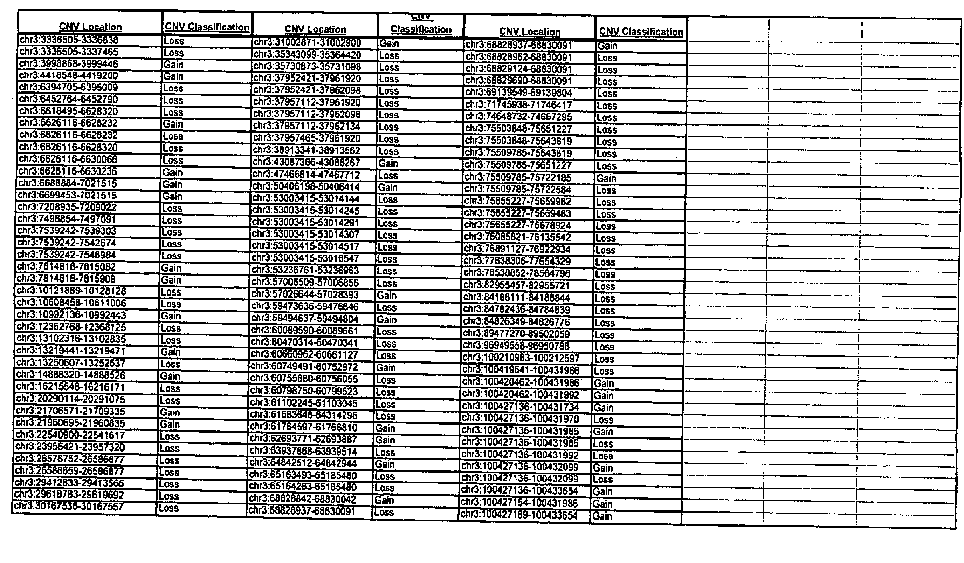

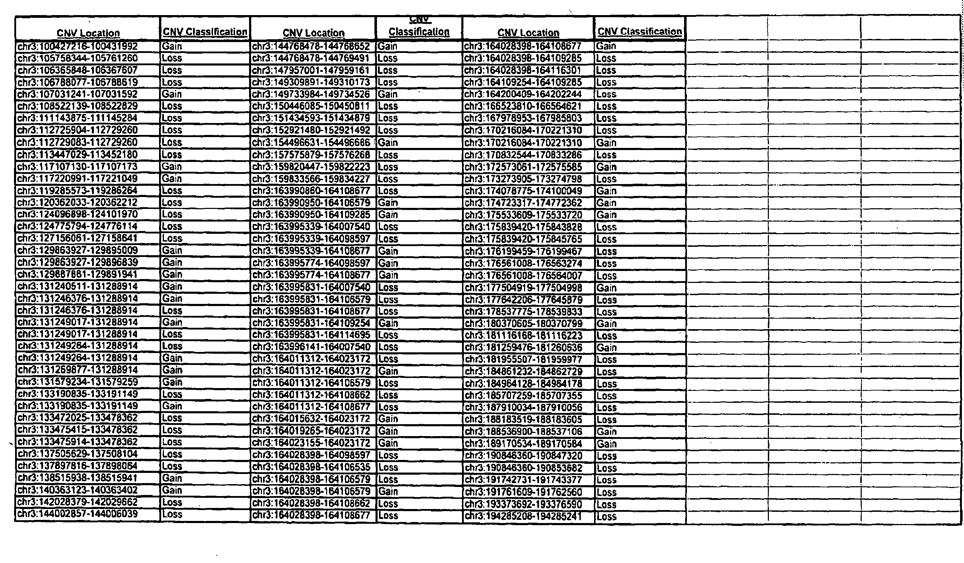

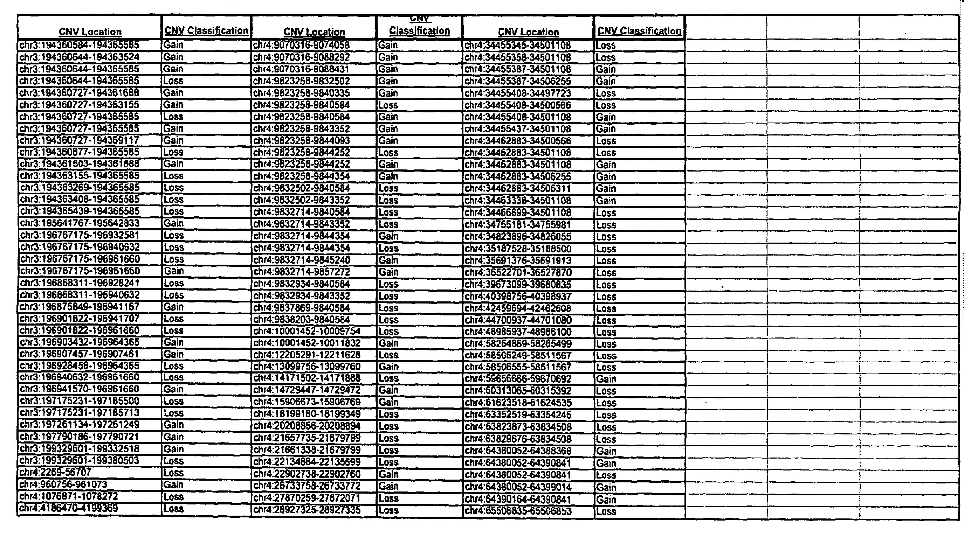

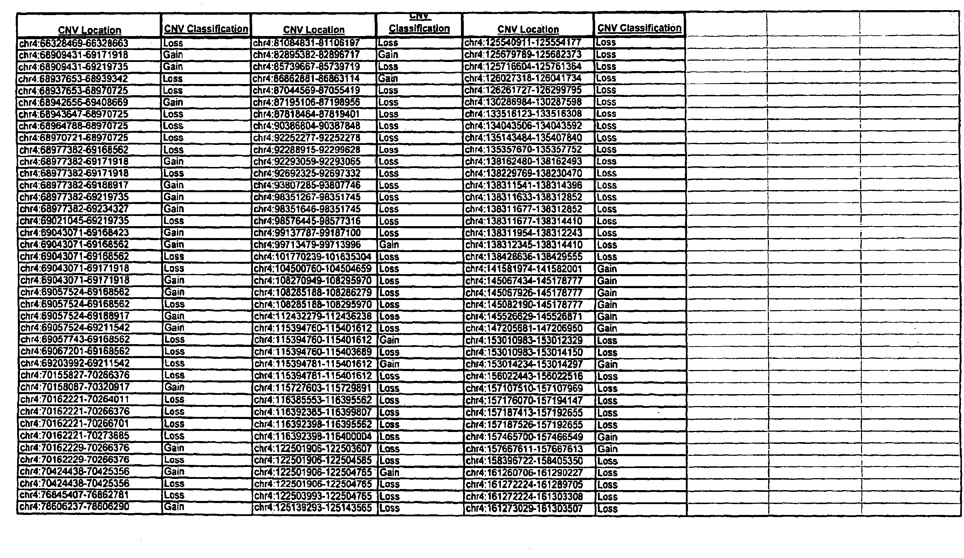

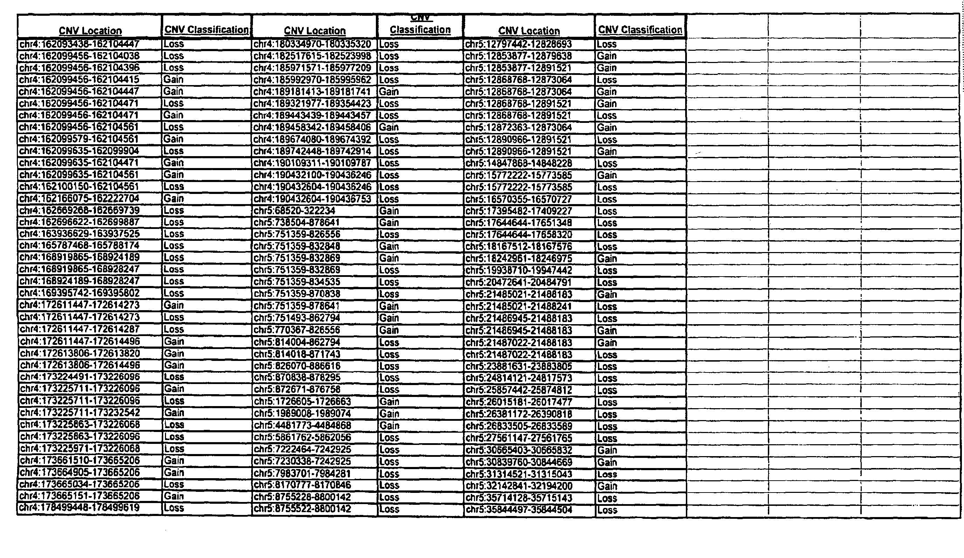

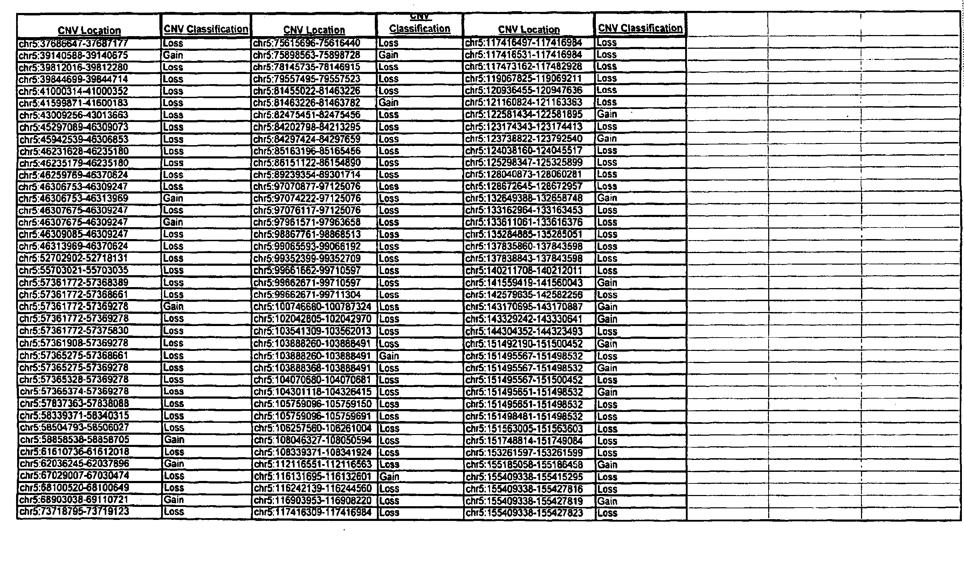

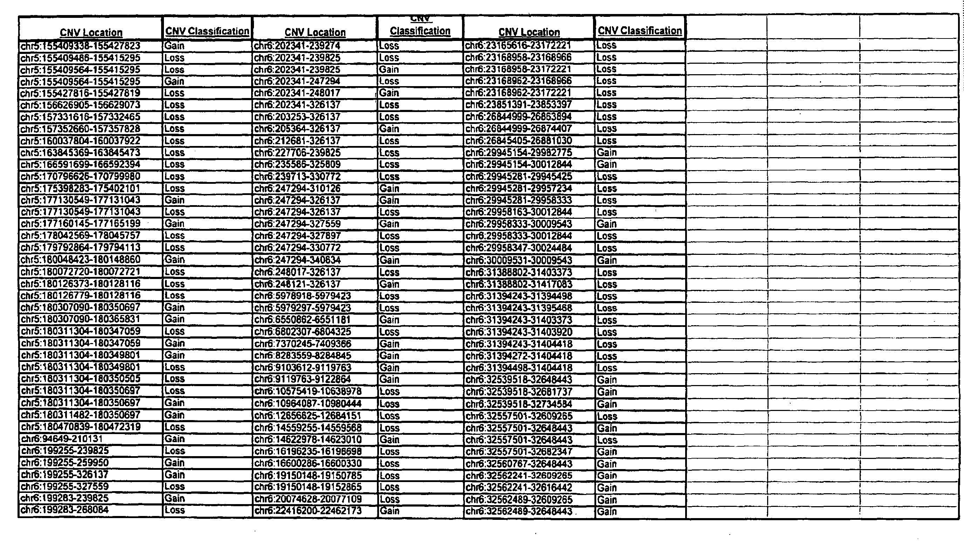

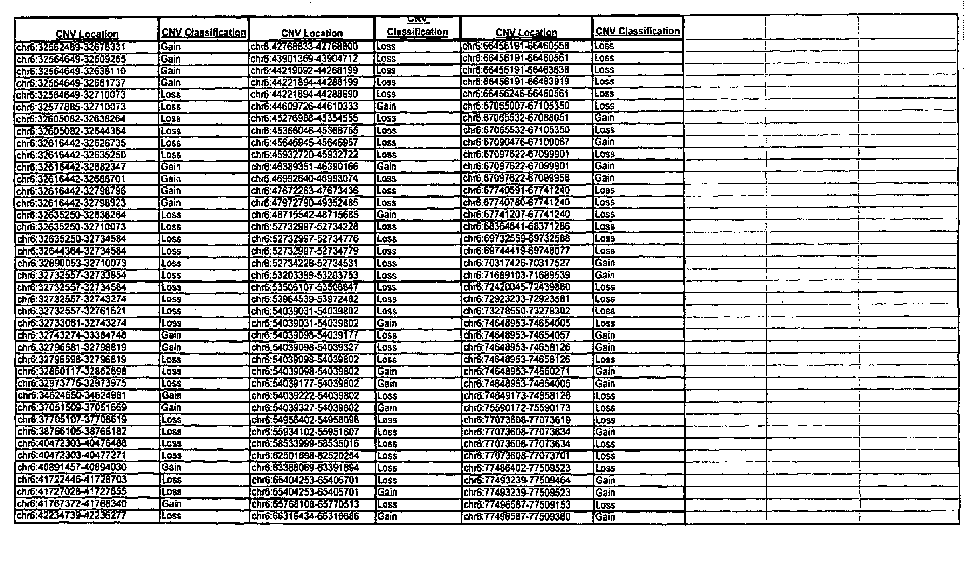

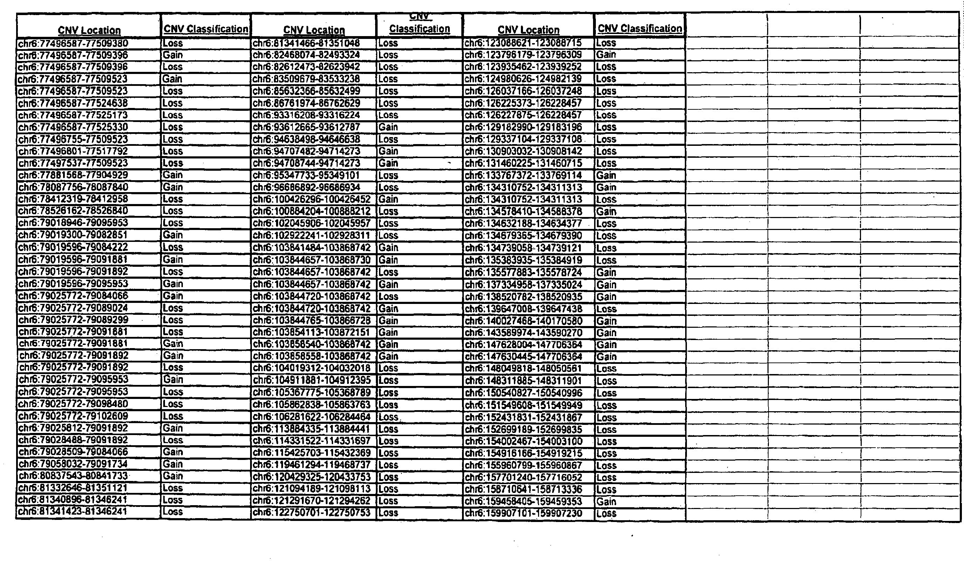

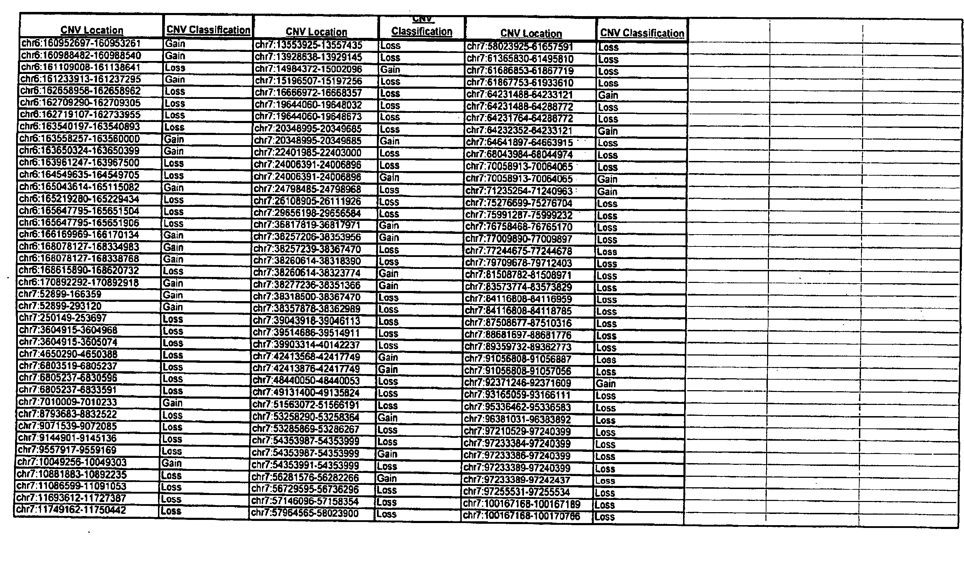

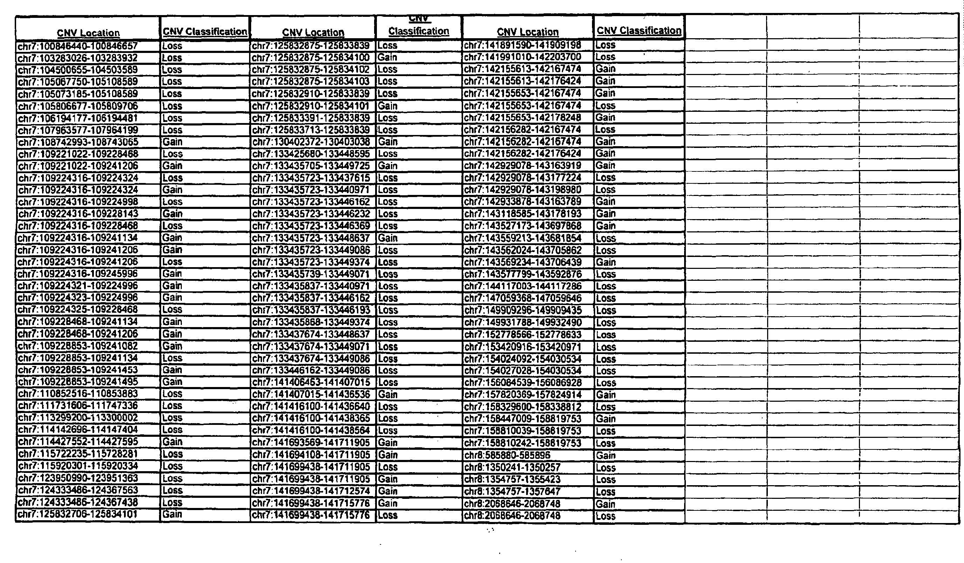

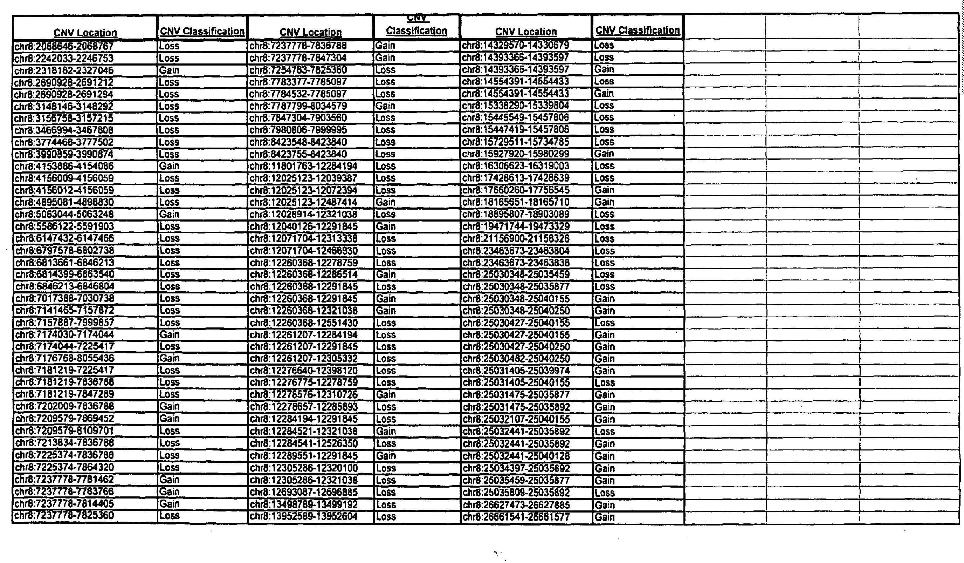

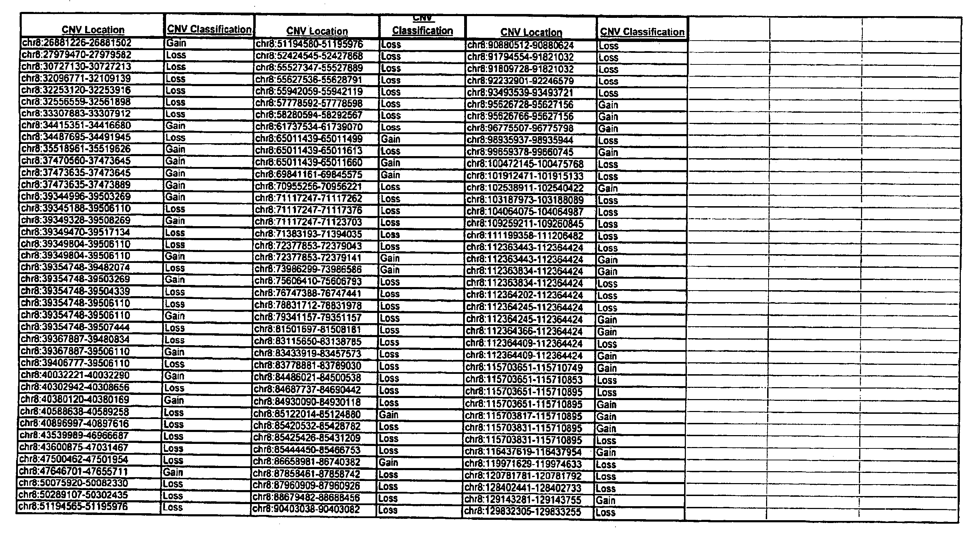

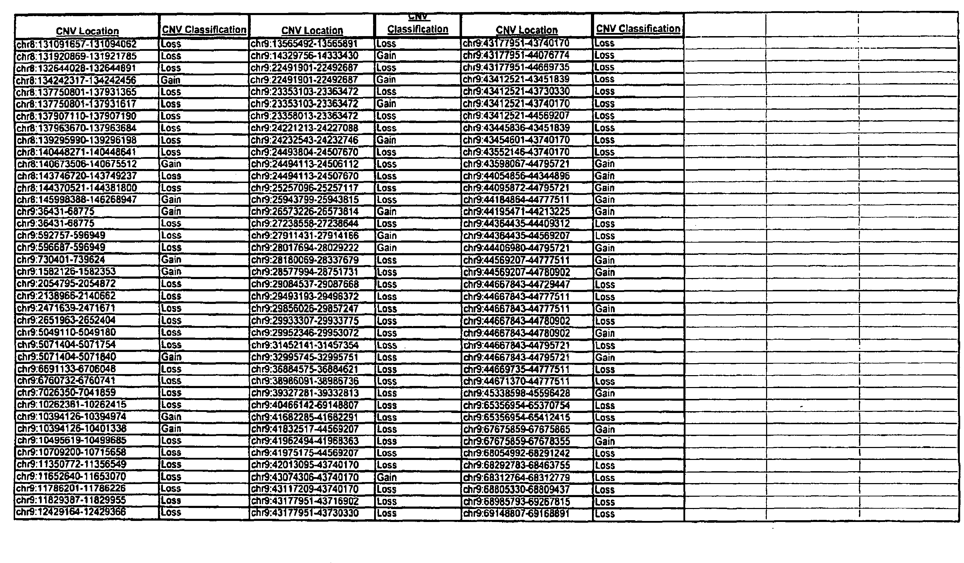

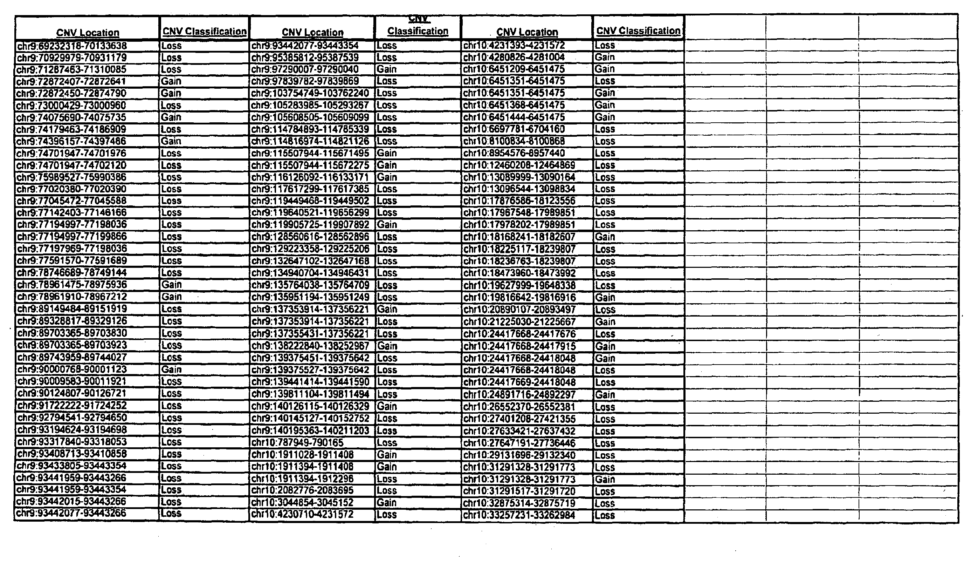

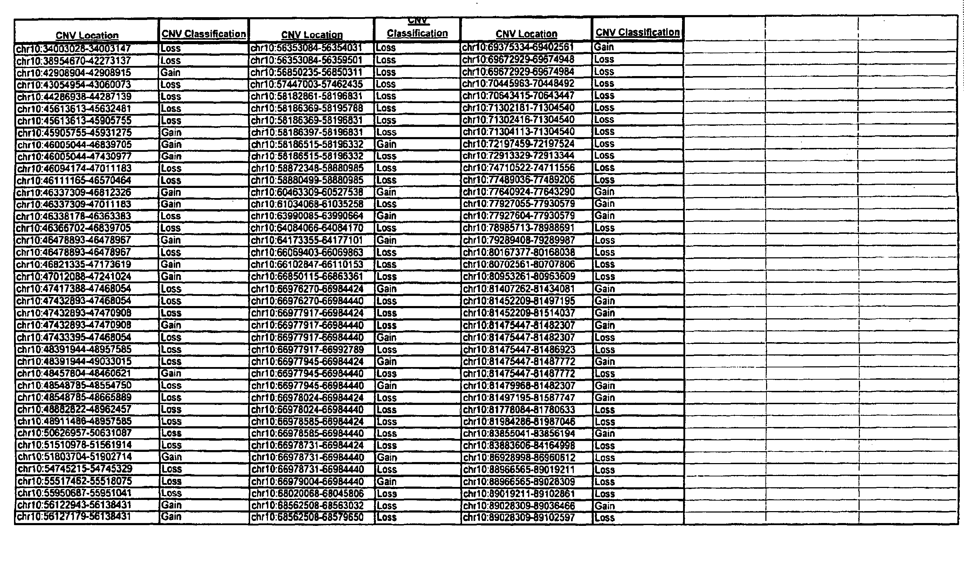

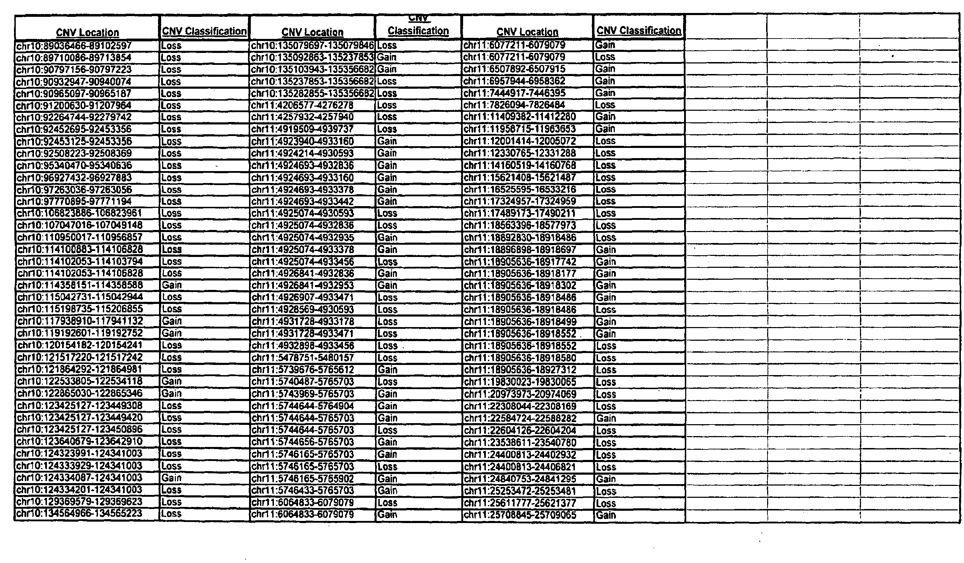

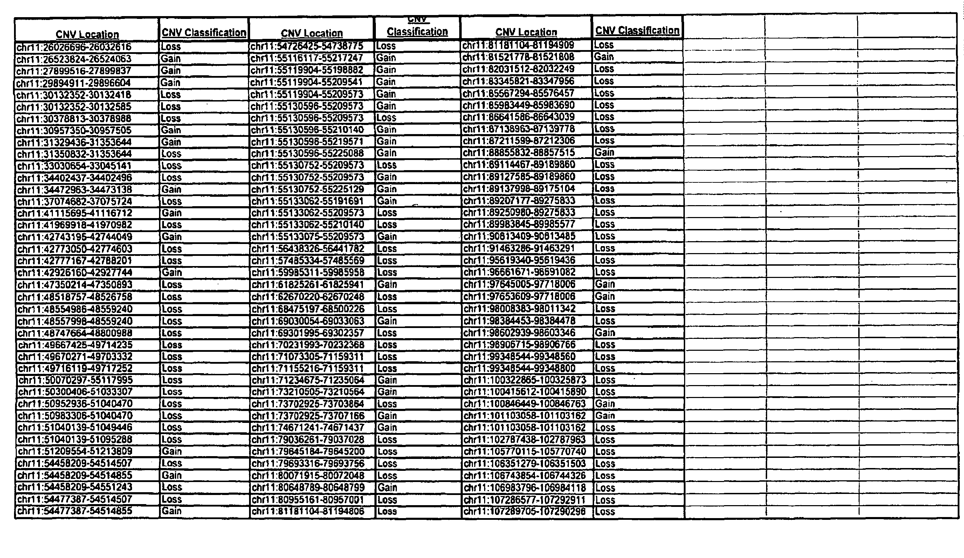

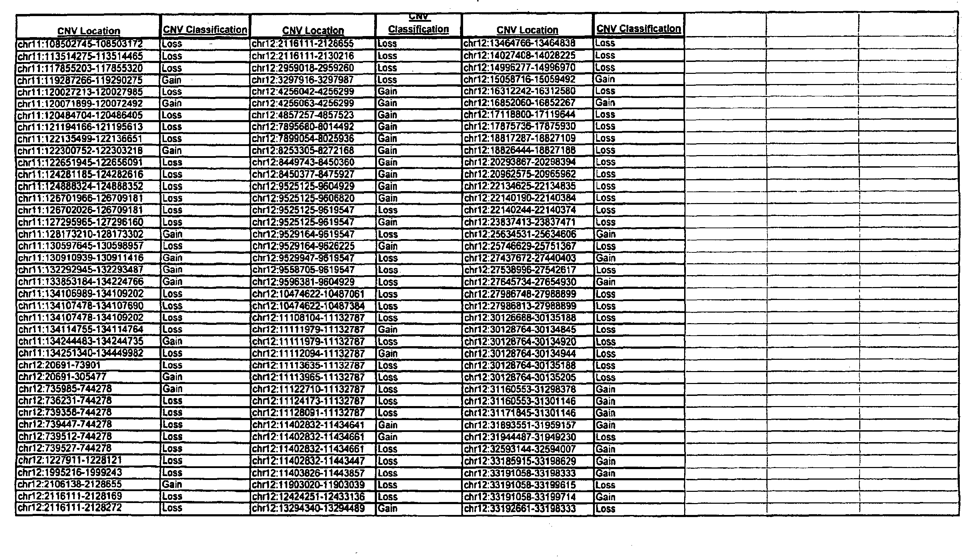

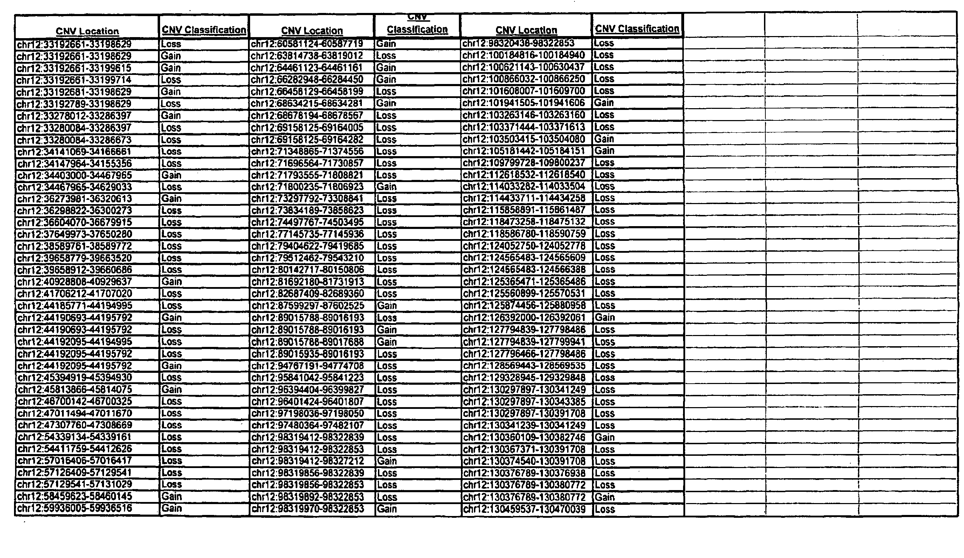

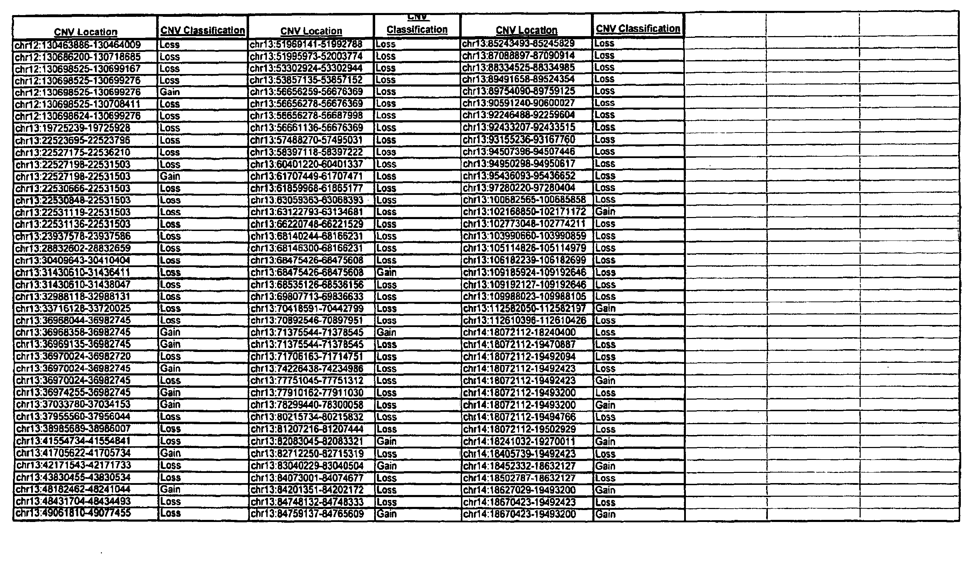

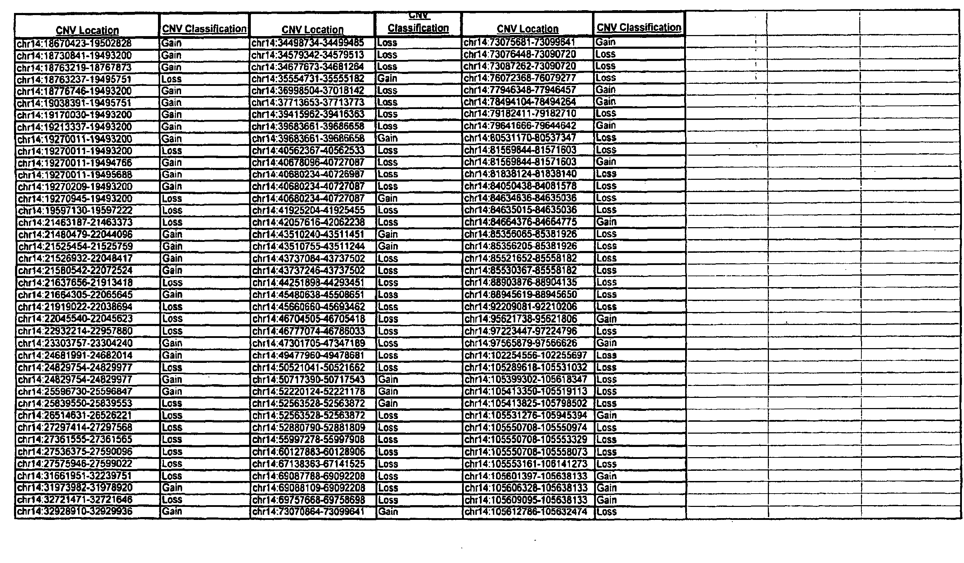

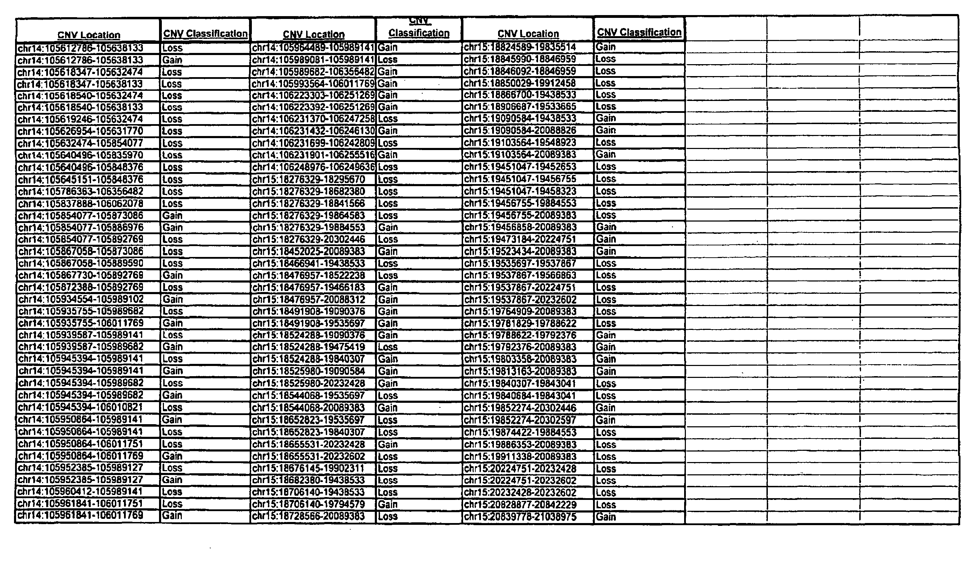

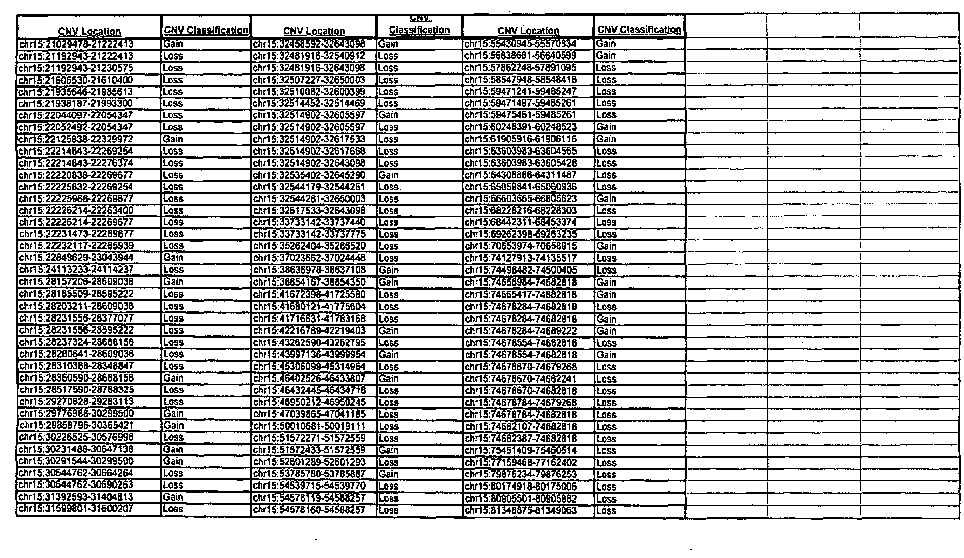

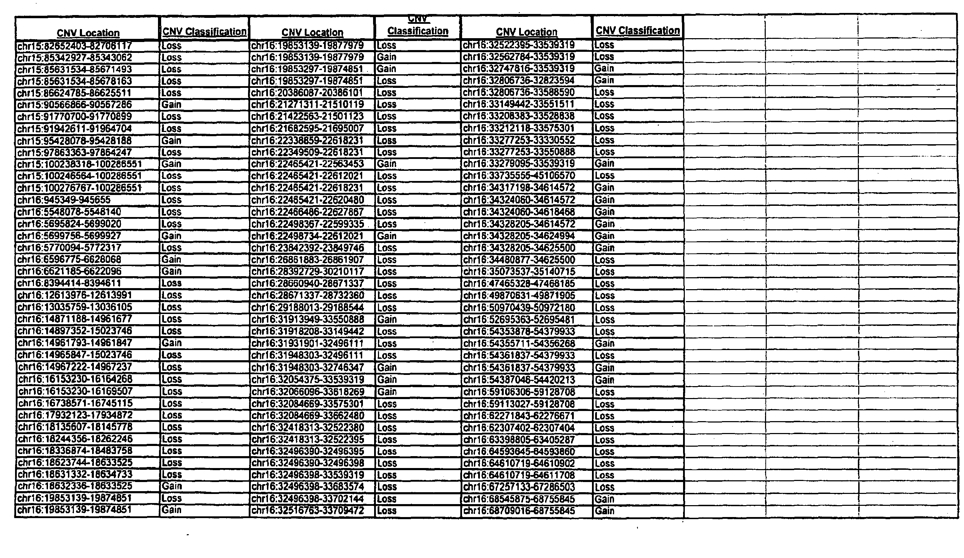

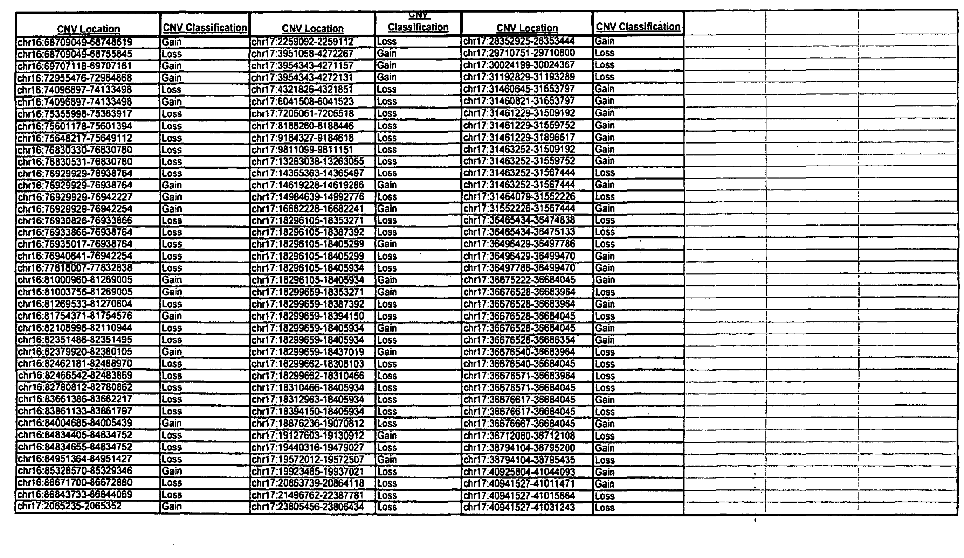

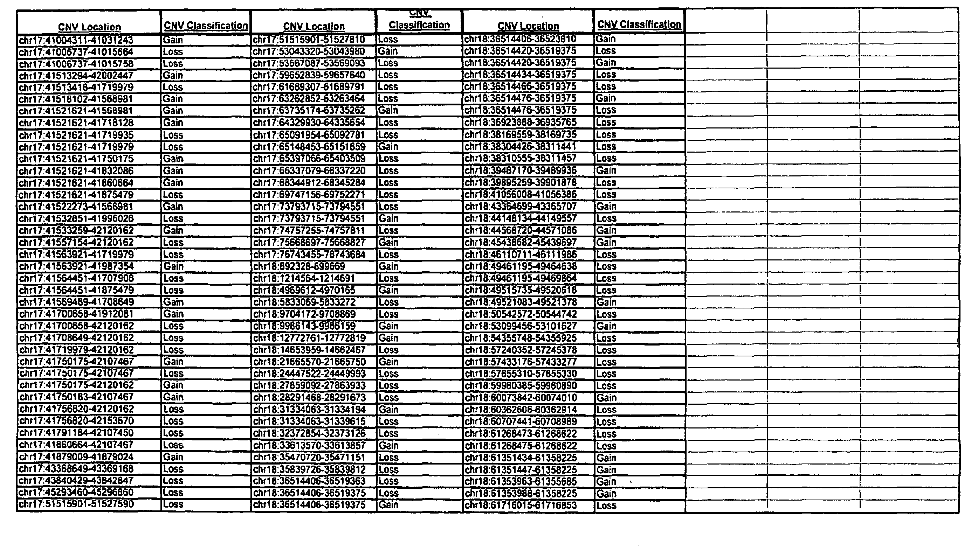

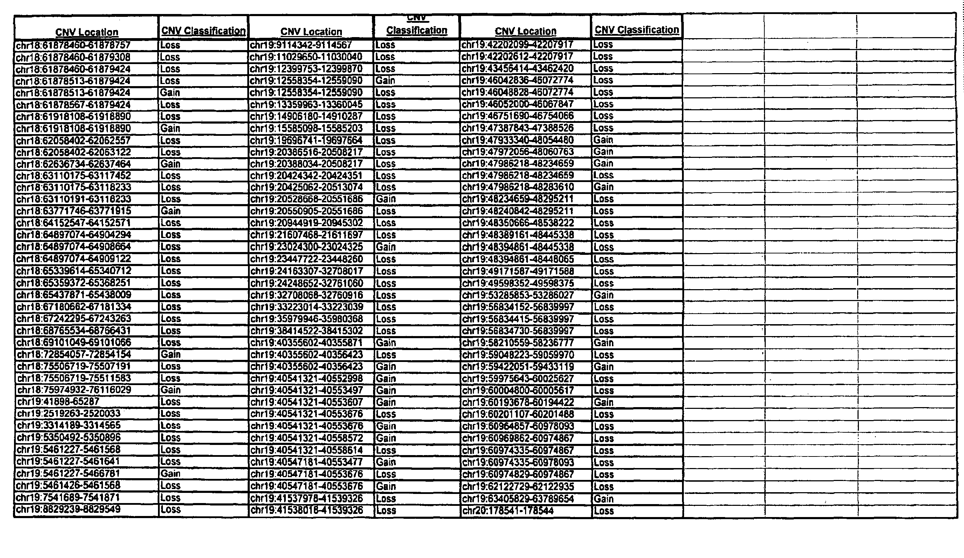

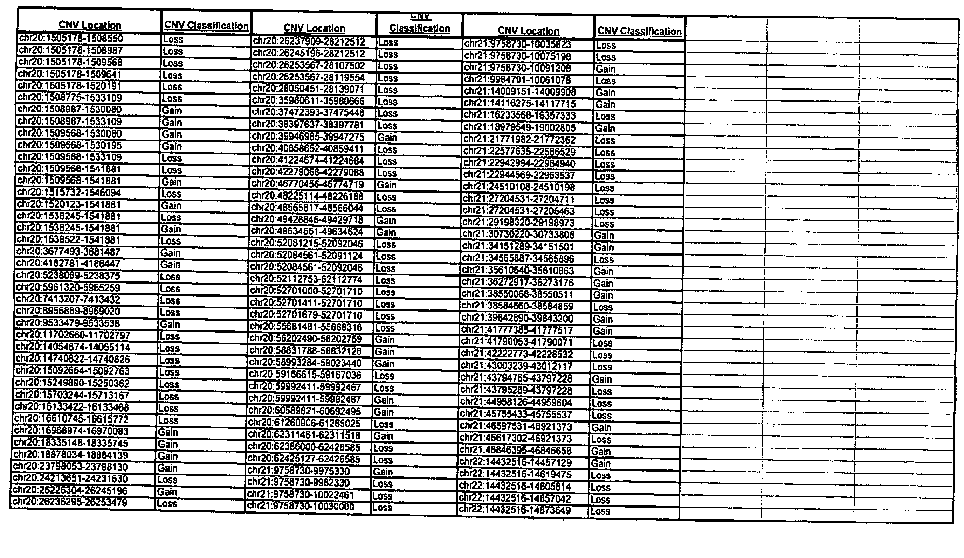

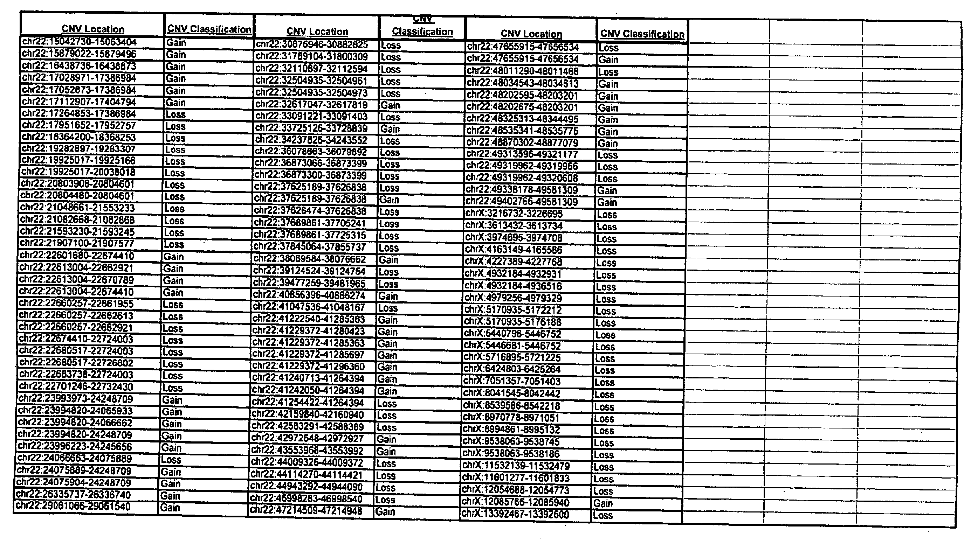

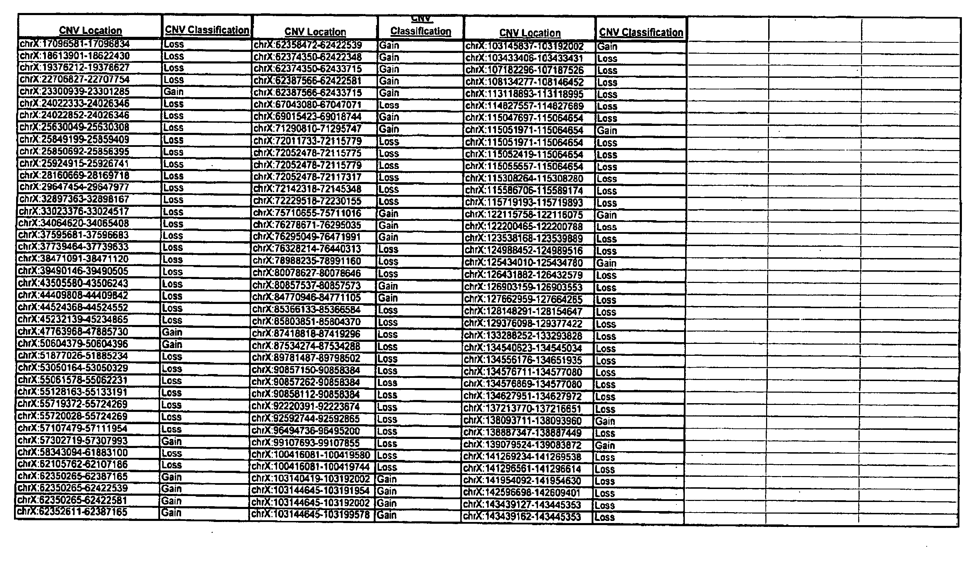

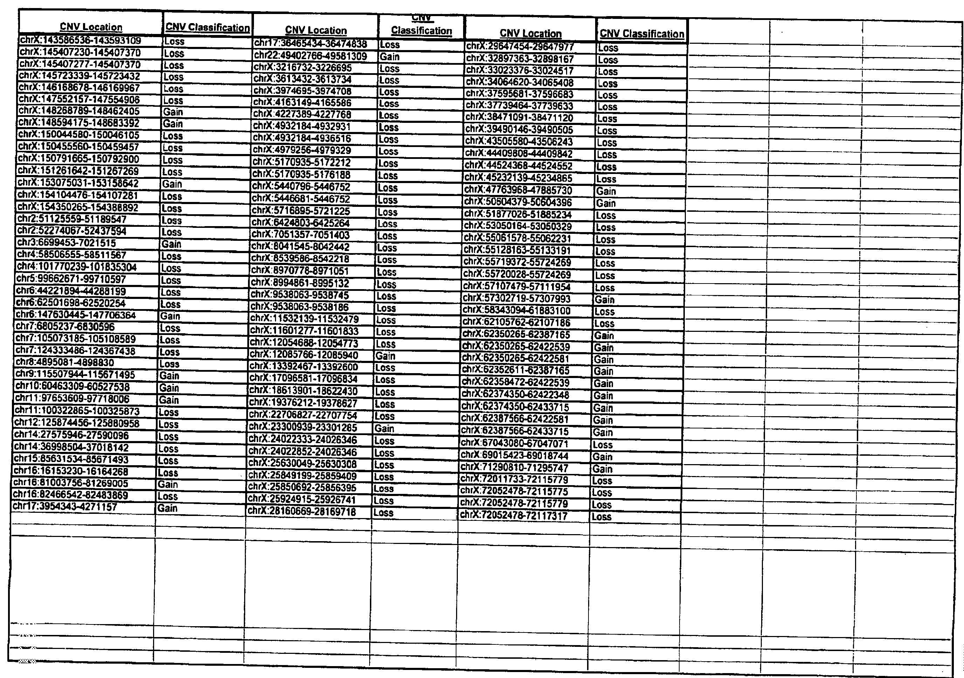

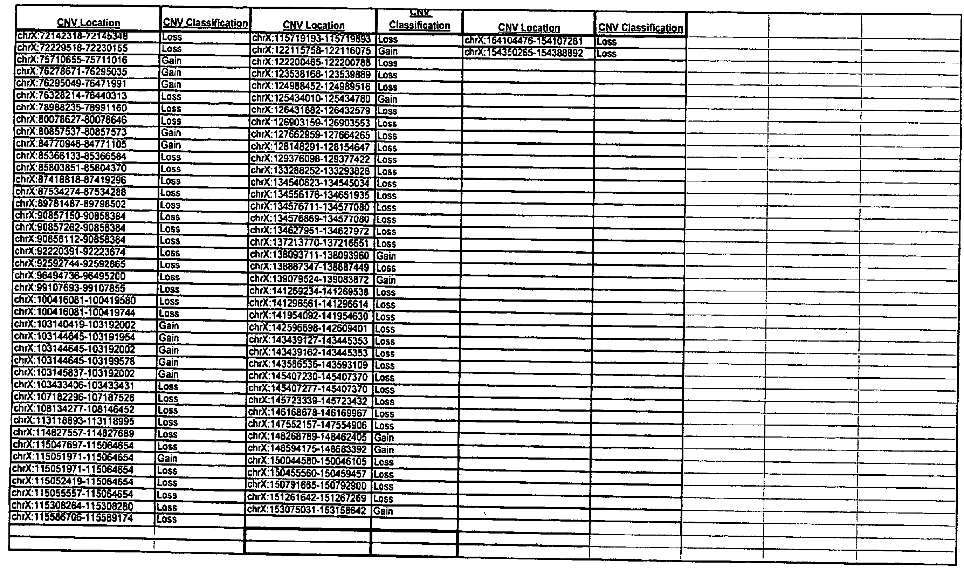

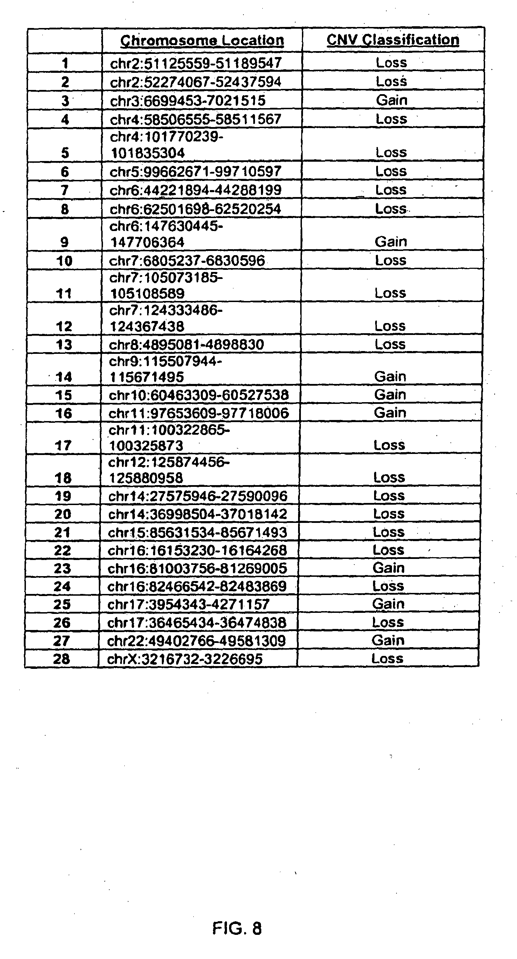

In one embodiment, the methods disclosed herein may comprise collecting a genetic sample from an individual and assaying the genetic sample for the presence of or the absence of one or more CNVs selected from the CNVs listed in FIG. 7 and FIG. 8. In one such embodiment, the methods disclosed herein may assay the genetic sample for the presence of one or more of the CNVs at the following chromosome locations: chr2:51125559-51189547, chr2:52274067-52437594, chr3:6699453-7021515, chr4:58506555-58511567, chr4:101770239-101835304, chr5:99662671-99710597, chr6:44221894-44288199, chr6:62501698-62520254, chr6:147630445-147706364, chr7:6805237-6830596, chr7:105073185-105108589, chr7:124333486-124367438, chr8:4895081-4898830, chr9:115507944-115671495, chr10:60463309-60527538, chr11:97653609-97718006, chr11:100322865-100325873, chr12:125874456-125880958, chr14:27575946-27590096, chr14:36998504-37018142, chr15:85631534-85671493, chr16:16153230-16164268, chr16:81003756-81269005, chr16:82466542-82483869, chr17:3954343-4271157, chr17:36465434-36474838, chr22:49402766-49581309, and chrX:3216732-3226695.

In one embodiment, one or more CNVs that are diagnostic or predictive of ASD may comprise genes and protein-coding regions of a chromosome. In one such embodiment, CNVs may impact genes that are expressed in any tissue. In another such embodiment, CNVs may be impact genes primarily expressed in the central nervous system. In another embodiment, a CNV that is diagnostic or predictive of ASD may be located in a non-coding region of a chromosome. In one such embodiment, CNVs impacting non-coding regions may affect gene regulation and expression.

In one embodiment, the CNVs described herein may be assayed and detected by any DNA, RNA (e.g., Northern blotting), or protein (e.g., Western blotting or protein activity) based method. Non-limiting examples of DNA-based methods include quantitative PCR; fluorescence in situ hybridization (FISH); Southern blotting; multiple amplifiable probe hybridization (MAPF, see Hollox et al., 2002, Expert Rev. Mol. Diagn., 2(4):370-8.); multiplex ligation-dependent probe amplification (MLPA, see Schouten et al., 2002, Nucleic Acids Res., 30(12):e57, kits available from MRC-Holland, Amsterdam, The Netherlands); QMPSF (Quantitative Multiplex PCR of Short Fluorescent Fragments, see Casilli et al., 2002, Hum. Mutat. 20(3):218-26), and combinations of such methods. These methods are well known in the art and one of ordinary skill in the art can perform the analyses using the genomic DNA isolated from the individual.

In one embodiment, the detection of the CNVs in the methods described herein is by oligonucleotide-based array comparative genomic hybridization (oligonucleotide-based array CGH). In one embodiment, the detection of the CNVs in the methods described herein is by bacterial artificial chromosome-based array comparative genomic hybridization (BAC-based array CGH). CGH are methods of determining the relative number of copies of nucleic acid sequences in one or more subject genomes or portions thereof (for example, a tumor cell) as a function of the location of those sequences in a reference genome (for example, a normal human genome, in one who is not diagnosed or predisposed with ASD). In one such embodiment, the intensity(ies) of the signals from each labeled subject nucleic acid and/or the differences in the ratios between different signals from the labeled subject nucleic acid sequences may be compared to determine the relative copy numbers of the nucleic acid sequences in the one or more subject genomes or portions thereof. U.S. Pat. Nos. 5,721,098, 5,665,549, 5,856,097, 5,976,790, 6,159,685, and 6,335,167 describes CGH and uses thereof.

In one embodiment, the methods disclosed herein may comprise using a BAC-based array CGH wherein, the CGH array chip is made using BAC amplified genomic sequences. In one embodiment, oligonucleotide-based array CGH, the chip may be made using a one or more synthetic oligonucleic acids comprising specific target genes or genomic region, or a combination thereof.

In one embodiment, the methods described herein may include the analysis of a genetic sample, wherein the analysis includes microarray-based analysis of the genomes of individuals that may be affected with ASD, or predisposed or at risk of ASD. In one such method, the genetic sequence of an individual's genome, or a portion of the genetic sequence of an individual's genome may be compared to the genetic test sequence of a normal healthy individual to detect genomic polymorphisms, such as SNPs and CNVs. In one embodiment, the analysis of a genetic sample from an individual may comprise a micro-array based method such as array comparative genomic hybridization (aCGH). In one such embodiment, the method of aCGH may comprise one or more of the following steps. First, DNA is extracted from a test sample (e.g., blood, skin, fetal cells). The test DNA is then labeled with a fluorescent dye of a specific color, while DNA from a normal control (reference) sample is labeled with a dye of a different color. The two genomic DNAs, test and reference, are then mixed together and applied to a microarray. Because the DNAs have been denatured, they are single strands; thus, when applied to the slide, they attempt to hybridize with the arrayed single-strand probes. Next, digital imaging systems may be used to capture and quantify the relative fluorescence intensities of the labeled DNA probes that have hybridized to each target. The fluorescence ratio of the test and reference hybridization signals is determined at different positions along the genome, and it provides information on the relative copy number of sequences in the test genome as compared to the normal genome.

In one embodiment, the methods disclosed herein may comprise the identification of known or novel CNVs. Generally, a normal base pair in a subject's genome has two copies, one on each chromosome. A base pair on the X chromosome in men will normally have only one copy. Even if the two base pairs are of different genotypes, there are still considered to be two copies. However, under certain circumstances, and especially in the case of certain diseases, there may sometimes be a base pair, or even an entire chromosome, that will be replicated more than two times, appear just once, or deleted entirely. The number of copies of a base pair is termed "copy number," and this variation of the copy number is termed "copy number variation," or CNV.

In one embodiment, the methods disclosed herein may comprise the use of microarray scans to assay and detect CNVs in a subject's genome. For microarray scans, the more copies there are of a base pair or chromosome region, the higher the total intensity will be, irrespective of which alleles may be present, even if the base pair is a polymorphism. Typically, processing is needed to transform intensity data to a quantile-normalized log base-2 (log2) ratio of intensities of observations versus a reference population. When the intensities of the observations are the same as the reference population median for a given base pair, the log2 ratio will be equal to zero. Amplifications over the reference standard will be significantly greater than zero, and deletions will be significantly less than zero.

In one embodiment, the CNVs as disclosed herein may include polymorphic CNVs that are functional CNVs. In another embodiment, the CNVs disclosed herein may include polymorphic CNVs that are not functional. In one embodiment, the genetic marker associated with autism or an ASD may be one or several CNVs or a haplotype of CNVs associated with autism. In one embodiment, one or more CNVs may be selected from those CNVs located in any region of any chromosome that shows association with one or more autism phenotypes. In one such embodiment, the CNVs may be selected from one or more of the CNVs listed in FIG. 7 and FIG. 8.

In one embodiment, the methods disclosed herein may comprise collecting a genetic sample from an individual and assaying the genetic sample for the presence of one or more SNPs and one or more CNVs, wherein the presence of the one or more SNPs and the one or more CNVs indicates that the individual is affected with ASD or may be at risk or predisposed to develop ASD. In one such embodiment, the genetic sample may be assayed for one or more SNPs and CNVs, wherein the SNPs may be selected from one or more the SNPs listed in FIG. 5 and FIG. 6; and wherein the CNVs may be selected from one or more of the CNVs listed in FIG. 7 and FIG. 8. In another embodiment, the genetic sample may be assayed for one or more SNPs and CNVs, wherein the SNPs may be selected from one or more of rs792065, rs1570056, rs909475, rs9295417, rs1990790, rs1419437, rs6490970, rs8033248, rs723049, rs11856, rs383902, rs725463, rs4801273, rs964795, rs2032088, rs1016694, rs2835667, rs1012959; and wherein the CNVs may be selected from one or more of the CNVs at the following chromosome locations: chr2:51125559-51189547, chr2:52274067-52437594, chr3:6699453-7021515, chr4:58506555-58511567, chr4:101770239-101835304, chr5:99662671-99710597, chr6:44221894-44288199, chr6:62501698-62520254, chr6:147630445-147706364, chr7:6805237-6830596, chr7:105073185-105108589, chr7:124333486-124367438, chr8:4895081-4898830, chr9:115507944-115671495, chr10:60463309-60527538, chr11:97653609-97718006, chr11:100322865-100325873, chr12:125874456-125880958, chr14:27575946-27590096, chr14:36998504-37018142, chr15:85631534-85671493, chr16:16153230-16164268, chr16:81003756-81269005, chr16:82466542-82483869, chr17:3954343-4271157, chr17:36465434-36474838, chr22:49402766-49581309, and chrX:3216732-3226695.

In another embodiment, the methods disclosed herein may comprise collecting a genetic sample from an individual and assaying the genetic sample for the presence or one or more SNPs, one or more CNVs, and at least one other polymorphic genetic marker, wherein the presence of the one or more SNPs, the one or more CNVs, and the at least one other polymorphic genetic marker indicates that the individual is affected with ASD or may be at risk or predisposed to develop ASD

Unless otherwise explained, all technical and scientific terms used herein have the same meaning as commonly understood by one of ordinary skill in the art to which this disclosure belongs. Definitions of common terms in molecular biology can be found in The Merck Manual of Diagnosis and Therapy, 18th Edition, published by Merck Research Laboratories, 2006 (ISBN 0-911910-18-2); Robert S. Porter et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8). Definitions of common terms in molecular biology may be found in Benjamin Lewin, Genes IX, published by Jones & Bartlett Publishing, 2007 (ISBN-13: 9780763740634); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

Unless otherwise stated, the present invention was performed using standard procedures, as described, for example in Maniatis et al., Molecular Cloning: A Laboratory Manual, Cold Spring Harbor Laboratory Press, Cold Spring Harbor, N.Y., USA (1982); Sambrook et al., Molecular Cloning: A Laboratory Manual (2 ed.), Cold Spring Harbor Laboratory Press, Cold Spring Harbor, N.Y., USA (1989); Davis et al., Basic Methods in Molecular Biology, Elsevier Science Publishing, Inc., New York, USA (1986); or Methods in Enzymology: Guide to Molecular Cloning Techniques Vol. 152, S. L. Berger and A. R. Kimmerl (eds.), Academic Press Inc., San Diego, USA (1987)).Current Protocols in Molecular Biology (CPMB) (Fred M. Ausubel, et al. ed., John Wiley and Sons, Inc.), Current Protocols in Protein Science (CPPS) (John E. Coligan, et. al., ed., John Wiley and Sons, Inc.), Current Protocols in Immunology (CPI) (John E. Coligan, et. al., ed. John Wiley and Sons, Inc.), Current Protocols in Cell Biology (CPCB) (Juan S. Bonifacino et. al. ed., John Wiley and Sons, Inc.), Culture of Animal Cells: A Manual of Basic Technique by R. Ian Freshney, Publisher: Wiley-Liss; 5th edition (2005), Animal Cell Culture Methods (Methods in Cell Biology, Vol. 57, Jennie P. Mather and David Barnes editors, Academic Press, 1st edition, 1998).

It should be understood that the following examples should not be construed as being limiting to the particular methodology, protocols, and reagents, etc., described herein and, as such, may vary. The terminology used herein is for the purpose of describing particular embodiments only, and is not intended to limit the scope of the embodiments disclosed herein.

### Examples

### Example 1

### Materials and Methods

*Subjects*: Subjects were members of 70 pedigrees having at least two family members with an ASD. A total of 653 subjects were genotyped, 192 of whom were defined as having either strictly defined Autistic Disorder or a more broadly defined ASD. Table 1 shows the characteristics of these families, which include 20 large extended pedigrees (6-9 generations), 6 families of moderate size (4-5 generations), and 44 smaller families (2-3 generations).

**Table 1**

| Type of pedigree | N of pedigrees | Avg # generations; SD (range) | Total subjects | Avg subjects per pedigree; SD (range) | Total ASD subjects | Avg ASD subjects per pedigree; SD (range) |
|---|---|---|---|---|---|---|
| Large (6-9 generations) | 20 | 7.96; 0.69 (6 to 9) | 331 | 17.21; 12.89 (5 to 50) | 82 | 5.22; 2.54 (2 to 9) |
| Moderate (4-5 generations) | 6 | 4; 0.00 (4) | 85 | 14.17; 11.34 (6 to 32) | 21 | 4.00; 3.39 (2 to 9) |
| Small (2-3 generations) | 44 | 2.5; 0.43 (2 to 3) | 237 | 5.39; 2.37 (2 to 11) | 89 | 2.04; 0.60 (1 to 3) |
| FULL SAMPLE | 70 | | 653 | | 192 | |

The 20 extended pedigrees were identified using the Utah Population Database (UPDB), a computerized genealogy database that contains family history information for over 6.5 million individuals who are, for the most part, descendants of the nineteenth century Utah pioneers (www.hci.utah.edu/groups/ppr/). Using the UPDB, many distant family relationships were identified between the individuals with ASD that were not known to the subjects or their families.

*Phenotyping:* Families interested in participating were asked to give questionnaire consent, then to give initial information regarding possible exclusion criteria and to complete the Social Communication Questionnaire (SCQ) (Berument SK, Rutter M, Lord C, Pickles A, Bailey A. Autism Screening Questionnaire. Los Angeles, CA: Western Psychological Services; 1999). The SCQ was developed as a parent report measure based on the Autism Diagnostic Interview-Revised (Lord C, Rutter M, Le Couteur A. Autism Diagnostic Interview-Revised: a revised version of a diagnostic interview for caregivers of individuals with possible pervasive developmental disorders. J Autism Dev Disord 1994;24:659-85). It has shown good discriminative validity (0.88) for ASDs, in addition to good sensitivity (0.83) and specificity (0.75) (Baranek GT, Bodfish JW, Gordon AM, Houser MB, Poe MD. Concurrent validity of the ADI-R and SCQ in high functioning autism In: Collaborative Programs of Excellence in Autism/Studies to Advance Autism Research and Treatment Annual Meeting; 2004; Bethesda, MD; 2004. Subjects were contacted based on records of previous diagnoses and/or if the SCQ score for the person with a suspected ASD was at least 15, the reported threshold used to identify autism. Subjects were excluded if they reported medical conditions known to be associated with autism (tuberous sclerosis, Fragile X, neurofibromatosis, congenital rubella, or PKU) or evidence of brain injury. If subjects were eligible for the study, they were asked to sign informed consent for DNA and additional assessments. When possible, all subjects with a suspected ASD were then given both the ADI-R and the Autism Diagnostic Observation Schedule-Generic (ADOS-G), and study diagnoses were made using these assessments (Lord C, Risi S, Lambrecht L, et al. The autism diagnostic observation schedule-generic: a standard measure of social and communication deficits associated with the spectrum of autism. J Autism Dev Disord 2000;30:205-23). For cases where assessments could not be obtained, diagnoses were made according to DSM-IV criteria by a psychologist trained in autism assessment using all available information (clinical records, other behavioral data, and other questionnaire and interview information). All genotyped subjects who were not given an ASD diagnosis were considered to have an unknown phenotype for this analysis.

IQ was measured in subjects with ASDs using assessments appropriate for age and developmental level. The Wechsler Intelligence Scale for Children WISC-III or Wechsler Adult Intelligence Scale WAIS-III, Differential Abilities Scale (DAS), and the Mullen Scales of Early Development were used (Wechsler D. Manual for the Wechsler Intelligence Scale for Children-Third Edition. San Antonio, TX: The Psychological Corporation; 1991; Wechsler D. Wechsler Adult Intelligence Scale-Third Edition. San Antonio, TX: The Psychological Corporation; 1997; Elliott C. Differential Ability Scales. San Antonio, TX: The Psychological Corporation; 1990; and Mullen E. Mullen Scales of Early Learning, AGS Edition. Circle Pines, MN: American Guidance Service; 1995 ).

The DAS is appropriate for children ages 2-1/2 to 18 years with either typical or delayed development, and the General Conceptual Ability Score from the DAS correlates well with the Full Scale IQ score of the Wecshler (WISC-III and WAIS-III) scales (Dicerbo KE BA. A convergent validity study of the differential ability scales and the Wechsler Intelligence Scale for Children-Third Edition with Hispanic Children. J Psychoed Assess 2000:344-52; and Dumont R CC, Price L, Whelley P. The relationship between the Differential Ability Scales (DAS) and the Wechsler Intelligence Scale for Children - Third Edition (WISC-III) for students with learning disabilities. Psychology in the Schools 1996:203-9). If a valid score was not obtainable on a subject under 68 months on the DAS, the Mullen, a standardized measure of cognitive function in young children, was used. For those administered the Mullen, the Early Learning Composite *t*-score (mean=50, sd=10) was converted to a standard score (mean=100, sd=15) as a measure of overall IQ (Sattler J. Assessment of Children: Cognitive Applications. La Mesa, CA: Jerome M. Sattler, Publisher, Inc.; 2001).

*Genotyping.* Genotyping services were provided by the Center for Inherited Disease Research (CIDR) using the 6K SNP linkage panel. Originally, 703 samples from the pedigrees were sent for genotyping, in addition to 32 blind duplicates of these pedigree samples for quality control (QC), for a total of 735 samples. QC genotyping also included internal controls used by CIDR. The genotyping platform was the Illumina Linkage Panel 12, which includes 6090 SNP markers, with an average genetic coverage of 0.65 cM. Illumina BeadStudio software was used to evaluate all genotypes using the quantitative GenCall score, which is an indicator of how well a DNA sample performed over all released SNP assays (Illumina, Inc., San Diego, CA, USA). A total of 55 samples were not released due to one or more of the following reasons: 1) poorly defined clusters, 2) excessive replicate and/or Mendelian errors, 3) more than 50% missing data, or 4) a higher than expected missing data rate for markers on the X chromosome, suggesting a possible mosaic 46XX or 46XO karyotype. Five of these 55 unreleased samples were blind duplicate pairs and the rest were pedigree subjects. There were therefore a total of 680 successfully genotyped subjects, of which 653 were pedigree members and 27 were blind duplicates for QC. Three of the smaller families were left with only one affected case after this QC step, so there were effectively 67 informative families in the sample.

Of the 6,090 total SNPs possible, 6,044 were released. Loci were dropped if atypical clustering patterns were found. A total of 4,309,372 genotypes were released with a missing data rate of 0.064% and a Mendelian consistency rate of 99.96%. SNPs with Mendelian errors were subsequently zeroed using PedCheck (O'Connell JR WD. PedCheck: A program for identifying genotype incompatibilities in linkage analysis. Am J Hum Genet 1998:259-66). The 27 blind duplicate pairs were checked for consistency between pairs using the file cleaned by PedCheck. Within these cleaned genotypes, duplicate reproducibility was 100%.

*Analyses:* The genetic map provided by CIDR, based on the deCODE genetic map, was used for the analysis (Kong A, Gudbjartsson DF, Sainz J, et al. A high-resolution recombination map of the human genome. Nat Genet 2002;31:241-7). Base pair positions were obtained from the March 2006 human reference sequence (hg18) assembly. Analysis was done using the multipoint linkage software MCLINK, a Markov chain Monte Carlo (MCMC) method that allows for multilocus linkage analysis on large extended pedigrees (Thomas A, Gutin, A., Abkevich, V. & Bansal, A.. Multipoint linkage analysis by blocked Gibbs sampling. Statistics and Computing 2000:259-69, incorporated herein by reference). Using blocked Gibbs sampling, MCLINK generates inheritance matrices from haplotype chains for the markers being analyzed, and performs an approximate calculation of the log-likelihood function linkage statistics. Internally, MCLINK runs the analysis five times to ensure a consistent solution. MCLINK has been used previously to identify candidate genomic regions for a number of complex diseases (Coon H, Matsunami N, Stevens J, et al. Evidence for Linkage on Chromosome 3q25-27 in a Large Autism Extended Pedigree. Hum Hered 2006;60:220-6; and Christensen GB, Camp NJ, Farnham JM, Cannon-Albright LA. Genome-wide linkage analysis for aggressive prostate cancer in Utah high-risk pedigrees. Prostate 2007;67:605-13 ). Allele frequencies for the MCLINK analysis were estimated using all of the observed data.

A general parametric model-based analysis was performed using simple dominant and recessive model parameters that reproduced the reported population frequency of ASDs. This parametric approach is well suited to the analysis of a complex trait (such as ASDs), particularly when using complex, large pedigrees. Parametric models, which provide assumptions about the genotype-phenotype relationship, simplify the parameter space and allow for more powerful and efficient analyses without leading to false positive results (Terwilliger JD, Goring HH. Gene mapping in the 20th and 21st centuries: statistical methods, data analysis, and experimental design. Hum Biol 2000;72:63-132; Goring HH, Terwilliger JD. Linkage analysis in the presence of errors I: complex-valued recombination fractions and complex phenotypes. Am J Hum Genet 2000;66:1095-106; and Greenberg DA, Abreu P, Hodge SE. The power to detect linkage in complex disease by means of simple LOD-score analyses. Am J Hum Genet 1998;63:870-9 ).

The multipoint heterogeneity LOD score (HLOD) allows for unlinked pedigrees and variation in the recombination fraction. HLOD scores may reflect the true position of a linkage peak more accurately under conditions of appreciable heterogeneity (as is the case with ASD), and HLOD scores have been shown to be more powerful than homogeneity LOD scores or model-free methods under these conditions (Goldin LR. Detection of linkage under heterogeneity: comparison of the two-locus vs. admixture models. Genet Epidemiol 1992;9:61-6; and Abreu PC, Greenberg DA, Hodge SE. Direct power comparisons between simple LOD scores and non-parametric LOD (NPL) scores for linkage analysis in complex diseases. Am J Hum Genet 1999;65:847-57).

As an additional check for false positive results, linkage peaks (defined by a 1-lod drop) achieving at least suggestive linkage evidence (HLOD > 1.86) were reanalyzed accounting for possible inflation due to LD between markers. SNPs were screened for LD using the PLINK software package, which recursively removes SNPs within a sliding window. A window size of 50 SNPs was set and shifted by 5 SNPs at each step, and used a Variance Inflation Factor (VIF) of 1.5, which is equivalent to an r2 of 0.33 regressed simultaneously over all SNPs in the selected window. This relatively strict threshold for LD means that peaks remaining after this screening effort are quite robust to possible inflation due to LD. Also, as part of the validation procedure, rare SNPs with a minor allele frequency less than 0.10 were removed. The screening deleted 63 of the 209 SNPs across all 10 of the re-analyzed regions. The SNPs were checked for Hardy-Weinberg Equilibrium (HWE) using PLINK, and one additional SNP was deleted for being out of HWE.

*Result:* Table 2 describes the diagnosis information for affected subjects.

**Table 2**

| | | | | | **Mean ADI Domain Scores (SD)** | | | | **ADOS: N subjects given each module** |
|---|---|---|---|---|---|---|---|---|---|
| **Diagnostic group** | **N** | **Male: Female** | **Mean Age (SD)** | **IQ>70 (%)** | **Soc** | **Verbal** | **Non-verbal** | **Restr/ Repet** | **Module 1; 2; 3; 4** |
| Autism (ADI/ADOS) | 122 | 107:15 | 11.4 (9.0) | 62/115 (53.91%) | 22.2 (6.0) | 17.7 (3.9) | 12.4 (2.3; N=27) | 6.9 (2.5) | 37; 29; 28; 28 |
| Autism (DSM-IV; 5 with ADI; 11 with ADOS) | 18 | 15:3 | 16.0 (12.5) | 10/11 (90.91%) | 21.0 (7.4) | 17.0 (1.7) | 13.0 (N=1) | 5.2 (1.6) | 1; 2; 2; 6 |
| ALL AUTISM | 140 | 122:18 | 12.0 (9.6) | 72/126 (57.14%) | 22.2 (6.0) | 17.6 (3.9) | 12.4 (2.3; N=28) | 6.8 (2.5) | 38; 31; 30; 34 |
| | | | | | | | | | |
| ASD (ADI/ADOS) | 44 | 33:11 | 13.4 (12.4) | 33/39 (84.62%) | 12.8 (5.7) | 10.9 (6.3) | 12.0 (N=1) | 5.2 (2.7) | 4; 10; 18; 12 |
| ASD (DSM-V; 0 with ADI; 6 with ADOS) | 8 | 7:1 | 30.0 (22.6) | 7/7 (100%) | | | | | 0; 0; 2; 4 |
| ALL ASD | 52 | 40:12 | 15.9 (15.3) | 40/46 (86.96%) | 12.8 (5.7) | 10.9 (6.3) | 12.0 (N=1) | 5.2 (27) | 4; 10; 20; 16 |
| | | | | | | | | | |
| All affected subjects | 192 | 163:30 | 13.1 (11.6) | 112/172 (65.12%) | 19.7 (7.2) | 15.5 (5.7) | 12.4 (2.2; N=29) | 6.4 (2.7) | 42; 41; 50; 50 |

Of the 192 total affected subjects, 166 had data on both the ADI-R and ADOS-G. Of these 166 subjects with complete information, 122 met criteria for strictly defined Autistic Disorder on both assessments, and 44 met criteria for an ASD, having closely missed the cut-off scores for strictly defined autism on one or both measures. The other 27 cases were missing one (N=22) or both (N=5) assessments due to testing difficulties and/or unavailability of a reliable informant. There was a 6.7:1 male/female ratio among the subjects with strictly defined autism, which fell to 3.3:1 among the subjects with an ASD. For all subjects combined, the male/female ratio was 5.4:1. Subjects with ASDs were older than subjects with strictly defined autism at entry into the study (mean age: 15.9 vs. 12.0 years), though the difference was not significant (t=1.74, p=0.09). As expected, ADI-R scores were significantly higher for the autism group compared to the ASD group (t=9.78, p<0.0001 for social; t=8.30, p<0.0001 for verbal; t=3.86, p=0.0002 for restricted interests/repetitive behaviors).

The nonverbal total cannot be compared because only one ASD subject was nonverbal. In addition, more subjects in the autism group were given the ADOS module 1 when compared to the ASD group. Quantitative scores on the ADOS are not compared because they were not designed to be used for that purpose. IQ was obtained for 172 of the 192 affected subjects. Of these, 112 (65.12%) had IQ>70. Significantly fewer subjects with strictly defined autism had IQ>70 (57.14%) compared to the percentage of ASD subjects with IQ>70 (86.96%; p<0.0001).

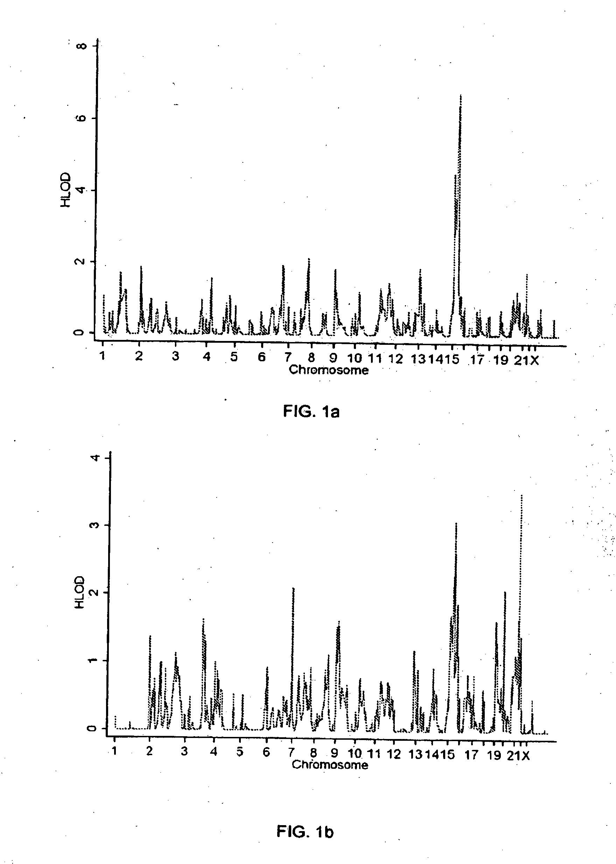

FIG. 1 shows genome-wide linkage results, and Table 3 gives scores for regions with evidence for linkage (HLOD≥ 1.86) (Lander E, Kruglyak L. Genetic dissection of complex traits: guidelines for interpreting and reporting linkage results. Nat Genet 1995;11:241-7). Each of these regions was screened for possible inflation due to LD as described above. Significant evidence of linkage (HLOD ≥ 3.3) was found on chromosome 15 and on chromosome 21.

**Table 3**

| Chromosome region location | SNP at maximum HLOD (basepair) | Original HLOD (model) | HLOD after LD screening (model) |
|---|---|---|---|
| 2p25.3-p24.1 | rs792065 (5,434,974) | 2.03 (rec) | 1.87 (rec) |
| 6q22.32-q24.1 | rs1570056 (137,101,370) | 1.98 (rec) | 1.81 (rec) |
| 6q27 | rs909475 (170,655,714) [screened: rs9295417 (170,734,025)] | 2.11 (dom) | 0.00 (dom) |
| 7q31.31-q32.3 | rs1990790 (129,820,866) [screened: rs1419437 (126,447,341)] | 2.45 (rec) | 1.97 (rec) |
| 13q12.11-q12.3 | rs6490970 (24,132,738) | 1.88 (rec) | 1.93 (rec) |
| 15q13.1-q14 | rs8033248 (29,459,872) | 5.01 (rec) | 4.09 (rec) |
| 15q14-q21.1 | rs723049 (36,837,208) | 4.05 (rec) | 3.59 (rec) |
| 15q21.2-q22.1 | rs11856 (55.629.733) | 6.59 (rec) | 5.31 (rec) |
| 15q21.1-q22.2 | rs383902 (56,821,466) [screened: rs725463 (57,930,371)] | 3.10 (dom) | 1.49 (dom) |
| 19q13.43 | rs4801273 (63,692,085) [screened: rs964795 (63,029,177)] | 2.09 (dom) | 0.01 (dom) |
| 21q22.12-q22.13 | rs2032088 (37,399,200); [screened rs1016694 (38,156,688)] | 3.52 (dom) | 0.01 (dom) |
| 21q22.12-q22.13 | rs2835667 (37,501,784) [screened: rs1012959 (36,983,492)] | 2.06 (rec) | 0.10 (rec) |

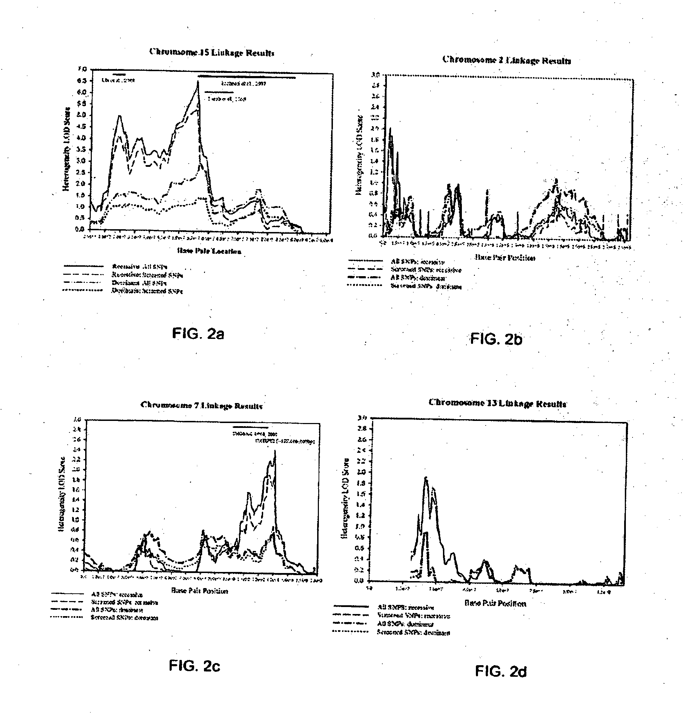

The chromosome 15 scores in Table 3 represent three possibly distinct regions, as shown in more detail in FIG 2a. Using a 1-LOD drop to define regions, the approximate boundaries of these three regions are: 27,440,000 bp - 32,790,000 bp; 32,790,000 bp - 43,260,000 bp; and 50,770,000 bp - 56,800,000 bp. Of particular interest are the SNP markers most closely associated with the maximum HLOD scores on a chromosomal region associated with ASD. For example, on chromosome 2, SNP rs792065, at basepair 5,434,974, showed a HLOD of 1.87. On chromosome 7, SNP rs1990790, at basepair 129,820,866, showed a HLOD of 1.97. On chromosome 13, SNP rs6490970 at basepair 24,132,738, showed a HLOD of 1.93. Linkage analysis on chromosome 15 revealed SNP rs8033248 at basepair 29,459,872, with a HLOD score of 4.09; SNP rs723049 at basepair 36,837,208 with a HLOD score of 3.59; SNP rs11856 at 55,629,733 with a HLOD score of 5.31; and SNP rs383902 at basepair 56,821,466 with a HLOD of 1.49.

A particular candidate gene of interest in chromosome 15 is the alpha 7 nicotinic receptor subunit gene in the 15q13-14 region, previously implicated in studies of schizophrenia (Iwata Y, Nakajima M, Yamada K, et al. Linkage disequilibrium analysis of the CHRNA7 gene and its partially duplicated region in schizophrenia. Neurosci Res 2007;57:194-202 and Severance EG, Yolken RH. Novel alpha7 nicotinic receptor isoforms and deficient cholinergic transcription in schizophrenia. Genes Brain Behav 2008;7:37-45 ). Other candidate genes of interest in the chromosome 15 region may be ubiquitin protein ligase E3A, UBEA, GABA-A receptor, and GABRB3. Additional candidate genes showing genetic linkage with autism are neuroligins, neurexins, contactin associated protein (CNTNAP2), serotonin transporter (SLC6A4), Engrailed 2 (EN2), and oxytocin receptor (OXTR).

As shown in Table 3 and FIGs. 2b-2d, regions of interest were also found on chromosomes 2, 6, 7, 13, 19, and 21. Linkage evidence was observed on chromosome 2p25.3-p24.1, from about 2,960,000 bp to about 10,660,000 bp that remained after LD screening. The relatively broad chromosome 7q31.31-q32.3 peak maintained linkage even after LD screening. The chromosome 13q12.11-q12.3 peak also exceeded the suggestive linkage evidence threshold even after eliminating SNPs in LD.

Linkage evidence was provided by multiple pedigrees, both large and small. Maximum scores for individual large pedigrees were not large enough to suggest complete sharing across all affected cases within any pedigree. The highest score for an individual pedigree within the three chromosome 15 peaks was a LOD of 2.27 under the 15q21.1-q22.2 peak in a 7-generation family with nine affected cases.

Characteristics of the autism phenotype were investigated for cases in the families supporting the three chromosome 15 linkage peaks in the subject samples. For the pedigrees that achieved nominal point-wise significance (i.e., LOD > 0.588 for an individual pedigree, p=0.05) within the three peaks, the proportion of cases with strict autism diagnoses was 72.7%, 71.6%, and 70.0% respectively, not significantly different from the overall proportion of autism cases in the entire sample (72.9%). Similarly, the proportion of female affected pedigree members was 16%, 18%, and 21%, not significantly different from the overall total female percentage of 19%. Finally, the proportion of affected subjects with IQ>70 was 66.7%, 63.2%, and 57.4%, not significantly different from the overall proportion of 65.12%.

### Example 2

*Subjects:* For this study, 386 subjects in 33 families were sampled for a whole-genome autism association study with the Affymetrix 250K chip comprising approximately 250,000 (250K) SNP genetic markers (Affymetrix, Inc., Santa Clara, CA). Of those individuals sampled, 125 were ASD-affected cases. Most of these families were identified with the UPDB where the search was performed with over 800 cases identified through multiple sources, producing about 25 extended families.

*Phenotyping and Genotyping:* Phenotyping was performed as described previously in Example 1. The Affymetrix 250K chip analysis was completed on all 386 subjects and quality control was performed on the SNP genetic marker data using PLINK software (Purcell S, Neale B, Todd-Brown K, et al. PLINK: a tool set for whole-genome association and population-based linkage analyses. Am J Hum Genet 2007;81:559-75).

*Analysis:* The analysis included using a broad affection status using all 125 affected cases. A Transmission Disequilibrium Test (TDT) was performed using nuclear families within extended pedigrees. The first pass showed that there were 17 regions with p ≤ 10⁻⁵. Simulation analysis was used to determine true positives.

Analysis also included the identification blocks of SNPs shared among affected cases in pedigrees, including the region size, and number of cases sharing within a pedigree. Priority was given to regions overlapping across 2 or more pedigrees. As discussed previously, linkage analysis was performed using the multipoint linkage software MCLINK. During testing for linkage using MCLINK, SNPs with minor allele frequencies (MAF) < 0.20 were deleted. Also, SNPs in LD and with Mendelian errors were deleted. This produced about 30,000 SNPs that are the most informative and provide independent information.

*Results:* Table 4 shows results from the genome-wide linkage analysis with LOD scores and non-parametric LOD (NPL) scores.

**Table 4**

| Chromosome Region Location | LOD Score | Non-parametric LOD Score |
|---|---|---|
| 1p12 | 3.73 | 3.0 |
| 2p13-14 | 2.38 | 4.09 |
| 3q26 | - | 3.77 |
| 4p15 | 4.12 | 3.7 |
| 15q11 | 5.2 | 4.24 |
| 15q13-14 | 4.00 | 3.64 |
| 15q14-15 | 3.22 | 3.31 |
| 15q21-22 | 3.75 | 3.48 |
| 15q22 | 3.72 | 4.84 |
| 20q11-12 | 2.8 | 3.51 |
| 20q13 | 2.92 | 2.99 |

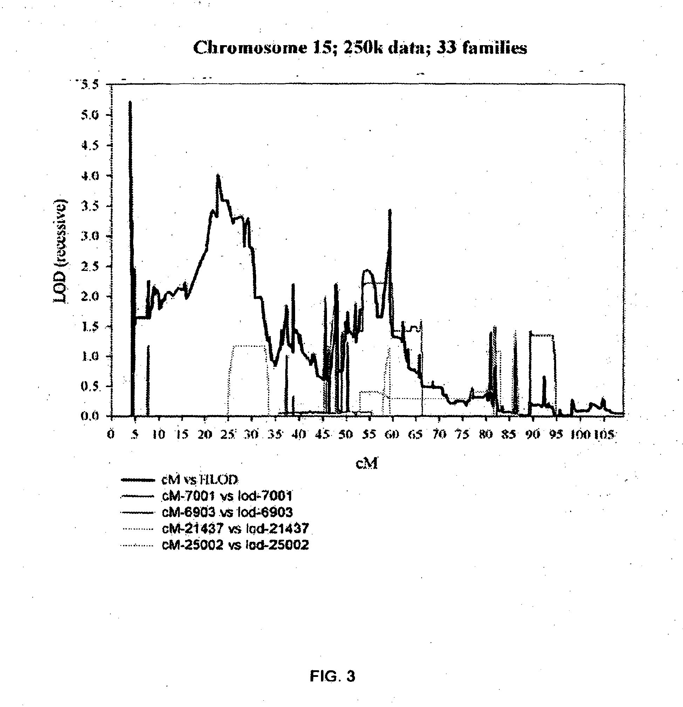

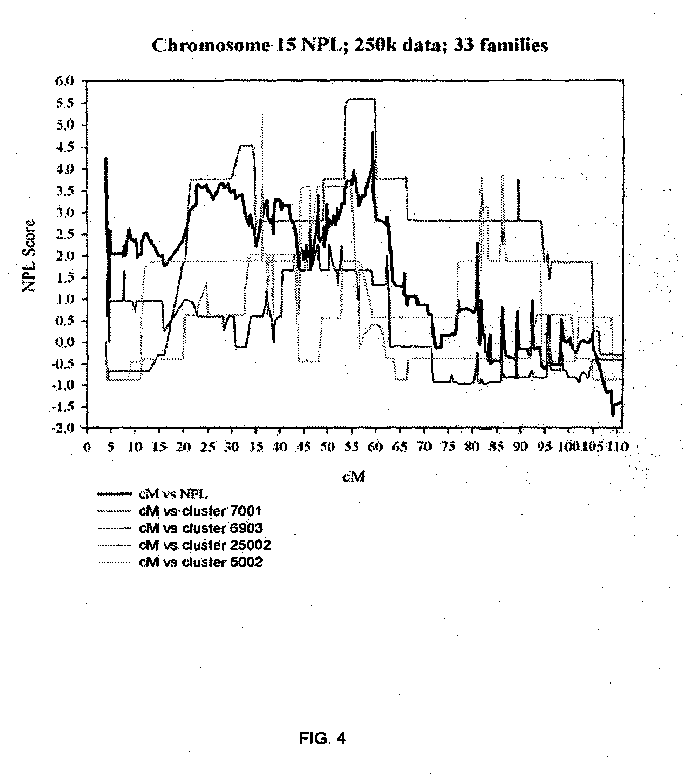

FIG. 3 and FIG. 4 show the LOD scores and the NPL scores, respectively, for the linkage analysis of chromosome 15.

### Example 3

*Subjects:* In this example, 360 subjects were genotyped in 25 families, including original 6-generation pedigree. Of these subjects, a total of 119 individuals were affected with ASD. Table 5 shows the description of the subjects sampled in this example. The subjects included 16 large extended pedigrees (6-9 generations), 9 smaller multiplex pedigrees (2-4 generations), and extended pedigrees, both of which were identified using the UPDB.

**Table 5**

| Type of pedigree | N of pedigrees | Avg # generations; SD | Total subjects | Avg subjects per pedigree; SD (range) | Total ASD subjects | Avg ASD subjects per pedigree; SD (range) |
|---|---|---|---|---|---|---|
| Large (6-9 generations) | 16 | 7.9; 0.7 | 266 | 15.8; 10.7 (6 to 41) | 81 | 4.72; 2.59 (2 to 10) |
| Small (2-4 generations) | 9 | 2.8; 0.8 | 94 | 10.4; 5.7 (5 to 22) | 38 | 4.22; 2.22 (2 to 9) |
| FULL SAMPLE | 25 | | 360 | | 119 | |

*Phenotype:* Initial screening for study entry was done using the SCQ. Inclusion criteria for affected subjects relied on the record of previous ASD diagnoses and/or SCQ score ≥ 15. The exclusion criteria included medical conditions known to be associated with autism (tuberous sclerosis, Fragile X, neurofibromatosis, congenital rubella, or PKU) or evidence of brain injury. When possible, the subjects were assessed using both ADI-R and the ADOS-G. If ADOS and ADI could not be obtained, diagnoses made according to DSM-IV criteria by a psychologist trained in autism assessment. Referring to Table 6, for subjects with Autistic Disorder (AD), 82 of 91 had both ADI and ADOS, 2 were missing ADI, and 6 were missing ADOS. For subjects with ASD, 25 of 28 had both ADI and ADOS, and 3 were missing ADOS.

**Table 6**

| | | | | **Mean ADI Domain Scores (SD)** | | | |
|---|---|---|---|---|---|---|---|
| **Diagnostic group** | **N** | **Male: Female** | **IQ>70 (%)** | **Comm** | **Verbal** | **Non-verbal** | **Restr/Repet** |
| AD | 91 | 80:11 (88% male) | 44/79 (55.7%) | 19.0 (6.0) | 15.1 (4.2; n=73) | 13.4 (1.3; n=18) | 6.1 (2.5) |
| ASD | 28 | 21:7 (75% male) | 19/24 (79.2%) | 11.6 (5.5) | 10.8 (5.0;n=27) | 12.0 (n=l) | 4.5 (2.3) |
| All affected subjects | 119 | 101:18 (85% male) | 63/103 (61.2%) | 19.7 (7.3) | 15.4 (5.2; n=100) | 13.0 (1.3; n=19) | 6.0 (2.6) |

*Genotype Data and Analysis:* Genotyping was performed using the Affymetrix 250K chip assay and error checking and data quality were checked with PLINK. A linkage subset of markers (n=∼30,000 SNPs) were identified by removing SNPs with minor allele frequencies < 0.20 and removing SNPs in high LD with each other. A window size of 50 SNPs was set and shifted by 5 SNPs at each step, and used a Variance Inflation Factor (VIF) of 1.5. As described in Example 1, the linkage analysis was performed using multipoint Markov chain Monte Carlo (MCMC) method MCLINK. Data were analyzed using general dominant and recessive parametric models, and NPL. The genetic map provided by CIDR, based on the deCODE genetic map, was used for the analysis

*Results:* As shown in Table 7, five linkage peaks were identified in the genome-wide linkage analysis including peaks at chromosome locations 3q13.2-q13.31, 3q26.31-q27.3, 20q11.21-q13.12, 7p14.1-p11.22 and 9p24.3. Of particular interest in this example is the peak on chromosome 20 as a possible location of an autism predisposition gene which exceeded suggestive evidence for linkage under both the NPL model (i.e., suggestive evidence threshold NPL ≥3.18) and the recessive model (i.e., suggestive evidence threshold LOD≥1.86).

**Table 7**

| | 3q13.2-q13.31 | 3q26.31-q27.3 | 20q11.21-q13.12 | 7p14.1-p11.22 | 9p24.3 |
|---|---|---|---|---|---|
| Max NPL score in region | 2.23 | 1.47 | 3.51 | 1.42 | 0.54 |
| Max recessive model (HLOD) | 1.05 | 1.01 | 2.80 | 0.066 | 0.19 |
| Max dominant model (HLOD) | 0.54 | 0.70 | 1.61 | 0.14 | 0.43 |

### Example 4

Chromosomal regions shared among affected members were identified within a given autism family/pedigree identified through the Utah Population Database. This method of identification of shared regions is supported by software developed at the University of Utah. The software automatically detects blocks of identical SNPs (haplotypes) in the autism-affected family members. This can detect potential disease carrying chromosomal regions. This shared haplotype analysis complements linkage analysis. Table 8 list these region and the size of haplotype blocks combined with linkage findings from previous linkage analysis.

**Table 8**

| **Chromosome Region** | **Chr** | **Begin Location (b)** | **End Location (b)** | **Method for Detection of Region** |
|---|---|---|---|---|
| 1p12 | 1 | 119,700,000 | 120,300,000 | Shared Haplotype and Linkage |
| 1q21 | 1 | 142,500,000 | 143,700,000 | Linkage |
| 2p14-p12 | 2 | 65,612,029 | 76,349,401 | Shared Haplotype and Linkage |
| 2q23-q31 | 2 | 153,638,312 | 174,296,304 | Shared Haplotype |
| 2q37 | 2 | 231,435,643 | 238,617,145 | Shared Haplotype |
| 3q13 | 3 | 111,604,019 | 112,685,490 | Shared Haplotype |
| 3q26-q27 | 3 | 174,594,938 | 185,701,563 | Shared Haplotype and Linkage |
| 4p15 | 4 | 24,300,000 | 32,500,000 | Linkage |
| 4q28-q31 | 4 | 137,362,554 | 141,629,142 | Shared Haplotype |
| 7p21 | 7 | 7,381,742 | 11,861,952 | Shared Haplotype and Linkage |
| 7p14 | 7 | 36,090,817 | 41,521,542 | Shared Haplotype |
| 7q21-q31 | 7 | 90,511,244 | 107,823,133 | Shared Haplotype |
| 7q31 | 7 | 118,907,651 | 120,298,906 | Linkage |
| 7q35-36 | 7 | 142,750,349 | 151,152,511 | Shared Haplotype |
| 12q21 | 12 | 76,119,990 | 77,788,028 | Shared Haplotype |
| 12q21 | 12 | 79,689,788 | 87,939,487 | Shared Haplotype |
| 14q11-q21 | 14 | 22,912,579 | 45,661,808 | Shared Haplotype |
| 14q32 | 14 | 92,331,535 | 103,509,782 | Shared Haplotype |
| 15q11 | 15 | 18,711,364 | 19,378,495 | Linkage |
| 15q12-q21 | 15 | 24,339,787 | 43,759,484 | Shared Haplotype and Linkage |
| 15q21-q22 | 15 | 51,907,830 | 57,389,313 | Shared Haplotype and Linkage |
| 16q22-23 | 16 | 73,415,053 | 77,780,513 | Shared Haplotype |
| 20p12 | 20 | 7,419,576 | 9,685,413 | Linkage |
| 20p11-q13 | 20 | 25,253,250 | 41,225,971 | Shared Haplotype and Linkage |
| 20q13 | 20 | 49,062,886 | 57,757,418 | Shared Haplotype and Linkage |

| | | | | |
|---|---|---|---|---|
| hg18 March 2006 (NCBI Build 36.1) | | | | |

### Example 5

*Subjects:* Using large, multiplex autism families, genomic regions of shared haplotypes and/or positive linkage with autism were identified. For 26 individuals affected with autism, the identified genomic regions were completely sequenced by capture of all the genes within the identified genomic regions. The exclusion criteria for the selected individuals included medical conditions known to be associated with autism (tuberous sclerosis, Fragile X, neurofibromatosis, congenital rubella, or PKU) or evidence of brain injury. When possible, the subjects were assessed using both ADI-R and the ADOS-G. If ADOS and ADI could not be obtained, diagnoses made according to DSM-IV criteria by a psychologist trained in autism assessment.

*Genotype and Analysis:* Nucleotide sequence data was collected for the 26 ASD-affected subjects using the Illumina Genome Analyzer IIx sequencer (Illumina, Inc., San Diego, CA, USA). The DNA sequence assembly was carried out using Mosaik (Michael Stromberg, Department of Biology, Boston College, MA, USA), MAQ (Mapping an Assembly with Qualities, Heng Li, The Wellcome Trust Sanger Institute, Hinxton, Cambridge, CB10 1SA, UK), Bowtie (Ben Langmead and Cole Trapnell, University of Maryland, MD, USA) and CLC Genomics Workbench (CLC bio USA, Cambridge, MA, USA). The SNP polymorphism detection was carried out using GigaBayes (Garbor Marth, Boston College, Chestnut Hill, MA, USA), MAQ, and CLC Genomics Workbench. Details of SNP identification using GigaBayes can be found at http://bioinformatics.bc.edu/marthlab/GigaBayes. Details of SNP identification using MAQ can be found at http://maq.sourceforge.net/maq-man.shtml. Details of SNP identification using CLCBio can be found at http://www.clcbio.com/index.php. The Human March 2006 assembly (NCBI Build36.1, hg18) was used as the reference human genome sequence.

From the total SNPs detected in the population of 26 ASD-affected subjects, functional SNPs were identified according to the function of gene-associated SNPs by cross-referencing to UCSC and RefSeq gene tracks. Info on USCS and RefSeq gene tracks can be found at the following links: http://genome.hmgc.mcw.edu/cgi-bin/hgTrackUi?hgsid=2274332&c=chrX&g=knownGene and http://genome.hmgc.mcw.edu/cgi-bin/hgTrackUi?hgsid=2274332&c=chrX&g=refGene.

The identified functional SNPs were classified as synonymous (no amino acid substitution), nonsense (STOP codon), nonconservative missense (nonconservative amino acid substitution), conservative missense (conservative amino acid substitution), or insertion/deletion in coding region (may cause frame-shift mutation). For nonconservative vs. conservative missense SNPs, BLOOSOM62 alignment score was used (Henikoff et al. Performance evaluation of amino acid substitution matrices. Proteins 17(1): 49-61, 1993) to predict the effects of coding amino acid substitutions on protein function.

*Results:* FIG. 5 shows the chromosome location (hg18 positions) and SNP classification of the 4,477 functional SNPs identified in the genetic samples from the 26 ASD-affected individuals. Of the total 4,477 SNPs that were initially identified, candidate SNPs were chosen according to the following methods. From the SNPs already reported in the dbSNP database, rare SNPs were selected with less than 5% minor allele frequencies along with the SNPs without reported allele frequency information. For the previously unknown and novel functional SNPs that were identified, each individual SNP was evaluated by visual inspection of each sequence alignment track to remove obvious false positives that may have been caused by PCR and sequencing chemistry artifacts.

FIG. 6 shows the chromosome location (hg18 positions) and SNP classification for the 388 candidate SNPs selected from the total 4,477 functional SNPs first identified in the 26 ASD-affected individuals. FIG. 6 also indicates the rs numbers (dbSNP reference ID), where available, for individual SNPs as well as the validation status for select SNPs. The indicated SNPs were validated, first, by DNA melting curve analysis using the LightScanner instrument (Idaho Technology, Inc., Salt Lake City, UT, USA) and carried out on PCR products from ASD-affected subjects and healthy control subjects, including the affected subjects in which the functional SNPs were originally indentified. Next, the PCR product was sequenced by a conventional Sanger method to confirm the presence of the SNP polymorphism. As shown in FIG. 6, the results of the SNP validation include 9 nonsense SNPs, 28 nonconservative-missense SNPs, and 1 splice-site SNP.

### Example 6

*Subjects and Genetic Analysis:* From large, multiplex autism families, 55 autistic family members were selected for genome-wide CNV analysis and identification. A population of 600 healthy subjects were used as the reference control population.

Briefly, CNV analysis on autism and control subjects was carried out on Affymetrix Human Genome-Wide SNP 6.0 microarray data. First, Affymetrix's Genotyping Console (GTC 4.0) (Affymetrix, Inc., Santa Clara, CA, USA) was used to perform copy number analysis. This analysis first creates a reference model file using the array data (CEL files). Then, each CEL file that were used to make the reference model file was analyzed against this reference model file. From this comparison, the sample's copy number and LOH (loss-of-heterozygosity) data are generated implementing hidden Markov model.

CNV identification also utilized GoldenHelix Inc's CNV analysis tool (CNAM) provided in their genetic analysis program package SNP & Variation Suite 7 (Golden Helix, Inc., Bozeman, MT, USA). CNAM incorporated a rigorous quality control process to minimize the bias that may be introduced by batch effects (plate, machine, and site variation), genomics waves, population stratification, inconsistent sample extraction and preparation procedures, cell types, temperature fluctuation, and even ambient ozone levels in a lab. These batch effects can lead to complications ranging from poorly defined segments to false and non-replicable findings. CNAM utilizes a powerful principal component analysis approach that enables it to simultaneously correct for all these variations, while significantly improving signal-to-noise ratios. CNAM also employs an optimal segmenting algorithm using dynamic programming to detect inherited and *de novo* CNVs on a per-sample (univariate) and multi-sample (multivariate) basis. Unlike hidden Markov models, which assume the means of different copy number states are consistent, optimal segmenting properly delineates CNV boundaries in the presence of mosaicism, even at. a single-probe level, and with controllable sensitivity and false discovery rate.

*Results:* FIG. 7 shows the 4,449 total CNVs identified along with each of their chromosome locations (hg18 positions) and CNV classifications. As shown in FIG. 7, the CNV classifications of gain or loss indicate whether each CNV region found in the autism subjects was duplicated/amplified (gain) or deleted (loss) in the genome. Also shown in FIG. 7, if the same CNV region shows gain in one patient and loss in another, the same CNV region is listed twice with gain and loss indications, respectively.

FIG. 8 shows the chromosome location and classification for the 28 candidate CNVs chosen from the 4,449 total CNVs shown in FIG. 7 that were determined by selecting only those CNVs that were observed in more than one of the 55 affected subjects and not observed at all in the 600 healthy control subjects.

The scope of the present invention should, therefore, be determined only by the following claims.

## Claims

1. A method of determining the presence of an Autism Spectrum Disorder (ASD) or the risk of an ASD in an individual, the method comprising:
assaying a genetic sample from the individual for the presence of at least one allele of a SNP located at human chromosome location chr2:73156164 (hg18 position),
wherein the presence in the genetic sample of the at least one allele of the SNP indicates that the individual has an ASD or is at risk of an ASD.

2. The method of claim 1, wherein the ASD is autism, Asperger Disorder or Pervasive Developmental Disorder - Not Otherwise Specified (PDD_NOS).

3. The method of claim 1 or 2, wherein assaying the genetic sample comprises purifying the genetic sample.

4. The method of any of claims 1 to 3, wherein assaying the genetic sample comprises amplifying the genetic sample.

5. The method of claim 4, wherein amplifying the sample comprises a polymerase chain reaction, ligase chain reaction, strand displacement amplification, transcription-based amplification, self sustained sequence replication (3SR), Qβ replicase protocols, nucleic acid sequence-based amplification (NASBA), repair chain reaction (RCR) or boomerang DNA amplification (BDA).

6. The method of any one of the preceding claims, wherein assaying the genetic sample comprises the use of a sequencing method.

7. The method of any one of claims 1 to 5, wherein assaying the genetic sample comprises a microarray based analysis.

8. The method of claim 7, wherein the microarray based analysis comprises hybridising the genetic sample to the microarray, wherein the microarray comprises DNA oligonucleotides between 5-20, 10-30, 12-30, 15-30, 10-50, 20-50 or 20-100 bases in length.

9. The method of claim 7 or 8, wherein DNA is extracted from the individual's genetic sample and labelled with a fluorescent dye prior to hybridisation to the microarray.

10. The method of claim 9, further comprising amplifying the DNA after extraction.

11. The method of any one of the preceding claims, wherein the genetic sample is collected from the individual as a whole blood, serum or plasma sample.

12. The method of any one of claims 1 to 10, wherein the genetic sample is collected from a swab.

13. An *in vitro* diagnostic test kit for diagnosing or predicting the presence of an ASD in a subject, comprising:
at least one oligonucleotide for detecting at least one SNP located at the human chromosome location chr2:73156164 (hg 18 position).

14. A method comprising:
assaying a genetic sample from an individual for the presence of at least one allele of a SNP located at human chromosome location chr2:73156164 (hg 18 position).

## Patentansprüche

1. Verfahren zum Bestimmen des Vorliegens einer Autismus-Spektrum-Störung (ASS) oder der Gefahr einer ASS in einem Individuum, wobei das Verfahren die Schritte umfasst:
Untersuchen einer genetischen Probe eines Individuums in der Gegenwart von wenigstens einem Allel eines SNP auf der menschlichen Chromosom-Lokalisierung chr2:73156164 (Position hg18),
wobei die Gegenwart von dem wenigstens einen Allel des SNP in der genetischen Probe anzeigt, dass ein Individuum eine ASS aufweist oder dem Risiko einer ASS unterliegt.

2. Verfahren nach Anspruch 1, bei dem die ASS Autismus, Asperger-Syndrom oder pervasive Entwicklungsstörung - nicht näher spezifiziert (PDD-NOS) ist.

3. Verfahren nach Anspruch 1 oder 2, bei dem das Untersuchen der genetischen Probe ein Reinigen der genetischen Probe umfasst.

4. Verfahren nach einem der Ansprüche 1 bis 3, bei dem das Untersuchen der genetischen Probe ein Amplifizieren der genetischen Probe umfasst.

5. Verfahren nach Anspruch 4, bei dem das Amplifizieren der Probe eine Polymerase-Kettenreaktion, eine Ligase-Kettenreaktion, eine Strand-Displacement-Amplifikation, eine transskriptionsbasierte Amplifikation, eine selbsterhaltende Sequenzreplikation (3SR), Qβ-Replikase-Protokolle, eine Nukleinsäuresequenz-basierte Amplifikation (NASBA), eine Reparatur-Kettenreaktion (RCR) oder eine Bumerang-DNA-Amplifikation (BDA) umfasst.

6. Verfahren nach einem der vorhergehenden Ansprüche, bei dem das Untersuchen der genetischen Probe das Verwenden eines Sequenzierungsverfahrens umfasst.

7. Verfahren nach einem der Ansprüche 1 bis 5, bei dem das Untersuchen der genetischen Probe eine Mikroarray-basierte Analyse umfasst.

8. Verfahren nach Anspruch 7, bei dem die Mikroarray-basierte Analyse ein Hybridisieren der genetischen Probe an das Mikroarray umfasst, wobei das Mikroarray DNA-Oligonukleotide zwischen 5-20, 10-30, 12-30, 15-30, 10-50, 20-50 oder 20-100 Basen in der Länge umfasst.

9. Verfahren nach Anspruch 7 oder 8, bei dem DNA aus der genetischen Probe des Individuums extrahiert und vor dem Hybridisieren an das Mikroarray mit einem Fluoreszenzfarbstoff markiert wird.

10. Verfahren nach Anspruch 9, ferner ein Amplifizieren der DNA nach dem Extrahieren umfassend.

11. Verfahren nach einem der vorhergehenden Ansprüche, bei dem die genetische Probe von dem Individuum als Vollblut-, Serum- oder Plasma-Probe genommen wird.

12. Verfahren nach einem der Ansprüche 1 bis 10, bei dem die genetische Probe von einem Abstrich genommen wird.

13. *In vitro*-Diagnose-Kit zur Diagnose oder Vorhersage des Vorliegens einer ASS in einem Probanden, umfassend:
wenigstens ein Oligonukleotid zum Detektieren von wenigstens einer SNP auf der menschlichen Chromosom-Lokalisierung chr2:73156164 (Position h18) .

14. Verfahren, umfassend:
Untersuchen einer genetischen Probe eines Individuums auf die Gegenwart von wenigstens einem Allel eines SNP auf der menschlichen Chromosom-Lokalisierung chr2:73156164 (Position h18).

## Revendications

1. Procédé pour déterminer la présence d'un trouble du spectre autistique (TSA) ou le risque de TSA chez un individu, le procédé comprenant :
l'analyse d'un échantillon génétique de l'individu pour identifier la présence d'au moins un allèle d'un SNP localisé à l'emplacement chromosomique humain chr2:73156164 (position hg18),
où la présence dans l'échantillon génétique du au moins un allèle du SNP indique que l'individu présente un TSA ou est exposé à un risque de TSA.

2. Procédé selon la revendication 1, dans lequel le TSA est l'autisme, le syndrome d'Asperger ou un trouble envahissant du développement non spécifié (TED-NS).

3. Procédé selon la revendication 1 ou 2, dans lequel l'analyse de l'échantillon génétique comprend la purification de l'échantillon génétique.

4. Procédé selon l'une quelconque des revendications 1 à 3, dans lequel l'analyse de l'échantillon génétique comprend l'amplification de l'échantillon génétique.

5. Procédé selon la revendication 4, dans lequel l'amplification de l'échantillon comprend une réaction en chaîne par polymérase, une réaction en chaîne par ligase, une amplification par déplacement de brin, une amplification médiée par la transcription, une réplication de séquence auto-entretenue (3SR), des protocoles utilisant une Qβ réplicase, une amplification basée sur une séquence d'acide nucléique (NASBA), une réaction en chaîne par réparation (RCR) ou une amplification d'ADN par effet boomerang (BDA).

6. Procédé selon l'une quelconque des revendications précédentes, dans lequel l'analyse de l'échantillon génétique comprend l'utilisation d'un procédé de séquençage.

7. Procédé selon l'une quelconque des revendications 1 à 5, dans lequel l'analyse de l'échantillon génétique comprend une analyse basée sur un microréseau.

8. Procédé selon la revendication 7, dans lequel l'analyse basée sur un microréseau comprend une hybridation de l'échantillon génétique au microréseau, où le microréseau comprend des oligonucléotides d'ADN d'une longueur comprise entre 5-20, 10-30, 12-30, 15-30, 10-50, 20-50 ou 20-100 bases.

9. Procédé selon la revendication 7 ou 8, dans lequel l'ADN est extrait de l'échantillon génétique de l'individu puis est marqué avec un colorant fluorescent avant l'hybridation au microréseau.

10. Procédé selon la revendication 9, comprenant en outre une amplification de l'ADN après l'extraction.

11. Procédé selon l'une quelconque des revendications précédentes, dans lequel l'échantillon génétique est collecté chez l'individu sous la forme d'un échantillon de sang total, de sérum ou de plasma.

12. Procédé selon l'une quelconque des revendications 1 à 10, dans lequel l'échantillon génétique est collecté à partir d'un prélèvement à l'écouvillon.

13. Kit de test de diagnostic *in vitro* pour diagnostiquer ou prédire la présence d'un TSA chez un sujet, comprenant :
au moins un oligonucléotide pour détecter au moins un SNP localisé à l'emplacement chromosomique humain chr2:73156164 (position hg18).

14. Procédé comprenant :
l'analyse d'un échantillon génétique d'un individu pour identifier la présence d'au moins un allèle d'un SNP localisé à l'emplacement chromosomique humain chr2:73156164 (position hg18).
